# EUROPEAN PATENT SPECIFICATION

(11) **EP 1 404 682 B1**
(45) Date of publication and mention of the grant of the patent: **14.09.2005**
(21) Application number: 02740410.2
(22) Date of filing: 25.06.2002
(51) Int. Cl.: C07D 495/08, C07D 495/18, C07D 498/08, A61K 31/4365, A61K 31/4375, A61K 31/5365

(54) **METHOD OF INHIBITING PTP 1B AND/OR T-CELL PTP AND/OR OTHER PTPASES WITH AN ASP RESIDUE AT POSITION 48**
VERFAHREN ZUR HEMMUNG VON TYROSINPHOSPHATASE 1B UND/ODER T-ZELLENPROTEIN-TYROSINPHOSPHATASE UND/ODER ANDEREN PTPASEN MIT EINEM ASP-REST IN POSITION 48
PROCEDE PERMETTANT D'INHIBER PTP1B ET / OU PTP DE LYMPHOCYTEET ET / OU D'AUTRES PTPASES A RESTE ASP EN POSITION 48

(30) Priority: 29.06.2001 DK 200101022; 21.01.2002 DK 200200105
(43) Date of publication of application: 07.04.2004
(73) Proprietor: NOVO NORDISK A/S, 2880 Bagsvaerd (DK)
(72) Inventor: HANSEN, Thomas, Kruse, DK-2730 Herlev (DK); OLSEN, Ole, Hvilsted, DK-Bronshoj 2700 (DK); PETERSEN, Anders, Klarskov, DK-Denmark 2850 (DK); LAU, Jesper, DK-Farum 3520 (DK); ANDERSEN, Henrik, Sune, DK-2800 Lyngby (DK); MOLLER, Niels, Peter, Hundahl, DK-2100 Kobenhavn (DK)
(74) Representative: Nielsen, Lars Balzer
(86) International application number: PCT/DK2002/000425
(87) International publication number: WO 2003/002569

(56) References cited:
- WO-A-99/46237
- WO-A-99/46267

## Description

### Field of the Invention

This invention relates to oxalylamide inhibitors of Protein Tyrosine Phosphatase 1B (PTP1B) and/or T-cell Protein Tyrosine Phosphatase (TC-PTP) and/or Protein Tyrosine Phosphatases (PTPases) having an aspartic acid (Asp) in position 48 (PTP1B numbering, Chernoff *et al., Proc. Natl. Acad. Sci. USA 87*: 2735-2789 (1989)) and a method of inhibiting such PTPases by exposing the enzyme to inhibitor compounds according to the invention. This invention also relates to (I) the design and selection of inhibitors, which bind to the active site of PTP1B and/or TC-PTP and/or PTPases having an aspartic acid (Asp) in position 48 (II) the synthesis of said inhibitors, methods for their preparation and (III) to compositions comprising the inhibitor compounds.

### Background of the Invention

Reversible protein phosphorylation is now well recognized as an important mechanism utilized by cells to transduce and regulate signals during different stages of cellular function (Hunter, *Phil. Trans. R. Soc. Lond*. B *353*: 583-605 (1998); Chan *et al., Annu. Rev. Immunol*. *12*: 555-592 (1994); Zhang, *Curr. Top. Cell. Reg. 35*: 21-68 (1997); Matozaki and Kasuga, *Cell. Signal. 8:* 113-19 (1996); Fischer *et al, Science 253*: 401-6 (1991); Flint *et al., EMBO J. 12*: 1937-46 (1993)). The level of tyrosine phosphorylation is balanced by the opposing action of protein tyrosine kinases and protein tyrosine phosphatases. There are at least two major classes of phosphatases: (1) those that dephosphorylate proteins (or peptides) that contain a phosphate group(s) on a serine or threonine moiety (termed Ser/Thr phosphatases) and (2) those that remove a phosphate group(s) from the amino acid tyrosine (termed protein tyrosine phosphatases or PTPases or PTPs).

The PTPases are a family of enzymes that can be classified into two groups: a) intracellular or nontransmembrane PTPases and b) receptor-type or transmembrane PTPases. In addition, dual-specificity phosphatases and low molecular weight phosphatases are able to dephosphorylate phospho tyrosyl proteins. See, e.g., WO 97/ 39746; WO 97/40017; WO 99/15529; WO 97/08934; WO 98/27065; WO 99/46236; WO 99/46244; WO 99/46267; WO 99/46268 and WO 99/46237.

***Intracellular PTPases:*** Most known intracellular type PTPases contain a single conserved catalytic phosphatase domain consisting of 220-240 amino acid residues. The regions outside the PTPase domains are believed to play important roles in localizing the intracellular PTPases subcellularly (Mauro, LJ. and Dixon, J.E. *TIBS 19*: 151-155 (1994)). The first intracellular PTPase to be purified and characterized was **PTP1B**, which was isolated from human placenta (Tonks *et al., J. Biol. Chem.* 263: 6722-6730 (1988)). Shortly after, PTP1B was expressed recombinantly (Charbonneau *et al*., *Proc. Natl. Acad. Sci. USA 86*: 5252-5256 (1989); Chernoff *et al., Proc. Natl. Acad. Sci. USA.87*: 2735-2789 (1989)). Other examples of intracellular PTPases include (1) T-cell **PTPase/ TC-PTP** (Cool *et al*. *Proc. Natl. Acad. Sci. USA 86*: 5257-5261 (1989)), (2) **rat brain PTPase** (Guan *et al., Proc. Natl. Acad. Sci. USA 87*:1501-1502 (1990)), (3) neuronal phosphatase **STEP** (Lombroso *et al., Proc. Natl. Acad. Sci. USA 88*: 7242-7246 (1991)), (4) ezrin-domain containing PTPases: **PTPMEG1** *(Guet al., Proc. Natl. Acad. Sci. USA 88*: 5867-57871 (1991)), **PTPH1** (Yang and Tonks, *Proc. Natl. Acad. Sci. USA 88*: 5949-5953 (1991)), **PTPD1 and PTPD2** (Møller *et al., Proc. Natl. Acad. Sci. USA 91:* 7477-7481 (1994)), **FAP-1/BAS** (Sato *et al., Science 268*: 411-415 (1995); Banville *et al., J. Biol. Chem. 269*: 22320-22327 (1994); Maekawa *et al., FEBS Letters 337*: 200-206 (1994)), and SH2 domain containing PTPases: **PTP1C/SH-PTP1/SHP-1** (Plutzky *et al*., *Proc. Natl. Acad. Sci. USA 89*: 1123-1127 (1992); Shen *et al., Nature Lond. 352*: 736-739 (1991)) and **PTP1D/Syp/SH-PTP2/SHP-2** (Vogel *et al., Science 259*: 1611-1614 (1993); Feng *et al., Science 259*: 1607-1611 (1993); Bastein *et al., Biochem. Biophys. Res. Comm. 196*: 124-133 (1993)).

***Receptor-type PTPases*** consist of a) a putative ligand-binding extracellular domain, b) a transmembrane segment, and c) an intracellular catalytic region. The structures and sizes of the putative ligand-binding extracellular domains of receptor-type PTPases are quite divergent. In contrast, the intracellular catalytic regions of receptor-type PTPases are very homologous to each other and to the intracellular PTPases. Most receptor-type PTPases have two tandemly duplicated catalytic PTPase domains.

The first receptor-type PTPases to be identified were (1) **CD45/LCA** (Ralph, S.J., *EMBO J. 6*: 1251-1257 (1987)) and (2) **LAR** (Streuli *et al., J. Exp. Med. 168*: 1523-1530 (1988)) that were recognized to belong to this class of enzymes based on homology to PTP1B (Charbonneau *et al., Proc. Natl. Acad.*

*Sci. USA 86*: 5252-5256 (1989)). CD45 is a family of high molecular weight glycoproteins and is one of the most abundant leukocyte cell surface glycoproteins and appears to be exclusively expressed upon cells of the hematopoietic system (Trowbridge and Thomas, *Ann. Rev. Immunol. 12*: 85-116 (1994)).

The identification of CD45 and LAR as members of the PTPase family was quickly followed by identification and cloning of several different members of the receptor-type PTPase group. Thus, 5 different PTPases, (3) **PTPα,** (4) **PTPβ,** (5) **PTPδ,** (6) **PTPε,** and (7) **PTPζ,** were identified in one early study (Krueger *et al., EMBO J. 9*: 3241-3252 (1990)). Other examples of receptor-type PTPases include (8) **PTPγ** (Bamea *et al., Mol. Cell. Biol. 13*: 1497-1506 (1995)) which, like **PTPζ** (Krueger and Saito, *Proc. Natl. Acad. Sci. USA 89:* 7417-7421 (1992)) contains a carbonic anhydrase-like domain in the extracellular region, (9) **PTPµ** (Gebbink *et al., FEBS Letters 290*: 123-130 (1991)), (10) **PTPκ** (Jiang *et al., Mol. Cell. Biol. 13:* 2942-2951 (1993)). Based on structural differences the receptor-type PTPases may be classified into subtypes (Fscher *et al*., *Science 253*: 401-406 (1991)): (I) CD45; (II) LAR, PTPd, (11) **PTPσ ;** (III) **PTPβ,** (12) SAP-1 (Matozaki *et al., J. Biol. Chem. 269:* 2075-2081 (1994)), (13) **PTP-U2/GLEPP1** (Seimiya *et al., Oncogene 10*: 1731-1738 (1995); Thomas *et al., J. Biol. Chem. 269*: 19953-19962 (1994)), and (14) **DEP-1;** (IV) **PTPα, PTPε.** All receptor-type PTPases except Type III contain two PTPase domains. Novel PTPases are frequently identified, and it is anticipated that between 100 and more than 500 different species will be found in the human genome.

### Description of the invention

PTPases are the biological counterparts to protein tyrosine kinases (PTKs). Therefore, one important function of PTPases is to control, and especially down-regulate, the activity of PTKs. However, a more complex picture of the function of PTPases has emerged. Thus, several studies indicate that some PTPases act as positive mediators of cellular signaling. As an example, the SH2 domain-containing SHP-2 acts as a positive mediator in insulin-stimulated Ras activation (Noguchi *et al., Mol. Cell. Biol*. *14:* 6674-6682 (1994)) and of growth factor-induced mitogenic signal transduction (Xiao et *al*., *J. Biol. Chem. 269:* 21244-21248 (1994)), whereas the homologous SHP-1 acts as a negative regulator of growth factor-stimulated proliferation (Bignon and Siminovitch, *Clin.Immunol. Immunopathol. 73*: 168-179 (1994)). Another example of PTPases as positive regulators has been provided by studies designed to define the activation of the Src-family of tyrosine kinases. In particular, several lines of evidence indicate that CD45 is positively regulating the activation of hematopoietic cells, and that the mechanism of such positive regulation may involve dephosphorylation of the C-terminal tyrosine of Fyn and Lck (Chan *et al., Annu. Rev. Immunol. 12*: 555-592 (1994)). In addition, recent studies have shown that CD45 suppresses JAK (Janus kinase) kinases and negatively regulates cytokine receptor signaling. Thus, targeted disruption of the cd45 gene leads to enhanced cytokine and interferon-receptor-mediated activation of JAKs and STAT (signal transducer and activators of transcription) proteins. In vitro, CD45 directly dephosphorylates and binds to JAKs (Irie-Sasaki *et al., Nature 409*: 349-354 (2001)).

The association of many PTPases with cell proliferation, tranformation and differentiation has now been established. PTP1B, a phosphatase whose structure was the first PTPase to be elucidated (Barford *et al*., *Science 263*: 1397-1404 (1994)) has been shown to be involved in insulin-induced oocyte maturation (Flint *et al*., *The EMBO J. 12*: 1937-46 (1993)) and the overexpression of this enzyme has been implicated in p185^{c-erb B2} -associated breast and ovarian cancers (Weiner, *et al*., *J. Natl. cancer Inst*. *86:* 372-8 (1994); Weiner *et al*., *Am. J. Obstet. Gynecol. 170*: 1177-883 (1994)). The association with cancer is on the basis of evidence that overexpression of PTP1B is statistically correlated with increased levels of p185^{*c-erb* B2} in ovarian and breast cancer. The role of PTP1B in the etiology and progression of the disease has not yet been elucidated. Inhibitors of PTP1B therefore would help clarify the role of PTP1B in cancer and in some cases provide therapeutic treatment for certain forms of cancer.

### PTPases: the insulin receptor signaling pathway/diabetes

Insulin is an important regulator of different metabolic processes and plays a key role in the control of blood glucose. Defects related to its synthesis or signalling lead to diabetes mellitus. Binding of insulin to the insulin receptor (IR) causes rapid (auto)phosphorylation of several tyrosine residues in the intracellular part of the β-subunit. Three closely positioned tyrosine residues (the tyrosine-1150 domain) must all be phosphorylated to obtain full activity of the insulin receptor tyrosine kinase (IRTK) which transmits the signal further downstream by tyrosine phosphorylation of other cellular substrates, including insulin receptor substrate-1 (IRS-1) (Wilden *et al., J. Biol. Chem. 267*: 16660-16668 (1992); Myers and White, *Diabetes 42:* 643-650 (1993); Lee and Pilch, *Am. J. Physiol. 266*: C319-C334 (1994); White *et al., J. Biol. Chem. 263*: 2969-2980 (1988)). The structural basis for the function of the tyrosine-triplet has been provided by X-ray crystallographic studies of IRTK that showed the tyrosine-1150 domain to be autoinhibitory in its unphosphorylated state (Hubbard *et al., Nature 372:* 746-754 (1994)) and of the activated IRTK (Hubbard, *EMBO J. 16*: 5572-5581 (1997)).

Several studies clearly indicate that the activity of the auto-phosphorylated IRTK can be reversed by dephosphorylation *in vitro* (reviewed in Goldstein, *Receptor 3*: 1-15 (1993); Mooney and Anderson, *J*. *Biol. Chem. 264*: 6850-6857 (1989)), with the tri-phosphorylated tyrosine-1150 domain being the most sensitive target for protein-tyrosine phosphatases (PTPases) as compared to the di- and mono- phosphorylated forms (King *et al*., *Biochem. J. 275*: 413-418 (1991)). This tyrosine-triplet functions as a control switch of IRTK activity and IRTK appears to be tightly regulated by PTP-mediated dephosphorylation *in vivo* (Khan *et al., J. Biol. Chem. 264:* 12931-12940 (1989); Faure *et al., J. Biol. Chem. 267*: 11215-11221 (1992); Rothenberg *et al., J. Biol. Chem. 266*: 8302-8311 (1991)). The intimate coupling of PTPases to the insulin signaling pathway is further evidenced by the finding that insulin differentially regulates PTPase activity in rat hepatoma cells (Meyerovitch *et al., Biochemistry 31:* 10338-10344 (1992)) and in livers from alloxan diabetic rats (Boylan *et al., J. Clin. Invest. 90*: 174-179 (1992)).

Until recently, relatively little was known about the identity of the PTPases involved in IRTK regulation. However, the existence of PTPases with activity towards the insulin receptor can be demonstrated as indicated above. Further, when the strong PTPase-inhibitor pervanadate is added to whole cells an almost full insulin response can be obtained in adipocytes (Fantus *et al*., *Biochemistry 28:* 8864-8871 (1989); Eriksson *et al*., *Diabetologia 39:* 235-242 (1995)) and skeletal muscle (Leighton *et al*., *Biochem. J*. *276*: 289-292 (1991)). In addition, other studies show that a new class of peroxovanadium compounds act as potent hypoglycemic compounds *in vivo* (Posner *et al*.,*supra*). Two of these compounds were demonstrated to be more potent inhibitors of dephosphorylation of the insulin receptor than of the EGF-receptor, thus indicating that even such relatively unselective inhibitors may show some specificity in regulating different signal transduction pathways.

It was recently found that mice lacking the protein tyrosine phosphatase-1B gene (PTP1 B) (Elchebly *et al., Science 283:* 1544-1548 (1999)) yielded healthy mice that showed increased insulin sensitivity and were resistant to diet-induced obesity. These results were confirmed by Kaman at al *Mol. Cell Biol.* 20:5479-5489 (2000). The enhanced insulin sensitivity of the PTP^{-/-} mice was also evident in glucose and insulin tolerance tests.

The PTP-1B knock-out mouse showed many characteristics which would be highly desirable results for an anti-diabetes treatment. Most importantly, the knock-out mice grew normally and were fertile and have exhibited no increased incidence of cancer. Blood glucose and insulin levels were lowered, and insulin sensitivity increased. Moreover, the insulin-stimulated tyrosine phosphorylation levels of IR and IRS-1 were found to be increased/prolonged in muscle and liver - but not in fat tissue. Thus, the main target tissues for this type of approach would appear to be insulin action in liver and muscle.

Several other "diabetic" parameters were also improved, including plasma triglycerides, which were decreased in the knock-out mice. The knock-animals also exhibited a resistance to weight gain when placed on a high-fat diet. This is in contrast to the action of the PPARγ agonist class of insulin sensitizers, which rather induce weight gain (Murphy & Nolan, *Exp. Opin. Invest. Drugs 9*:1347-1361, 2000), and would suggest that inhibition of PTP-1B could be a particularly attractive option for treatment of obese Type 2 diabetics.

This is also supported by the fact that the heterozygous mice from this study showed many of these desirable features. The reduction in weight gain of the knock-out animals on the high fat diet was found to be due to a decreased fat cell mass, although differences were observed with respect to fat cell number. Leptin levels were also lower in the knock-out mice, presumably as a reflection of the decreased fat mass. Significantly, the Klaman et al group also found that the knockout animals had an increased energy expenditure of around 20% and an increased respiratory quotient compared to the wild-type; again, heterozygote animals displayed an intermediate level of energy expenditure. Therefore, inhibition of this enzyme may be an effective anti-diabetic and perhaps also anti-obesity therapy. Indeed, two recent publications have provided evidence that PTP1B is an important negative regulator of leptin signaling (Zabolotny *et al*., *Developmental Cell 2:* 489-495 (2002); Cheng *et al., Developmental Cell 2:* 497-503 (2002)).

It should also be noted that in the PTP-1B knock-out mice the basal tyrosine phosphorylation level of the insulin receptor tyrosine kinase does not appear to be increased, which is in contrast to the situation after insulin treatment where there is an increased or prolonged phosphorylation. This might indicate that other PTPs are controlling the basic phosphorylation state of the insulin receptor in the knock-out mice - and is expected to do so in man.

Also other PTPases have been implicated as regulators of the insulin-signaling pathway. Thus, it was found that the ubiquitously expressed SH2 domain containing PTPase, PTP1D/SHP-2 (Vogel *et al*., 1993, *supra),* associates with and dephosphorylates IRS-1, but apparently not the IR itself (Kuhné *et al., J. Biol. Chem. 268:* 11479-11481 (1993); (Kuhné *et al., J. Biol. Chem. 269*: 15833-15837 (1994)).

Other studies suggest that receptor-type or membrane-associated PTPases are involved in IRTK regulation (Faure *et al., J. Biol. Chem. 267*: 11215-11221 (1992), (Häring *et al., Biochemistry 23:* 3298-3306 (1984); Sale, *Adv. Prot Phosphatases 6*: 159-186 (1991)).

While previous reports indicate a role of PTPα in signal transduction through src activation (Zheng *et al., Nature 359*: 336-339 (1992); den Hertog *et al., EMBO J. 12*: 3789-3798 (1993)) and interaction with GRB-2 (den Hertog *et al., EMBO J. 13*: 3020-3032 (1994); Su *et al., J. Biol. Chem. 269*: 18731-18734 (1994)), Møller, Lammers and coworkers provided results that suggest a function for this phosphatase and its close relative PTPε as negative regulators of the insulin receptor signal (Møller *et al*., 1995 *supra;* Lammers, *et al., FEBS Lett. 404:* 37-40 (1997). These studies also indicated that receptor-like PTPases may play a significant role in regulating the IRTK, including through direct influence on the insulin receptor itself.

Other studies have shown that PTP1B and TC-PTP are likely to be involved in the regulation of several other cellular processes in addition to the described regulatory roles in insulin signalling. Therefore, PTP1B and/or TC-PTP as well as other PTPases showing key structural features with PTP1B and TC-PTP are likely to be important therapeutic targets in a variety of human and animal diseases. The compounds of the present invention are useful for modulating or inhibiting PTP1B and/or TC-PTP and/or other PTPases showing key structural features with said PTPases and thus elucidating their function and for treating disease states in which said modulation or inhibition is indicated.

Further, PTPases influence the following hormones or diseases or disease states: somatostatin, the immune system/autoimmunity, cell-cell interactions/cancer, platelet aggregation, osteoporosis, and microorganisms, as disclosed in PCT Publication WO 99/15529.

### PTPases: the immune system/autoimmunity

Several studies suggest that the receptor-type PTPase CD45 plays a critical role not only for initiation of T cell activation, but also for maintaining the T cell receptor-mediated signalling cascade. These studies are reviewed in: (Weiss A., *Ann. Rev. Genet. 25*: 487-510 (1991); Chan *et al., Annu. Rev. Immunol. 12*: 555-592 (1994); Trowbridge and Thomas, *Annu. Rev. Immunol. 12*: 85-116 (1994)).

CD45 is one of the most abundant of the cell surface glycoproteins and is expressed exclusively on hemopoetic cells. In T cells, it has been shown that CD45 is one of the critical components of the signal transduction machinery of lymphocytes. In particular, there is evidence that CD45 phosphatase plays a pivotal role in antigen-stimulated proliferation of T lymphocytes after an antigen has bound to the T cell receptor (Trowbridge, *Ann. Rev. Immunol, 12*: 85-116 (1994)). Several studies indicate that the PTPase activity of CD45 plays a role in the activation of Lck, a lymphocyte-specific member of the Src family protein-tyrosine kinase (Mustelin etal., *Proc. Natl. Acad. Sci. USA 86:* 6302-6306 (1989); Ostergaard *et al., Proc. Natl. Acad. Sci. USA 86:* 8959-8963 (1989)). Studies using transgenic mice with a mutation for the CD45-exon6 exhibited a lack of mature T cells. These mice did not respond to an antigenic challenge with the typical T cell mediated response (Kishihara *et al., Cell 74:* 143-56 (1993)). Inhibitors of CD45 phosphatase would therefore be very effective therapeutic agents in conditions that are associated with autoimmune diseases, such as rheumatoid arthritis, systemic lupus erythematosus, type 1 diabetes, and inflammatory bowel disease. Another important function of CD45 phosphatase inhibitors is in effecting immunosuppression, where such a result is indicated, e.g., in transplantation and other conditions in need of immunosuppressive treatment.

CD45 has also been shown to be essential for the antibody-mediated degranulation of mast cells (Berger *et* al., *J. Exp. Med. 180*: 471-476 (1994)). These studies were also done with mice that were CD45-deficient. In this case, an IgE-mediated degranulation was demonstrated in wild type but not CD45-deficient T cells from mice. These data suggest that CD45 inhibitors could also play a role in the symptomatic or therapeutic treatment of allergic disorders, such as asthma, allergic rhinitis, food allergies, eczema, urticaria and anaphylaxis. Another PTPase, an inducible lymphoid-specific protein tyrosine phosphatase (HePTP) has also been implicated in the immune response. This phosphatase is expressed in both resting T and B lymphocytes, but not non-hemopoetic cells. Upon stimulation of these cells, mRNA levels from the HePTP gene increase 10-15 fold (Zanke *et al., Eur. J. Immunol. 22:* 235-239 (1992)).

Likewise, the hematopoietic cell specific SHP-1 acts as a negative regulator and thus appears to play an essential role in immune cell development.In accordance with the above-mentioned important function of CD45, HePTP and SHP-1, selective PTPase inhibitors are early development candidates or prototype drugs both as immunosuppressors and as immunostimulants. Recent studies illustrate the potential of PTPase inhibitors as immunmodulators by demonstrating the capacity of the vanadium-based relatively nonselective PTPase inhibitor, BMLOV, to induce apparent B cell selective apoptosis compared to T cells (Schieven *et al., J. Biol. Chem. 270*: 20824-20831 (1995)).

### PTPases: cell-cell interactions/cancer

Focal adhesion plaques, an *in vitro* phenomenon in which specific contact points are formed when fibroblasts grow on appropriate substrates, mimic, in certain respects, cells and their natural surroundings. Several focal adhesion proteins are phosphorylated on tyrosine residues when fibroblasts adhere to and spread on extracellular matrix (Gumbiner, *Neuron 11:* 551-564 (1993)). However, aberrant tyrosine phosphorylation of these proteins can lead to cellular transformation. The intimate association between PTPases and focal adhesions is supported by the finding of several intracellular PTPases with ezrin-like N-terminal domains, e.g. PTPMEG1 (Gu *et al., Proc. Natl. Acad. Sci. USA 88*: 5867-5871 (1991), PTPH1 (Yang and Tonks, *Proc. Natl. Acad Sci. USA 88*: 5949-5953 (1991)) and PTPD1 (Møller *et al., Proc. Natl. Acad. Sci.* *USA 91*: 7477-7481 (1994)). The ezrin-like domains show similarity to several proteins that are believed to act as links between the cell membrane and the cytoskeleton. PTPD1 was found to be phosphorylated by and associated with c-src *in vitro* and is hypothesized to be involved in the regulation of phosphorylation of focal adhesions (Møller *et al., supra).*

PTPases may oppose the action of tyrosine kinases, including those responsible for phosphorylation of focal adhesion proteins, and may therefore function as natural inhibitors of transformation. TC-PTP, and especially the truncated form of this enzyme (Cool *et al., Proc. Natl. Acad Sci. USA 87:* 7280-7284 (1990)), can inhibit the transforming activity of v-*erb* and v*-fms* (Lammers *et al., J. Biol. Chem. 268:* 22456-22462 (1993), Zander *et al., Oncogene 8:* 1175-1182 (1993)). Moreover, it was found that transformation by the oncogenic form of the *HER2*/*neu* gene was suppressed in NIH 3T3 fibroblasts overexpressing PTP1B (Brown-Shimer *et al., Cancer Res. 52:* 478-482 (1992)).

The expression level of PTP1B was found to be increased in a mammary cell line transformed with *neu* (Zhay *et al., Cancer Res. 53:* 2272-2278 (1993)). The intimate relationship between tyrosine kinases and PTPases in the development of cancer is further evidenced by the recent finding that PTPe is highly expressed in murine mammary tumors in transgenic mice overexpressing c*-neu* and v-Ha-*ras*, but not c-*myc* or *int-2* (Elson and Leder, *J. Biol. Chem. 270:* 26116-26122 (1995)). Further, the human gene encoding PTPγ was mapped to 3p21, a chromosomal region, which is frequently deleted in renal and lung carcinomas (LaForgia *et al*., *Proc. Natl. Acad. Sci. USA 88:* 5036-5040 (1991)).

PTPases appear to be involved in controlling the growth of fibroblasts. In a recent study it was found that Swiss 3T3 cells harvested at high density contain a membrane-associated PTPase whose activity on an average is 8-fold higher than that of cells harvested at low or medium density (Pallen and Tong, *Proc. Natl. Acad. Sci. USA 88*: 6996-7000 (1991)).

Two closely related receptor-type PTPases, PTPκ and PTPµ, can mediate homophilic cell-cell interaction when expressed in non-adherent insect cells, suggesting that a normal physiological function for these PTPases in cell-to-cell signalling (Gebbink *et al., J. Biol. Chem. 268*: 16101-16104 (1993), Brady-Kalnay *et al., J. Cell Biol. 122*: 961-972 (1993); Sap *et al., Mol. Cell. Biol.* *14*: 1-9 (1994)). Interestingly, PTPκ and PTPµ do not bind to each other (PTPκ does self-associate), despite their structural similarity (Zondag *et al., J. Biol. Chem. 270*: 14247-14250 (1995)).

From the studies described above it is apparent that PTPases play an important role in regulating normal cell growth. Additionally, as pointed out above, PTPases may also function as positive mediators of intracellular signaling and thereby induce or enhance mitogenic responses. Increased activity of certain PTPases might therefore result in cellular transformation and tumor formation. See, Zheng, *supra;* Uchida *et al., J. Biol. Chem. 269*: 12220-12228 (1994 Hunter, *Cell 80*: 225-236 (1995). Inhibitors of specific PTPases are therefore likely to be of significant therapeutic value in the treatment of certain forms of cancer.

### PTPases: platelet aggregation

PTPases are centrally involved in platelet aggregation. Thus, agonist-induced platelet activation results in calpain-catalyzed cleavage of PTP1B with a concomitant 2-fold stimulation of PTPase activity (Frangioni *et al., EMBO J. 12:* 4843-4856 (1993)). The cleavage of PTP1B leads to subcellular relocation of the enzyme and correlates with the transition from reversible to irreversible platelet aggregation in platelet-rich plasma. In addition, the SH2 domain containing PTPase, SHP-1, was found to translocate to the cytoskeleton in platelets after thrombin stimulation in an aggregation-dependent manner (U *et al., FEBS Lett. 343:* 89-93 (1994)).

Although some details in the above two studies have been questioned, there is overall agreement that PTP1B and SHP-1 play significant functional roles in platelet aggregation (Ezumi *et al., J. Biol. Chem. 270:* 11927-11934 (1995)). In accordance with these observations, treatment of platelets with the PTPase inhibitor pervanadate leads to significant increase in tyrosine phosphorylation, secretion and aggregation (Pumiglia *et al., Biochem. J. 286:* 441-449 (1992)).

### PTPases: osteoporosis

The number and the activity of osteoblasts determine the rate of bone formation. In tum, these are determined by the rate of proliferation and differentiation of osteoblast progenitor cells, respectively. Histomorphometric studies indicate that the osteoblast number is the primary determinant of the rate of bone formation in humans (Gruber *et al., Mineral Electrolyte Metab. 12*: 246-254 (1987), reviewed in Lau *et al., Biochem. J. 257:* 23-36 (1989)). Acid phosphatases/PTPases are implicated in negative regulation of osteoblast proliferation. Thus, fluoride, which has phosphatase inhibitory activity, has been found to increase spinal bone density in osteoporotics by increasing osteoblast proliferation (Lau *et al*., *supra*). Consistent with this observation, an osteoblastic acid phosphatase with PTPase activity was found to be highly sensitive to mitogenic concentrations of fluoride (Lau *et al., J. Biol. Chem. 260*: 4653-4660 (1985), Lau *et al., J. Biol. Chem. 262*: 1389-1397 (1987), Lau *et al., Adv. Protein Phosphatases 4*: 165-198 (1987)). The mitogenic action of fluoride and other phosphatase inhibitors (molybdate and vanadate) may thus be explained by their inhibition of acid phosphatases/PTPases that negatively regulate the cell proliferation of osteoblasts. The complex nature of the involvement of PTPases in bone formation is further suggested by the recent identification of a novel parathyroid regulated, receptor-like PTPase, OST-PTP, expressed in bone and testis (Mauro *et al., J. Biol. Chem. 269*: 30659-30667 (1994)). OST-PTP is up-regulated following differentiation and matrix formation of primary osteoblasts and subsequently down-regulated in the osteoblasts which are actively mineralizing bone in culture. In addition, it was recently observed that vanadate, vanadyl and pervanadate all increased the growth of the osteoblast-like cell line UMR106. Vanadyl and pervanadate were stronger stimulators of cell growth than vanadate. Only vanadate was able to regulate the cell differentiation as measured by cell alkaline phosphatase activity (Cortizo *et al., Mol. Cell. Biochem. 145:* 97-102 (1995)). More important, several studies have shown that biphosphonates, such as alendronate and tiludronate, inhibit PTPase activity in osteoclasts and that the inhibition of PTPase activity correlated with the inhibition of *in vitro* osteoclast formation and bone resorption (Scmidt, et al., *Proc. Nail Acad. Sci. U.S.A. 93*: 3068-3073, (1996); Murakami et al., *Bone 20*: 399-404, (1997); Opas et al., *Biochem. Pharmacol. 54:* 721-727, (1997); Skorey et al., *J. Biol. Chem. 272*: 22472-22480, (1997)). Thus, other PTPase inhibitors are potentially effective in countering osteoclast activity, and thus treating osteoporosis.

### PTPases: microorganisms

Dixon and coworkers have called attention to the fact that PTPases may be a key element in the pathogenic properties of *Yersinia* (reviewed in Clemens *et* *al. Molecular Microbiology 5*: 2617-2620 (1991)). This finding was rather surprising since tyrosine phosphate is thought to be absent in bacteria. The genus *Yersinia* comprises 3 species: *Y*. *pestis* (responsible for the bubonic plague), *Y. pseudoturberculosis* and *Y. enterocolitica* (causing enteritis and mesenteric lymphadenitis). A dual-specificity phosphatase, VH1, has been identified in Vaccinia virus (Guan *et al., Nature 350*: 359-263 (1991)). These observations indicate that PTPases may play critical roles in microbial and parasitic infections, and they further point to PTPase inhibitors as a novel, putative treatment principle of infectious diseases. Availibility of PTPase inhibitors would help shed light in all the foregoing specualations about PTPase function because they would enable assaying techniques which would answer some of these questions as will be illustrated below.

### PTP1B: Leptine

Recently two detailed studies on PTP1B knockout mice clearly pointed to a role of PTP1B in maintaining glucose homeostasis (Elchebly *et al. Science 283*: 1544-1548 (1999); Klaman *et al*. *Mol. Cell. Biol*. 20: 5479-5489 (2000)). Concomitant increased insulin sensitivity and a prolonged tyrosine phosphorylation of the insulin receptor tyrosine kinase (IRTK) in these mice suggest a direct action of PTP1B on the IRTK itself. Further, treatment of *ob*/*ob* mice and *db*/*db* mice for four weeks with PTP1B antisense oligonucleotides (ASOs) caused significant reduction in blood glucose levels to near normal values (personal communication, Brett Monia, ISIS Pharmaceuticals). Surprisingly, resistance to diet-induced obesity was observed both in the PTP1B knockout and the PTP1B ASO studies. In the case of PTP1B knockout mice, increased energy expenditure was observed (Klaman *et al. Mol. Cell. Biol. 20:* 5479-5489 (2000)).

While the above effects on the blood glucose levels are consistent with a direct role for PTP1B in regulating the insulin receptor signalling pathway, it seems unlikely that the increased energy expenditure and resistance to diet-induced weight gain is related to an effect on insulin signalling. Indeed, an increase in body weight would actually be expected due to the anabolic effects of insulin. Other effects of PTP1B are therefore likely to be at play. An increase in energy expenditure would be consistent with a central effect on leptin-signalling. In agreement with this hypothesis, Neel and coworkers observed increased tyrosine phosphorylation levels of specific proteins in the hypothalamus of PTP1B knockout mice (B.G. Neel, personal communication). However, a central effect on the leptin signal pathway is less conceivable to be the mechanism underlying the resistance to diet-induced weight gain in normal mice after treatment with PTP1B ASOs, since the oligonucleotides may not cross the blood brain barrier. It is likely that PTP1B could regulate peripheral leptin signalling. Recent studies indicate that leptin also induces insulin-like signalling in hepatocytes and a hepatic cell line (Szanto *et al*. *Proc. Natl. Acad. Sci. U.S.A. 97*: 2355-2360 (2000); Zhao *et al. J. Biol. Chem. 275:* 11348-11354 (2000)). Theoretically, PTP1B could regulate leptin signalling both at the Janus kinase (JAK) and the STAT levels. Indeed, PTP1B has been found to dephosphorylate and deactivate prolactin-activated STAT5a and STAT5b in transfected COS cells (Aoki *et al*. *J. Biol. Chem. 275:* 39718-39726 (2000)). Further, in the context of JAK/STAT signaling it is intriguing that JAKs, similar to the IRTK, contain two adjacent tyrosine residues in the activation loop (corresponding to tyrosine residues 1162 and 1163 of the insulin receptor). It was recently shown by Tonks, Barford and their coworkers that these double phosphorylated residues (pTyr) are extremely good targets for PTP1B. This is due to simultaneous binding to the active site and a second aryl phosphate binding site, which seems to be rather unique for PTP1B (Puius *et al*. *Proc. Natl. Acad. Sci. U.S.A 94*: 13420-13425 (1997)). Therefore, both the IRTK and the JAKs with the adjacent pTyr residues could be natural substrates for PTP1B.

Based on the above, selective inhibitors of PTP1B could be very useful for simultaneous treatment of several defects in type 2 diabetes: (1) insulin resistance; (2) dyslipidemia; and (3) obesity.

It has been found that PTPases play a major role in the above modulation and regulation of fundamental cellular signaling mechanisms involved in metabolism, growth, proliferation and differentiation (Fisher *et al*, *Science 253:* 401-406 (1991); Tonks and Neel, *Cell 87*: 365-368 (1966)" Neel and Tonks, *Current Opinion in Cell Biology 9*: 193-204 (1997); Hunter, *Phil. Trans. R. Soc. Lond. B 353*: 583-605 (1998); Hunter, *Cell 100*: 113-120 (2000); Zhang, *Critical Reviews in Biochemistry and Molecular Biology* 33:1-52 (1988)). Reports from many laboratories have shown that PTPases can act both as positive and negative regulators of signal transduction processes. PTPases have been implicated in a variety of human diseases, including diabetes, obesity, autoimmune diseases, acute and chronic inflammation, osteoporosis, proliferative disorders including various forms of cancer, growth disorders, and defective platelet aggregation (WO97/39748, WO97/40017, WO99/1529, WO97/08934, WO98/27065, WO99/46236, WO99/46244, WO99146267, WO99/46268, WO99/46237). Accordingly there is increasing evidence which suggests that inhibition of these PTPases would help treat or manage these diseases (Hunter, *vide supra;* Neel and Tonks, *vide supra:* Frangione et al., *EMBO J. 12*: 4843-4856 (1993); Zhang, *Curr. Top. Cell. Reg. 35*.21-68 (1997): Zhang, *vide supra;* Evans and Jalian, *Exp. Opinion. Invest. Drugs 8:* 139-160 (1999); Burke and Zhang, *Bioploymers (Peptide Science) 47*: 225-241 (1998): Elchebly *et al.; Science 283*: 1544-1548 (1999); Wrobel *et al., J. Med. Chem. 42*: 3199-3202 (1999)). In addition, certain infectious diseases may also be treated or managed by administration PTPase inhibitors (Clemens *et al*., *Molecular Microbiology 5:* 2617-2620 (1991)).

Both selective PTPase inhibitors and inhibitors that bind to several PTPases (non-selective inhibitors) can be used therapeutically to partially or completely restore PTPase-mediated perturbed signal transduction processes and thus for management, treatment, palliation or prevention of the above diseases.
PTPase inhibitors are known e.g. from WO 99/46267. However, there is a need for PTPase inhibitors with increased potency and selectivity.

The compounds of Formula 1 are oxalylamide compounds having in common key structural features required of non hydrolysable protein tyrosine phosphatase inhibitors, most particularly PTP1B and/or TC-PTP inhibitors. These structural features endow the present compounds with the appropriate molecular shape necessary to fit into the enzymatic active site, to bind to such site in a non covalently way, thereby blocking the site and inhibiting enzymatic biological activity. Referring to Formula 1, such structural features include the oxalylamide and an ortho-carboxylic acid attached to a hydrophobic group, preferably an aryl as defined below.
The compounds of the invention can be further modified to act as pro-drugs.

The present invention relates to compounds of Formula 1

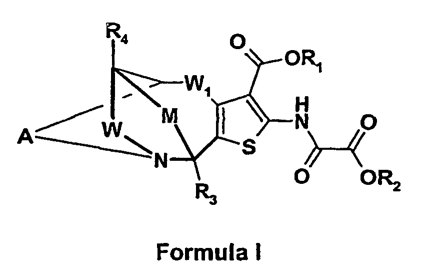

Wherein
R₁ and R₂ are independently hydrogen or a functional group that can be converted to hydrogen *in vivo;*
R₃ and R₄ are independently hydrogen, C₁-C₁₀alkyl, C₂-C₁₀alkenyl, C₂-C₁₀alkynyl, aryl, arylC₁-C₁₀alkyl, arylC₁-C₁₀alkenyl, C₁-C₁₀alkyloxyC₁-C₁₀alkyl, aryloxyC₁-C₁₀alkyl, arylC₁-C₁₀alkyloxyC₁-C₁₀alkyl, C₁-C₁₀alkylthioC₁-C₁₀alkyl, C₁-C₁₀alkyl-aminoC₁-C₁₀alkyl, arylC₁-C₁₀alkyl-aminoC₁-C₁₀alkyl, di(arylC₁-C₁₀alkyl)-aminoC₁-C₁₀alkyl, C₁-C₁₀alkylcarbonyl-aminoC₁-C₁₀alkyl, arylC₁₋C₁₀alkyl-carbonylaminoC₁-C₁₀alkyl, CONR₅R₆, wherein the alkyl, alkenyl, alkynyl and aryl groups are optionally substituted by one or more cyano, nitro, halo, hydroxy, trihalomethyl, C₁-C₁₀alkyl, C₁-C₁₀alkoxy, or aryl independently;
R₅ and R₆ are independently selected from hydrogen, C₁-C₁₀alkyl, C₂-C₁₀alkenyl, C₂-C₁₀alkynyl, aryl, arylC₁-C₁₀alkyl, C₁-C₁₀alkylcarbonyl, C₁-C₁₀alkyloxocarbonyl, arylcarbonyl, aryloxocarbonyl, arylC₁-C₁₀alkyl-carbonyl, arylC₁-C₁₀alkyloxocarbonyl, wherein the alkyl, alkenyl, alkynyl, and aryl groups are optionally substituted by one or more cyano, nitro, halo, hydroxy, trihalomethyl, C₁-C₁₀alkyl, C₁-C₁₀alkoxy, or aryl independently; or
R₅ and R₆ may form a saturated, partially saturated or aromatic cyclic, bicyclic or tricyclic ring system containing from 3 to 14 carbon atoms and from 0 to 3 additional heteroatoms selected from nitrogen, oxygen or sulphur with the nitrogen to which they are attached, the ring system can optionally be substituted with at least one C₁-C₁₀alkyl, C₂-C₁₀alkenyl, C₂-C₁₀alkynyl, aryl, arylC₁-C₁₀alkyl, hydroxy, oxo, C₁-C₁₀alkyloxy, arylC₁-C₁₀alkyl-oxy, C₁-C₁₀alkyloxyC₁-C₁₀alkyl, NR₇R₈ or C₁-C₁₀alkylaminoC₁-C₁₀alkyl, wherein R₇ and R₈ are independently selected from hydrogen, C₁-C₁₀alkyl, C₂-C₁₀alkenyl, C₂-C₁₀alkynyl, aryl, arylC₁-C₁₀alkyl, C₁-C₁₀alkyl-carbonyl, arylcarbonyl, arylC₁-C₁₀alkylcarbonyl, C₁-C₁₀alkylcarboxy or arylC₁-C₁₀alkyl-carboxy; wherein the alkyl, alkenyl, alkynyl, and aryl groups are optionally substituted by one or more cyano, nitro, halo, hydroxy, trihalomethyl, C₁-C₁₀alkyl, C₁-C₁₀alkoxy, or aryl independently ; or
R₅ and R₁₀ are independently a saturated or partial saturated cyclic 5, 6 or 7 membered amine, imide or lactam;
A is absent or -[C(R₉R₁₀)]ᵢ-, -[C(R₁₁R₁₂)]ⱼ-C(R₁₃)=C(R₁₄)-[C(R₁₅R₁₆)]ₖ-, -[C(R₁₇R₁₈)]_{y}-(X)-[C(R₁₉R₂₀)]_{z}-; wherein X is O, NR₂₁ or S; i is 1, 2, 3 or 4; y and z are independently 0, 1, 2 or 3; j and k are independently 0, 1 or 2;
M is absent or -[C(R₂₉R₃₀)]ₚ-; wherein p is 1, 2 or 3;
With the proviso that A and M cannot both be absent;
W is a valence bond or -[C(R₃₁R₃₂)]_{q}-; wherein q is 1 or 2;
W₁ is a valence bond or -[C(R₃₃R₃₄)]_{qq}; wherein qq is 1 or 2;
R₉, R₁₀, R₁₁, R₁₂, R₁₃, R₁₄, R₁₅, R₁₆, R₁₈, R₁₉, R₂₀, R₂₁ R₃₁, R₃₂, R₃₃ and R₃₄ are independently selected from hydrogen, C₁-C₄alkyl, C₂-C₁₀alkenyl, C₂-C₁₀alkynyl, aryl, and arylC₁-C₄alkyl; wherein the alkyl, alkenyl, alkynyl, and aryl groups are optionally substituted by one or more cyano, nitro, halo, hydroxy, trihalomethyl, C₁-C₁₀alkyl, C₁-C₁₀alkoxy, or aryl independently;
R₂₁, R₂₄, R₂₅, R₂₆, R₂₇, and R₂₈ are independently selected from hydrogen, C₁-C₁₀alkyl, C₂-C₁₀alkenyl, C₂-C₁₀alkynyl, aryl, or arylC₁-C₁₀alkyl; wherein the alkyl, alkenyl, alkynyl, and aryl groups are optionally substituted by one or more cyano, nitro, halo, hydroxy, trihalomethyl, C₁-C₁₀alkyl, C₁-C₁₀alkoxy, or aryl independently;
or a salt thereof with a pharmaceutically acceptable acid or base, or any optical isomer or mixture of optical isomers, including a racemic mixture, or any tautomeric forms.

In a preferred embodiment R₁ and R₂ are independently hydrogen, C₁-C₁₀alkyl, C₂-C₁₀alkenyl, C₂-C₁₀alkynyl, aryl, arylC₁-C₁₀alkyl, C₁-C₁₀alkyloxy, C₁-C₁₀alkyloxyC₁-C₁₀alkyloxy, aryloxy, and arylC₁-C₁₀alkyloxy; wherein the alkyl, alkenyl, alkynyl and aryl groups are optionally substituted by one or more cyano, nitro, halo, hydroxy, trihalomethyl, C₁-C₁₀alkyl, C₁-C₁₀alkoxy, or aryl independently;
In another preferred embodiment A is absent or -[C(R₉R₁₀)]ᵢ-, -[C(R₁₁R₁₂)]ⱼ-C(R₁₃)=C(R₁₄)-[C(R₁₅R₁₆)]ₖ-; wherein i is 1, 2, 3 or 4; j and k are independently 0, 1 or 2;

In another preferred embodiment A is -[C(R₉R₁₀)]ᵢ-, wherein i is 1, 2, 3 or 4.

In another preferred embodiment M is -[C(R₂₉R₃₀)]ₚ-; wherein p is 1, 2 or 3.

In another preferred embodiment M is absent.

In another preferred embodiment W is a valence bond.

In another preferred embodiment W is -[C(R₃₁R₃₂)]_{q}-; wherein q is 1 or 2;

In another preferred embodiment W₁ is a valence bond.

In another preferred embodiment W₁ is -[C(R₃₃R₃₄)]_{qq}; wherein qq is 1 or 2;

In another preferred embodiment R₁ is hydrogen, C₁-C₁₀alkyl, C₂-C₁₀alkenyl, C₂-C₁₀alkynyl, aryl, arylC₁-C₁₀alkyl, C₁-C₁₀alkyloxy; wherein the alkyl, alkenyl, alkynyl and aryl groups are optionally substituted by one or more cyano, nitro, halo, hydroxy, trihalomethyl, C₁-C₁₀alkyl, C₁-C₁₀alkoxy, or aryl independently.
In another preferred embodiment R₁ is hydrogen, C₁-C₁₀alkyl, C₂-C₁₀alkenyl, C₂-C₁₀alkynyl, arylC₁-C₁₀alkyl or aryl; wherein the alkyl, alkenyl, alkynyl and aryl groups are optionally substituted by one or more cyano, nitro, halo, hydroxy, trihalomethyl, C₁-C₁₀alkyl, C₁-C₁₀alkoxy, or aryl independently.

In another preferred embodiment R₁ is hydrogen, C₁-C₁₀alkyl, or arylC₁-C₁₀alkyl; wherein the alkyl groups are optionally substituted by one or more cyano, nitro, halo, hydroxy, trihalomethyl, C₁-C₁₀alkyl, C₁-C₁₀alkoxy, or aryl independently.

In another preferred embodiment R₁ is hydrogen or C₁-C₁₀alkyl wherein the alkyl group is optionally substituted by one or more cyano, nitro, halo, hydroxy, trihalomethyl, C₁-C₁₀alkyl, C₁-C₁₀alkoxy, or aryl independently..

In another preferred embodiment R₂ is hydrogen, C₁-C₁₀alkyl, C₂-C₁₀alkenyl, C₂-C₁₀alkynyl, aryl, arylC₁-C₁₀alkyl, C₁-C₁₀alkyloxy; wherein the alkyl, alkenyl, alkynyl and aryl groups are optionally substituted by one or more cyano, nitro, halo, hydroxy, trihalomethyl, C₁-C₁₀alkyl, C₁-C₁₀alkoxy, or aryl independently.

In another preferred embodiment R₂ is hydrogen, C₁-C₁₀alkyl, C₂-C₁₀alkenyl, C₂-C₁₀alkynyl, aryl; wherein the alkyl, alkenyl, alkynyl and aryl groups are optionally substituted by one or more cyano, nitro, halo, hydroxy, trihalomethyl, C₁-C₁₀alkyl, C₁-C₁₀alkoxy, or aryl independently.

In another preferred embodiment R₂ is hydrogen or C₁-C₁₀alkyl; wherein the alkyl group is optionally substituted by one or more cyano, nitro, halo, hydroxy, trihalomethyl, C₁-C₁₀alkyl, C₁-C₁₀alkoxy, or aryl independently.

In another preferred embodiment R₂ is hydrogen or C₁-C₁₀alkyl.

In another preferred embodiment R₃ is hydrogen, C₁-C₁₀alkyl, C₂-C₁₀alkenyl, C₂-C₁₀alkynyl, aryl, arylC₁-C₁₀alkyl, arylC₁-C₁₀alkenyl, aryloxyC₁-C₁₀alkyl, C₁-C₁₀alkylthioC₁-C₁₀alkyl, or arylC₁-C₁₀alkyloxyC₁-C₁₀alkyl, wherein the alkyl, alkenyl, alkynyl and aryl groups are optionally substituted by one or more cyano, nitro, halo, hydroxy, trihalomethyl, C₁-C₁₀alkyl, C₁-C₁₀alkoxy, or aryl independently.

In another preferred embodiment R₃ is hydrogen, C₁-C₁₀alkyl, C₂-C₁₀alkenyl, C₂-C₁₀alkynyl, aryl, arylC₁-C₁₀alkyl, C₁-C₁₀alkylthioC₁-C₁₀alkyl, or arylC₁-C₁₀alkenyl, wherein the alkyl, alkenyl, alkynyl and aryl groups are optionally substituted by one or more cyano, nitro, halo, hydroxy, trihalomethyl, C₁-C₁₀alkyl, C₁-C₁₀alkoxy, or aryl independently.

In another preferred embodiment R₃ is hydrogen or C₁-C₁₀alkyl, wherein the alkyl group is optionally substituted by one or more cyano, nitro, halo, hydroxy, trihalomethyl, C₁-C₁₀alkyl, C₁-C₁₀alkoxy, or aryl independently.

In another preferred embodiment R₃ is hydrogen or C₁-C₁₀alkyl.

In another preferred embodiment R₃ is C₁-C₁₀alkyl, arylC₁-C₁₀alkyl, or arylC₁-C₁₀alkenyl wherein aryl is phenyl, biphenyl, thienyl, furanyl, pyrrazolyl, pyridyl, naphthyl, quinolyl, isoquinolyl, indolyl, benzofuranyl, or carbazolyl.

In another preferred embodiment R₄ is hydrogen, C₁-C₁₀alkyl, C₂-C₁₀alkenyl, C₂-C₁₀alkynyl, aryl, arylC₁-C₁₀alkyl, aryloxyC₁-C₁₀alkyl, or arylC₁-C₁₀alkyloxyC₁-C₁₀alkyl, wherein the alkyl, alkenyl, alkynyl and aryl groups are optionally substituted by one or more cyano, nitro, halo, hydroxy, trihalomethyl, C₁-C₁₀alkyl, C₁-C₁₀alkoxy, or aryl independently.

In another preferred embodiment R₄ is hydrogen, C₁-C₁₀alkyl, C₂-C₁₀alkenyl, C₂-C₁₀alkynyl, aryl, or arylC₁-C₁₀alkyl, wherein the alkyl, alkenyl, alkynyl and aryl groups are optionally substituted by one or more cyano, nitro, halo, hydroxy, trihalomethyl, C₁-C₁₀alkyl, C₁-C₁₀alkoxy, or aryl independently.

In another preferred embodiment R₄ is hydrogen or C₁-C₁₀alkyl, wherein the alkyl group is optionally substituted by one or more cyano, nitro, halo, hydroxy, trihalomethyl, C₁-C₁₀alkyl, C₁-C₁₀alkoxy, or aryl independently.

In another preferred embodiment R₄ is hydrogen or C₁-C₁₀alkyl.

In another preferred embodiment R₉ or R₁₀ are independently selected from hydrogen, C₁-C₄alkyl or aryl, wherein the alkyl and aryl groups are optionally substituted by one or more cyano, nitro, halo, hydroxy, trihalomethyl, C₁-C₁₀alkyl, C₁-C₁₀alkoxy, or aryl independently.

In another preferred embodiment R₉ or R₁₀ are independently selected from hydrogen, C₁-C₄alkyl or aryl.

The following compounds are preferred:
2-(Oxalyl-amino)-9*H*-4,5,6,7-tetrahydro-4,8-methano-thieno[2,3-c]azocine-3-carboxylic acid;
2-(Etoxyoxalyl-amino)-9*H*-4,5,6,7-tetrahydro-4,8-methano-thieno[2,3-c]azocine-3-carboxylic acid ethyl ester;
2-(Oxalyl-amino)-9*H*-4,5,6,7-tetrahydro-4,8-methano-thieno[2,3-c]azocine-3-carboxylic acid ethyl ester;
2-(Oxalyl-amino)-9*H*-4,7-dihydro-4,8-methano-benzo[f]thieno[2,3-c]azocine-3-carboxylic acid;
2-(Oxatyl-amino)-4,5,6,8-tetrahydro-4,7-methano-thieno[2,3-c]azepine-3-carboxylic acid;
9-Methyl-2-(oxalyl-amino)-4,5,6,7-tetrahydro-4,8-methano-thieno[2,3-c]azocine-3-carboxylic acid;
2-(Oxalyl-amino)-6-phenyl-9*H*-4,5,6,7-tetrahydro-4,8-methano-thieno[2,3-c]azocine-3-carboxylic acid;
7-Methyl-2-(oxalyl-amino)-9*H*-4,5,6,7-tetrahydro-4,8-methano-thieno[2,3-c]azocine-3-carboxylic acid;
2-(Oxalyl-amino)-4,5,6,8-tetrahydro-4,7-ethano-thieno[2,3-c]azepine-3-carboxylic acid;
2-(Oxalyl-amino)-4,5,8,10-tetrahydro-4,9-methano-benzo[g]thieno[2,3-c]azonine-3-carboxylic acid;
2-(Oxalyl-amino)-5,7-ethano-4,5,6,7-tetrahydro-thieno[2,3-c]pyridine-3-carboxylic acid;
2-(Oxalyl-amino)-1,3,4,6-tetrahydro-4,8-methano-thieno[2,3-f][1,4]-oxazocine-3-carboxylic acid;
2-(Oxalyl-amino)-9-phenethyl-4,5,6,7-tetrahydro-4,8-methano-thieno[2,3-c]azocine-3-carboxylic acid;
2-(Oxalyl-amino)-7-phenethyl-9*H*-4,5,6,7-tetrahydro-4,8-methano-thieno[2,3-c]azocine-3-carboxylic acid;
2-(Oxalyl-amino)-9-phenethyl-9*H*-4,5,6,7-tetrahydro-4,8-methano-thieno[2,3-c]azocine-3-carboxylic acid;
2-(Oxalyl-amino)-5-phenyl-9*H*-4,5,6,7-tetrahydro-4,8-methano-thieno[2,3-c]azocine-3-carboxylic acid;
2-(Oxalyl-amino)-10-phenethyl-9*H*-4,5,6,7-tetrahydro-4,8-methano-thieno[2,3-c]azocine-3-carboxylic acid;
9-Hepthyl-2-(oxalyl-amino)-4,5,6,7-tetrahydro-4,8-methano-thieno[2,3-c]azocine-3-carboxylic acid;
9-Hepthyl-2-(oxalyl-amino)-4,5,6,7-tetrahydro-4,8-methano-thieno[2,3-c]azocine-3-carboxylic acid;
2-(Oxalyl-amino)-4,5,8,10-tetrahydro-4,9-methano-naphtho[2,3-g]thieno[2,3-c]azonine-3-carboxylic acid;
2-(Oxalyl-amino)-7-phenyl-9*H*-4,5,6,7-tetrahydro-4,8-methano-thieno[2,3-c]azocine-3-carboxylic acid;
2-(Oxalyl-amino)-9-pentyl-4,5,6,7-tetrahydro-4,8-methano-thieno[2,3-c]azocine-3-carboxylic acid;
9-(2-Cyclohexyl-ethyl)-2-(oxalyl-amino)-4,5,6,7-tetrahydro-4,8-methano-thieno[2,3-c]azocine-3-carboxylic acid;
9-(2-Methylsulfanyl-ethyl)-2-(oxalyl-amino)-4,5,6,7-tetrahydro-4,8-methano-thieno[2,3-c]azocine-3-carboxylic acid;
9-(Ethyl)-2-(oxalyl-amino)-4,5,6,7-tetrahydro-4,8-methano-thieno[2,3-c]azocine-3-carboxylic acid;
2-(Oxalyl-amino)-9-(propyl)-4,5,6,7-tetrahydro-4,8-methano-thieno[2,3-c]azocine-3-carboxylic acid;
2-(*tert*-Butoxyoxalylamino)-4,5,6,7-tetrahydro-4,8-methano-thieno[2,3-c]azocine-3-carboxylic acid ethyl ester;
2-(Isopropoxyoxalylamino)-4,5,6,7-tetrahydro-4,8-methano-thieno[2,3-c]azocine-3-carboxylic acid ethyl ester;
2-(Benzoxyoxalylamino)-4,5,6,7-tetrahydro-4,8-methano-thieno[2,3-c]azocine-3-carboxylic acid ethyl ester;
2-(Benzoxyoxalylamino)-4,5,6,7-tetrahydro-4,8-methano-thieno[2,3-c]azocine-3-carboxylic acid benzyl ester;
2-(*tert*-Butoxyoxalylamino)-4,5,6,7-tetrahydro-4,8-methano-thieno[2,3-c]azocine-3-carboxylic acid benzyl ester;
2-(*tert*-Butoxyoxalylamino)-9-pentyl-4,5,6,7-tetrahydro-4,8-methano-thieno[2,3-c]azocine-3-carboxylic acid benzyl ester;
2-(*tert*-Butoxyoxalylamino)-9-pentyl-4,5,6,7-tetrahydro-4,8-methano-thieno[2,3-c]azocine-3-carboxylic acid ethyl ester;
2-(Oxalyl-amino)-9-(octyl)-4,5,6,7-tetrahydro-4,8-methano-thieno[2,3-c]azocine-3-carboxylic acid;
2-(Oxalyl-amino)-9-(4-phenyl-butyl))-4,5,6,7-tetrahydro-4,8-methano-thieno[2,3-c]azocine-3-carboxylic acid;
2-(Oxalyl-amino)-9-(2-cyclopentyl-ethyl)-4,5,6,7-tetrahydro-4,8-methano-thieno[2,3-c]azocine-3-carboxylic acid;
9-(3-Methyl-butyl)-2-(oxalyl-amino)-4,5,6,7-tetrahydro-4,8-methano-thieno[2,3-c]azocine-3-carboxylic acid;
2-(Oxalyl-amino)-9-(4-phenyl-2-methyl-butyl)-4,5,6,7-tetrahydro-4,8-methano-thieno[2,3-c]azocine-3-carboxylic acid;
2-(*tert*-Butoxyoxalylamino)-4,5,6,7-tetrahydro-4,8-methano-thieno[2,3-c]azocine-3-carboxylic acid ethyl ester;
2-(Isopropoxyoxalylamino)-4,5,6,7-tetrahydro-4,8-methano-thieno[2,3-c]azocine-3-carboxylic acid ethyl ester;
2-(*tert*-Butoxyoxalylamino)-4,5,6,7-tetrahydro-4,8-methano-thieno[2,3-c]azocine-3-carboxylic acid benzyl ester;
2-(*tert*-Butoxyoxalylamino)-9-pentyl-4,5,6,7-tetrahydro-4,8-methano-thieno[2,3-c]azocine-3-carboxylic acid benzyl ester;
2-(*tert*-Butoxyoxalylamino)-9-pentyl-4,5,6,7-tetrahydro-4,8-methano-thieno[2,3-c]azocine-3-carboxylic acid ethyl ester;
9-(3-Cyclohexyl-propyl)-2-(oxalyl-amino)-4,5,6,7-tetrahydro-4,8-methano-thieno[2,3-c]azocine-3-carboxylic acid;
9-Pentyl-2-(*iso*-propoxyoxalyl-amino)-4,5,6,7-tetrahydro-4,8-methano-thieno[2,3-c]azocine-3-carboxylic acid *tert*-butyl ester;
9-Pentyl-2-(*iso*-propoxyoxalyl-amino)-4,5,6,7-tetrahydro-4,8-methano-thieno[2,3-c]azocine-3-carboxylic acid;
9-(3-Cyclohexyl-propyl)-2-(oxalyl-amino)-4,5,6,7-tetrahydro-4,8-methano-thieno[2,3-c]azocine-3-carboxylic acid
9-Pentyl-2-(*iso*-propoxyoxalyl-amino)-4,5,6,7-tetrahydro-4,8-methano-thieno[2,3-c]azocine-3-carboxylic acid *tert*-butyl ester
9-Pentyl-2-(*iso*-propoxyoxalyl-amino)-4,5,6,7-tetrahydro-4,8-methano-thieno[2,3-c]azocine-3-carboxylic acid
or a pharmaceutically acceptable salt thereof.

Another aspect of the invention is a pharmaceutical composition comprising a compound of the invention or a pharmaceutically acceptable salt thereof with a pharmaceutically acceptable acid or base, or any optical isomer or mixture of optical isomers, including a racemic mixture, or any tautomeric form together with one or more pharmaceutically acceptable carriers or diluents.

Another aspect of the invention is a pharmaceutical composition suitable for treating type 1 diabetes, type 2 diabetes, impaired glucose tolerance, insulin resistance or obesity comprising a compound of the invention or a pharmaceutically acceptable salt thereof with a pharmaceutically acceptable acid or base, or any optical isomer or mixture of optical isomers, including a racemic mixture, or any tautomeric form together with one or more pharmaceutically acceptable carriers or diluents.

Another aspect of the invention is a pharmaceutical composition suitable for treating immune dysfunctions including autoimmunity, diseases with dysfunctions of the coagulation system, allergic diseases, osteoporosis, proliferative disorders including cancer and psoriasis, diseases with decreased or increased synthesis or effects of growth hormone, diseases with decreased or increased synthesis of hormones or cytokines that regulate the release of/or response to growth hormone, diseases of the brain including Alzheimer's disease and schizophrenia, and infectious diseases comprising a compound of the invention or a pharmaceutically acceptable salt thereof with a pharmaceutically acceptable acid or base, or any optical isomer or mixture of optical isomers, including a racemic mixture, or any tautomeric form together with one or more pharmaceutically acceptable carriers or diluents.

Another aspect of the invention is a pharmaceutical composition of the invention in the form of an oral dosage unit or parenteral dosage unit.

Another aspect of the invention is a pharmaceutical composition of the invention wherein said compound is administered as a dose in a range from about 0.05 to 1000 mg, preferably from about 0.1 to 500 mg and especially in the range from 50 to 200 mg per day.
Another aspect of the invention is a compound of the invention or a pharmaceutically acceptable salt thereof with a pharmaceutically acceptable acid or base, or any optical isomer or mixture of optical isomers, including a racemic mixture, or any tautomeric form for therapeutical use.

Another aspect of the invention is a compound of the invention or a pharmaceutically acceptable salt thereof with a pharmaceutically acceptable acid or base, or any optical isomer or mixture of optical isomers, including a racemic mixture, or any tautomeric form for therapeutical use in the treatment, management or prevention of type 1 diabetes, type 2 diabetes, impaired glucose tolerance, insulin resistance, leptin resistance and/or obesity.

Another aspect of the invention is a compound of the invention or a pharmaceutically acceptable salt thereof with a pharmaceutically acceptable acid or base, or any optical isomer or mixture of optical isomers, including a racemic mixture, or any tautomeric form for therapeutical use in the treatment or preventing of immune dysfunctions including autoimmunity, diseases with dysfunctions of the coagulation system, allergic diseases, osteoporosis, proliferative disorders including cancer and psoriasis, diseases with decreased or increased synthesis or effects of growth hormone, diseases with decreased or increased synthesis of hormones or cytokines that regulate the release of/or response to growth hormone, diseases of the brain including Alzheimer's disease and schizophrenia, and infectious diseases.

Another aspect of the invention is the use of a compound of the invention or a pharmaceutically acceptable salt thereof with a pharmaceutically acceptable acid or base, or any optical isomer or mixture of optical isomers, including a racemic mixture, or any tautomeric form as a medicament.

Another aspect of the invention is the use of a compound of the invention for preparing a medicament.

Another aspect of the invention is the use of a compound of the invention or a pharmaceutically acceptable salt thereof with a pharmaceutically acceptable acid or base, or any optical isomer or mixture of optical isomers, including a racemic mixture, or any tautomeric form for the preparation of a medicament suitable for the the treatment, management or prevention of type 1 diabetes, type 2 diabetes, impaired glucose tolerance, insulin resistance, leptin resistance and/or obesity.

Another aspect of the invention is the use of a compound of the invention or a pharmaceutically acceptable salt thereof with a pharmaceutically acceptable acid or base, or any optical isomer or mixture of optical isomers, including a racemic mixture, or any tautomeric form for the preparation of a medicament suitable for the treatment or preventing of immune dysfunctions including autoimmunity, diseases with dysfunctions of the coagulation system, allergic diseases, osteoporosis, proliferative disorders including cancer and psoriasis, diseases with decreased or increased synthesis or effects of growth hormone, diseases with decreased or increased synthesis of hormones or cytokines that regulate the release of/or response to growth hormone, diseases of the brain including Alzheimer's disease and schizophrenia, and infectious diseases.

Another aspect of the invention is a method of treating, managing or preventing type 1 diabetes, type 2 diabetes, impaired glucose tolerance, insulin resistance, leptin resistance and/or obesity comprising administering to a subject in need thereof an effective amount of a compound of the invention to said subject.

Another aspect of the invention is a method of treating immune dysfunctions including autoimmunity, diseases with dysfunctions of the coagulation system, allergic diseases, osteoporosis, proliferative disorders including cancer and psoriasis, diseases with decreased or increased synthesis or effects of growth hormone, diseases with decreased or increased synthesis of hormones or cytokines that regulate the release of/or response to growth hormone, diseases of the brain including Alzheimer's disease and schizophrenia, and infectious diseases comprising administering to a subject in need thereof an effective amount of a compound of the invention to said subject.

Another aspect of the invention is a process for the manufacture of a medicament, particular to be used in the treatment, management or prevention of type 1 diabetes, type 2 diabetes, impaired glucose tolerance, insulin resistance, leptin resistance and/or obesity which process comprising bringing a compound of the invention or a pharmaceutically acceptable salt thereof into a galenic dosage form.

Another aspect of the invention is a process for the manufacture of a medicament, particular to be used in the treatment or prevention of immune dysfunctions including autoimmunity, diseases with dysfunctions of the coagulation system, allergic diseases, osteoporosis, proliferative disorders including cancer and psoriasis, diseases with decreased or increased synthesis or effects of growth hormone, diseases with decreased or increased synthesis of hormones or cytokines that regulate the release of/or response to growth hormone, diseases of the brain including Alzheimer's disease and schizophrenia, and infectious diseases which process comprising bringing a compound of the invention or a pharmaceutically acceptable salt thereof into a galenic dosage form.

### Definitions

As used herein, the term "attached" or "-" signifies a stable covalent bond, certain preferred points of attachment points being apparent to those skilled in the art. The terms "halogen" and "halo" includes fluorine, chlorine, bromine, and iodine. The term "alkyl" includes C₁-C₁₀ straight chain saturated, C₁-C₁₀ branched chain saturated and C₃-C₁₀ cyclic saturated hydrocarbon groups. For example, this definition shall include but is not limited to methyl (Me), ethyl (Et), propyl (Pr), butyl (Bu), pentyl, hexyl, isopropyl (*i*-Pr), isobutyl (*i*-Bu), *tert*-butyl (*t*-Bu), *sec*-butyl (*s*-Bu), cyclopropyl, cyclobutyl, cyclopentyl, cyclohexyl and the like.

The term "alkenyl" includes C₂-C₁₀ unsaturated aliphatic hydrocarbon groups, C₂-C₁₀ branched unsaturated aliphatic hydrocarbon groups and C₃-C₁₀ cyclic unsaturated aliphatic hydrocarbon groups having the specified number of carbon atoms and at lest one double bond. For example, this definition shall include but is not limited to ethenyl, propenyl, butenyl, pentenyl, hexenyl, isopentenyl, neopentenyl, cyclobutenyl, cyclopentenyl, cyclohexenyl, and the like.
The term "alkynyl" includes C₂-C₁₀ straight chain unsaturated aliphatic, C₂-C₁₀ branched unsaturated and cyclic C₁₀ unsaturated aliphatic hydrocarbon groups having the specified number of carbon atoms and at lest one triple bond. For example, this definition shall include but is not limited to acetynyl, propynyl, butynyl, pentynyl, hexynyl, cyclohexynyl and the like.
The term "alkyloxy" (e.g. methoxy, ethoxy, propyloxy, allyloxy, cyclohexyloxy) represents an "alkyl" group as defined above having the indicated number of carbon atoms attached through an oxygen bridge.
The term "alkyloxyalkyl" represents an "alkyloxy" group attached through an alkyl group as defined above having the indicated number of carbon atoms.
The term "alkyloxyalkyloxy" represents an "alkyloxyalkyl" group attached through an oxygen atom as defined above having the indicated number of carbon atoms.

The term "aryloxy" (e.g. phenoxy, naphthyloxy and the like) represents an aryl group as defined below attached through an oxygen bridge.
The term "arylalkyloxy" (e.g. phenethyloxy, naphthylmethyloxy and the like) represents an "arylalkyl" group as defined below attached through an oxygen bridge.

The term "arylalkyloxyalkyl" represents an "arylalkyloxy" group as defined above attached through an "alkyl" group defined above having the indicated number of carbon atoms.
The term "aryl" represents an unsubstituted, mono-, di- or trisubstituted monocyclic, polycyclic, biaryl or heterocyclic aromatic group(s) covalently attached at any ring position capable of forming a stable covalent bond, certain preferred points of attachment being apparent to those skilled in the art (e.g., 3-indolyl, 4(5)-imidazolyl).
The term "arylalkyl" (e.g. benzyl, phenylethyl) represents an "aryl" group as defined above attached through an alkyl having the indicated number of carbon atoms.
The term "arylalkenyl" represents an "aryl" group as defined above attached through an alkenyl having the indicated number of carbon atoms.
The term "alkylamino" (e.g. methylamino, diethylamino, butylamino, N-propyl-N-hexylamino, (2-cyclopentyl)propylamino, hexenylamino, pyrrolidinyl, piperidinyl and the like) represents one or two "alkyl" groups as defined above having the indicated number of carbon atoms attached through an amine bridge. The two alkyl groups may form a saturated, partially saturated or aromatic cyclic, bicyclic or tricyclic ring system containing 3 to 14 carbon atoms and 0 to 3 additional heteroatoms selected from nitrogen, oxygen or sulfur with the nitrogen to which they are attached.
The term "arylalkylamino" (e.g. benzylamino, diphenylethylamino and the like) represents one or two "arylalkyl" groups as defined above having the indicated number of carbon atoms attached through an amine bridge. The two "arylalkyl" groups may form a saturated, partially saturated or aromatic cyclic, bicyclic or tricyclic ring system containing 3 to 14 carbon atoms and 0 to 3 additional heteroatoms selected from nitrogen, oxygen or sulfur with the nitrogen to which they are attached.
The term "alkylaminoalkyl" represents an "alkylamino" group attached through an alkyl group as defined above having the indicated number of carbon atoms.
The term "arylalkylaminoalkyl" represents an "arylalkylamino" group attached through an alkyl group as defined above having the indicated number of carbon atoms.
The term "alkylcarbonyl" (e.g. cyclooctylcarbonyl, pentylcarbonyl, 3-hexenylcarbonyl) represents an "alkyl" group as defined above having the indicated number of carbon atoms attached through a carbonyl group.

The term "arylcarbonyl" (benzoyl) represents an "aryl" group as defined above attached through a carbonyl group.
The term "arylalkylcarbonyl" (e.g. phenylcyclopropylcarbonyl, phenylethylcarbonyl and the like) represents an "arylalkyl" group as defined above having the indicated number of carbon atoms attached through a carbonyl group.
The term "alkylcarbonylalkyl" represents an "alkylcarbonyl" group attached through an "alkyl" group as defined above having the indicated number of carbon atoms.
The term "arylalkylcarbonylalkyl" represents an "arylalkylcarbonyl" group attached through an alkyl group as defined above having the indicated number of carbon atoms.
The term "alkylcarbonylamino" (e.g. hexylcarbonylamino, cyclopentylcarbonyl-aminomethyl, methylcarbonylaminophenyl) represents an "alkylcarbonyl" group as defined above wherein the carbonyl is in turn attached through the nitrogen atom of an amino group. The nitrogen atom may itself be substituted with an alkyl or aryl group.
The term "arylalkylcarbonylamino" (e.g. benzylcarbonylamino and the like) represents an "arylalkylcarbonyl" group as defined above wherein the carbonyl is in turn attached through the nitrogen atom of an amino group. The nitrogen atom may itself be substituted with an alkyl or aryl group.
The term "alkylcarbonylaminoalkyl" represents an "alkylcarbonylamino" group attached through an "alkyl" group as defined above having the indicated number of carbon atoms. The nitrogen atom may itself be substituted with an alkyl or aryl group.
The term "arylalkylcarbonylaminoalkyl" represents an "arylalkylcarbonylamino" group attached through an "alkyl" group as defined above having the indicated number of carbon atoms. The nitrogen atom may itself be substituted with an alkyl or aryl group.
The term "alkyloxycarbonyl" (e.g. *tert*-butyloxycarbonyl and the like) represents an "alkyloxy" group as defined above attached through a carbonyl group.
The term "aryloxycarbonyl" (e.g. phenyloxycarbonyl and the like) represents an "aryl" group as defined above attached through a carbonyl group.
The term "arylalkyloxycarbonyl" (e.g. benzyloxycarbonyl and the like) represents an "arylalkyl" group as defined above attached through a carbonyl group.

The term "alkylthio" (e.g. methylthio, ethylthio, propylthio, cyclohexenylthio and the like) represents an "alkyl" group as defined above having the indicated number of carbon atoms attached through a sulfur bridge.
The term "aryllthio" (e.g. phenylthio, 2-pyridylthio, and the like) represents an "alkyl" group as defined above having the indicated number of carbon atoms attached through a sulfur bridge.
The term "arylalkylthio" (e.g. phenylmethylthio, phenylethylthio, and the like) represents an "arylalkyl" group as defined above having the indicated number of carbon atoms attached through a sulfur bridge.
The term "alkylthioalkyl" represents an "alkylthio" group attached through an alkyl group as defined above having the indicated number of carbon atoms.
The term "arylalkylthioalkyl" represents an "arylalkylthio" group attached through an alkyl group as defined above having the indicated number of carbon atoms. The term "alkylcarbonylamino" (e.g. hexylcarbonylamino, cyclopentylcarbonyl-aminomethyl, methylcarbonylaminophenyl) represents an "alkylcarbonyl" group as defined above wherein the carbonyl is in turn attached through the nitrogen atom of an amino group. The nitrogen atom may itself be substituted with an alkyl or aryl group.
The term "arylalkylcarbonylamino" (e.g. benzylcarbonylamino and the like) represents an "arylalkylcarbonyl" group as defined above wherein the carbonyl is in turn attached through the nitrogen atom of an amino group. The nitrogen atom may itself be substituted with an alkyl or aryl group.
The term "alkylcarboxy" (e.g. heptylcarboxy, cyclopropylcarboxy, 3-pentenylcarboxy) represents an "alkylcarbonyl" group as defined above wherein the carbonyl is in turn attached through an oxygen bridge.
The term "arylalkylcarboxy" (e.g. benzylcarboxy, phenylcyclopropylcarboxy and the like) represents an "arylalkylcarbonyl" group as defined above wherein the carbonyl is in turn attached through an oxygen bridge.

The definition of aryl includes phenyl, biphenyl, indenyl, naphthyl (1-naphthyl, 2-naphthyl), imidazolyl (1-imidazolyl, 2-imidazolyl, 4-imidazolyl, 5-imidazolyl), triazolyl (1,2,3-triazol-1-yl, 1,2,3-triazol-2-yl 1,2,3-triazol-4-yl, 1,2,4-triazol-3-yl), thiophenyl (2-thiophenyl, 3-thiophenyl, 4-thiophenyl, 5-thiophenyl), pyridyl (2-pyridyl, 3-pyridyl, 4-pyridyl, 5-pyridyl), quinolyl (2-quinolyl, 3-quinolyl, 4-quinolyl, 5-quinolyl, 6-quinolyl, 7-quinolyl, 8-quinolyl), isoquinolyl (1-isoquinolyl, 3-isoquinolyl, 4-isoquinolyl, 5-isoquinolyl, 6-isoquinolyl, 7-isoquinolyl, 8-isoquinolyl), indolyl (1-indolyl, 2-indolyl, 3-indolyl, 4-indolyl, 5-indolyl, 6-indolyl, 7-indolyl), benzimidazolyl (1-benzimidazolyl, 2-benzimidazolyl, 4-benzimidazolyl, 5-benzimidazolyl, 6-benzimidazolyl, 7-benzimidazolyl, 8-benzimidazolyl).
The term "saturated, partially saturated or aromatic cyclic, bicyclic or tricyclic ring system" represents but are not limit to aziridinyl, pyrrolyl, pyrrolinyl, pyrrolidinyl, imidazolyl, 2-imidazolinyl, imidazolidinyl, pyrazolyl, 2-pyrazolinyl, 1,2,3-triazolyl, 1,2,4-triazolyl, morpholinyl, piperidinyl, thiomorpholinyl, piperazinyl, indolyl, isoindolyl, 1,2,3,4-tetrahydro-quinolinyl, 1,2,3,4-tetrahydro-isoquinolinyl, 1,2,3,4-tetrahydro-quinoxalinyl, indolinyl, indazolyl, benzimidazolyl, benzotriazolyl, purinyl, carbazolyl, acridinyl, phenothiazinyl, phenoxazinyl, iminodibenzyl, iminostilbenyl.
The compounds of the present invention have asymmetric centers and may occur as racemates, racemic mixtures, and as individual enantiomers or diastereoisomers, with all isomeric forms being included in the present invention as well as mixtures thereof.

It is a well known problem in drug discovery that compounds, such as enzyme inhibitors, may be very potent and selective in biochemical assays, yet be inactive in vivo. This lack of so-called bioavailability may be ascribed to a number of different factors such as lack of or poor absorption in the gut, first pass metabolism in the liver, poor uptake in cells. Although the factors determining bioavailability are not completely understood, there are many examples in the scientific literature - well known to those skilled in the art - of how to modify compounds, which are potent and selective in biochemical assays but show low or no activity *in vivo*, into drugs that are biologically active. By the term 'original compound' is understood a compound of Formula 1 wherein R₁ and R₂ are both hydrogen. It is within the scope of the invention to modify the original compounds of the invention by attaching chemical groups that will improve the bioavailability of said compounds in such a way that the uptake in cells or mammals is facilitated. Examples of said modifications, which are not intended in any way to limit the scope of the invention, include changing of one or more of the carboxy groups at the R₁ and R₂ position to esters (for instance methyl esters, ethyl esters, acetoxymethyl esters or other acyloxymethyl esters). Original compounds of the invention modified by attaching chemical groups are termed 'modified compounds'. Other examples of modified compounds, which are not intended in any way to limit the scope of the invention, are compounds that have been cyclized at specific positions - so called 'cyclic compounds' - which upon uptake in cells or mammals become hydrolysed at the same specific position(s) in the molecule to yield the compounds of the invention, the original compounds, which are then said to be 'non-cyclic'. For the avoidance of doubt, it is understood that the latter original compounds in most cases will contain other cyclic or heterocyclic structures that will not be hydrolysed after uptake in cells or mammals. Generally, said modified compounds may not show behaviour in biochemical assays similar to that of the original compound, i.e. the corresponding compounds of the invention without the attached chemical groups or said modifications. Said modified compounds may even be inactive in biochemical assays. However, after uptake in cells or mammals these attached chemical groups of the modified compounds may in turn be removed spontaneously or by endogenous enzymes or enzyme systems to yield compounds of the invention, original compounds. 'Uptake' is defined as any process that will lead to a substantial concentration of the compound inside cells or in mammals. After uptake in cells or mammals and after removal of said attached chemical group or hydrolysis of said cyclic compound, the compounds may have the same structure as the original compounds and thereby regain their activity and hence become active in cells and/or *in vivo* after uptake.
Thus, the term 'a functional group which can be converted to hydrogen *in vivo*' is intended to include any group which upon administering the present compounds to the subjects in need thereof can be converted to hydrogen e.g. enzymatically or by the acidic environment in the stomach.

The term "therapeutically effective amount" shall mean that amount of drug or pharmaceutical agent that will elicit the biological or medical response of a tissue, system, animal, or human that is being sought by a researcher, veterinarian, medical doctor or other.

### SYNTHESIS OF THE COMPOUNDS

In accordance with one aspect of the invention, the compounds of the invention are prepared as illustrated in the following reaction scheme, in which starting materials can be purchased at companies such as Aldrich, Fluka and Lancaster, or prepared by standard methods described in the chemical literature.

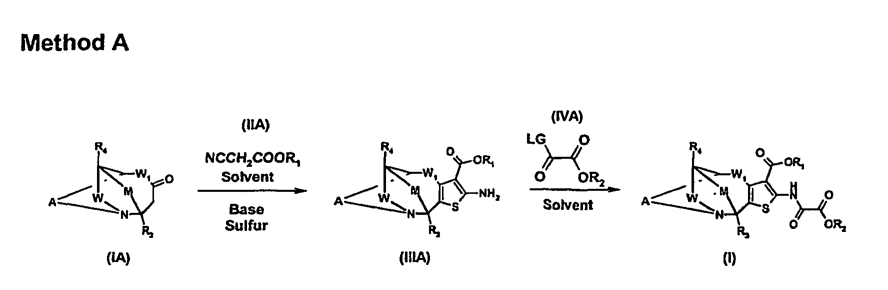

By allowing a substituted ketone (IA) to react (Gewald 2-aminothiophen conditions, K. Gewald et al. *Chem. Ber.* **99**, 94-100) with a mixture of a cyanoacetate of formula (IIA), a solvent (e.g. MeOH, EtOH, i-PrOH, *tert*-BuOH or DMF), a base (e.g. triethylamine, piperidine, morpholine, N-methyl-morpholine) and sulphur in the temperature range 25 - 70 °C to yield (IIIA), which is coupled with an activated oxalate of formula (IVA) in a solvent (e.g. dichloromethane, tetrahydrofuran, pyridine or acetone) assisted with a base (e.g. triethylamine, pyridine, K₂CO₃ or Na₂CO₃) affording (I), wherein A, M, W, W₁, R₁, R₂, R₃, and R₄ are defined as above. The protecting groups R₁ and R₂ can be removed by methods known to those skilled in the art.

Ketones of formula (IA) can either be obtained using known literature references such as *J. Med. Chem.* (1993) 683; *J. Med. Chem.* (1997) 226; *Chem. Pharm. Bull.* (1969) 434; *J. Am. Chem.* Soc. (1952) 2215; *J. org. Chem.* (1968) 4376 and *Eur. J. Med. Chem. Chim. Ther.* (1987) 383 or one of the following methods:

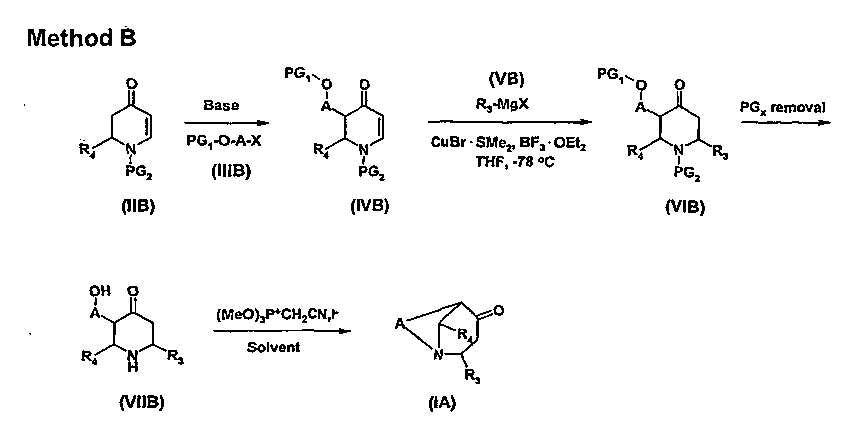

By allowing a substituted ketone of formula (VIB), which can be prepared as described by D.L. Comins in *J. Heterocyclic Chem*., **36,** 1491 (1999) to react under protective group (PGₓ) removal conditions - known to thus skilled in the art - affording compound (VIIB) which can undergo intramolecular cyclization when treated with e.g. Me₃P⁺CH₂CN(I⁻) in a solvent (e.g. propionitrile) affording ketones of formula (IA); wherein A, R₃ and R₄ are as defined above.

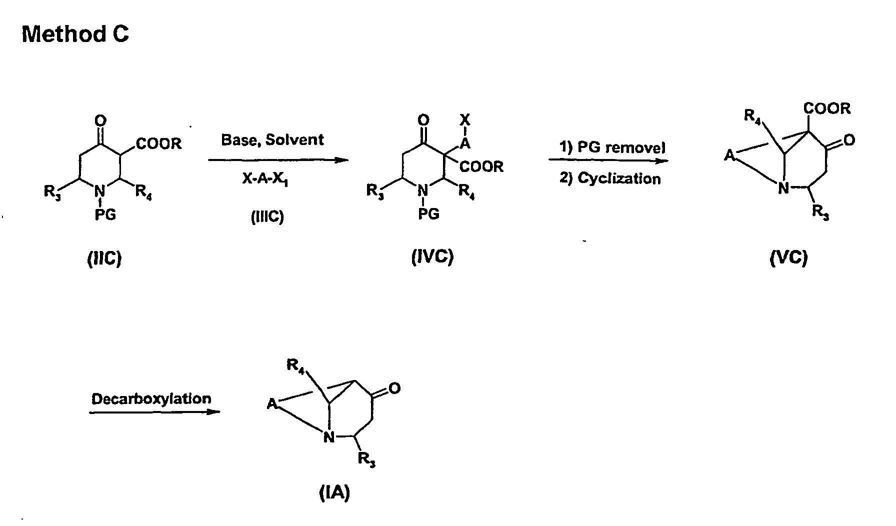

By allowing a substituted ketone of formula (IIC), to react with a difunctionalised alkylating agent (IIIC) catalysed with a base (e.g. LDA, sodium hydride, K₂CO₃ or EtO⁻Na⁺) in a solvent (e.g. tetrahydrofuran, diethyl ether or EtOH) affording (IVC). Removal of the protective group (PG) under conditions known to thus skilled in the art followed by a intramolecular cyclization catalysed with e.g. Me₃P⁺CH₂CN(I⁻) in a solvent (e.g. propionitrile) yields ketones of formula (VC), which undergoes decarboxylation when heated in a inert solvent (e.g. toluene) affording ketones of formula (IA); wherein X and X1 independently are halogen or SO₂Me and A, R₃ and R₄ are as defined above.

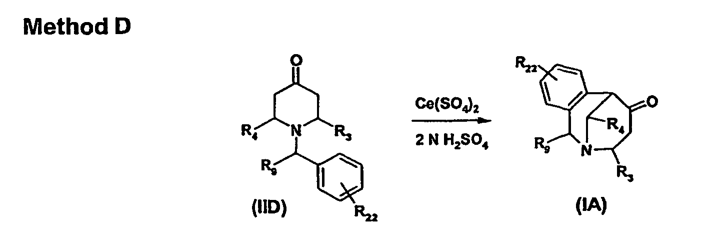

By allowing a substituted ketone of formula (IID) to undergo intramolecular cyclization when treated with cerium(IV)sulfate in 2N sulphuric acid affording ketones of formula (IA); wherein R₃, R₄, R₉ and R₂₂ are as defined above.

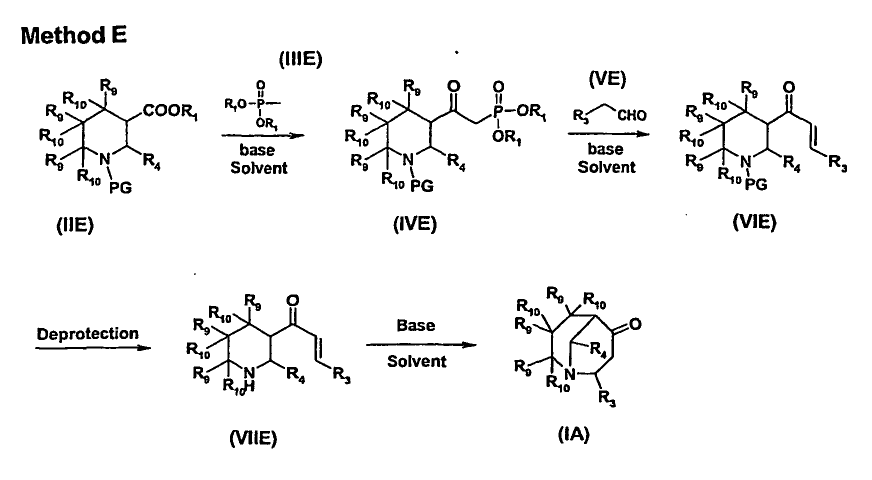

By allowing a substituted carboxylic acid ester of formula (IIE), to react with a methyl phosphonate (IIIE) catalysed with a base (e.g. n-BuLi, LDA, sodium hydride, K₂CO₃ or EtO⁻Na⁺) in a solvent (e.g. tetrahydrofuran, diethyl ether or EtOH) at low temperature (e.g. -50 to -78°C) affording a Wittig reagent of formula (IVE). Reacting (IVE) with an aldehyde of formula (VE) under Horner-Wadsworth-Emmons conditions *(Chem. Ber*. **92,**2499 (1959 and *J. Am. Chem. Soc.* **83,** 1733 (1961)) or with K₂CO₃ in acetonitrile followed by a deprotection step - known to those skilled in the art - affords (VII). Intramolecular cyclization catalysed with a base (e.g. Cs₂CO₃) in a solvent (e.g. toluene) at reflux temperature yields ketones of formula (IA); wherein R₃, R₄, R₉ and R₁₀ are as defined above.

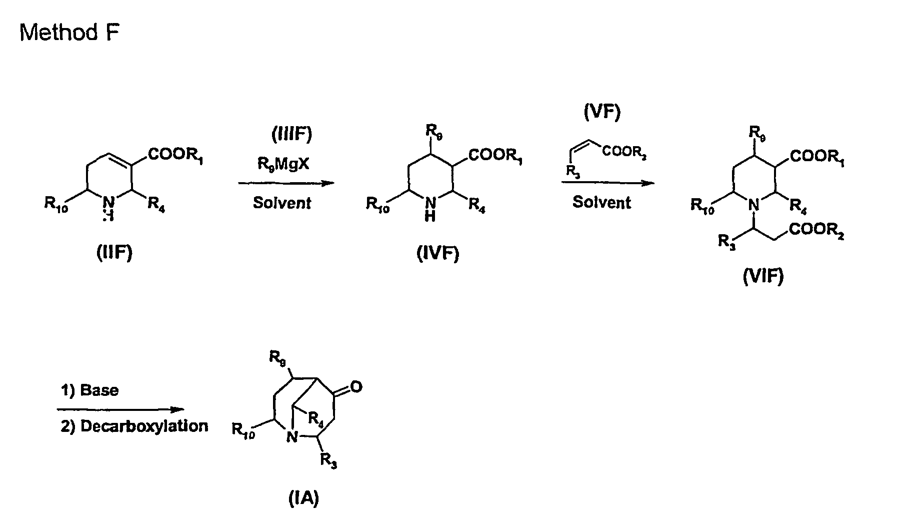

By allowing a substituted carboxylic acid ester of formula (IIF), to react with a Grignard reagent (IIIF) in a solvent (e.g. tetrahydrofuran or diethyl ether) affording (IVF) which is added to a substituted acrylate of formula (VF) affording (VIF). Intramolecular cyclization catalysed with a base (e.g. Cs₂CO₃) in a solvent (e.g. toluene) at reflux temperature followed by a thermal decarboxylation known to those skilled in the art yields ketones of formula (IA); wherein R₃, R₄, R₉ and R₁₀ are as defined above.

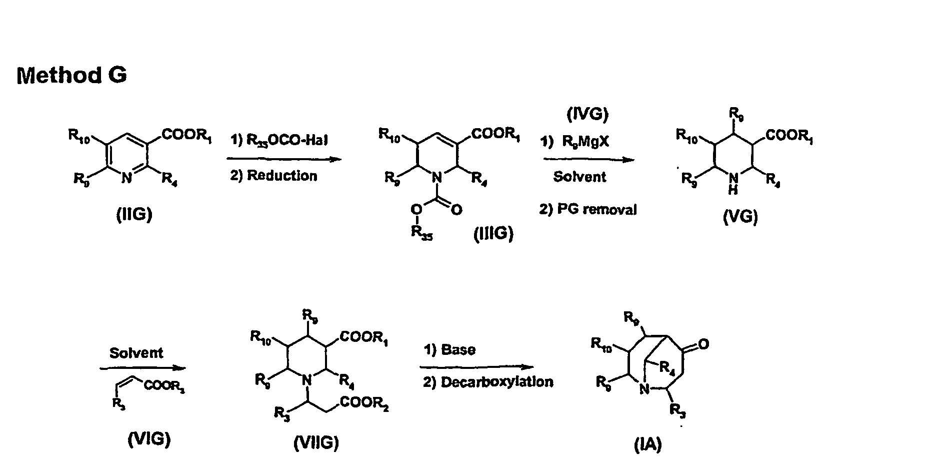

By allowing a substituted pyridine (IIG) to react with a halo carbonate (R₃₅OCO-Hal) followed by hydride reduction (e.g. NaBH₄) in a solvent (e.g. tetrahydrofurane, diethyl ether or ethanol) yielding piperidines of formula (IIIG), which are reacted with a Grignard reagent (IVG) in a solvent (e.g. tetrahydrofuran or diethyl ether) affording after protective group (PG) removal -known to thus skilled in the art - compounds of formula (VG). Compounds of formula (VG) are added to a substituted acrylate of formula (VIG) affording (VIIG). Intramolecular cyclization catalysed with a base (e.g. Cs₂CO₃) in a solvent (e.g. toluene) at reflux temperature followed by a thermal decarboxylation known to those skilled in the art yields ketones of formula (IA); wherein Hal is halogen, R₃₅ is C₁-C₁₀alkyl, aryl, C₁-C₁₀alkylaryl; wherein the alkyl and aryl groups are optionally substituted as described above; R₁, R₂, R₃, R₄, R₉ and R₁₀ are as defined above.

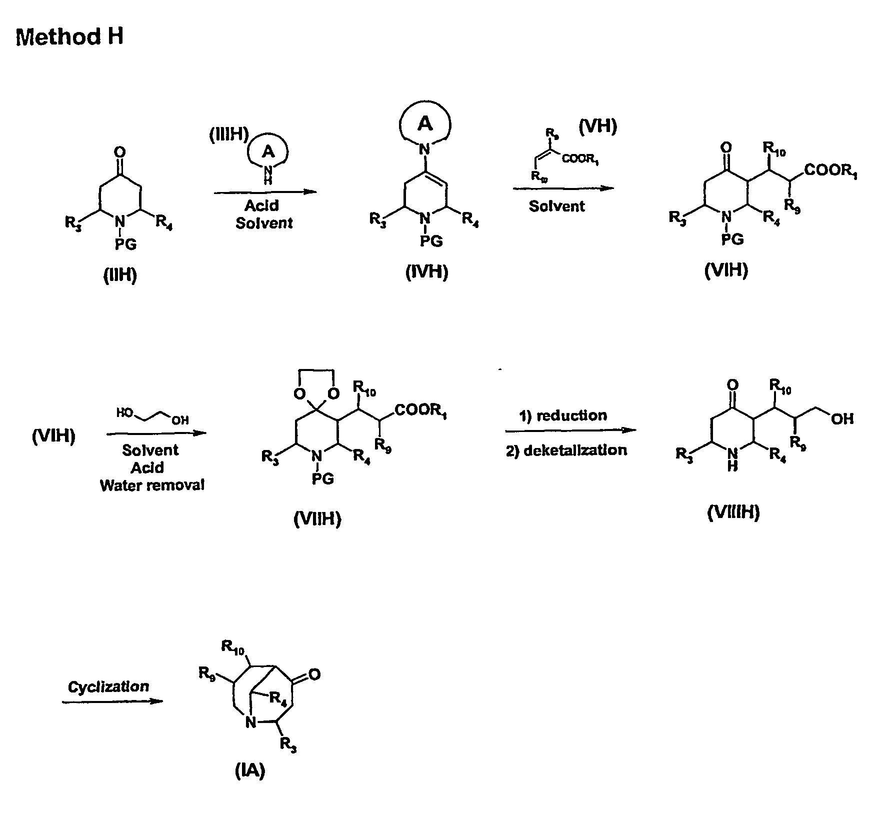

By allowing a ketone of formula (IIH) to react with a cyclic amine of formula (IIIH) under Stork enamine conditions (G. Stork *J. Org. Chem.* **85**, 207-222 (1963)) yielding an enamine (IVH) which is reacted with a substituted acrylate of formula (VH) affording ketones of formula (VIH). Ketalisation of (VIH) - known to those skilled in the art - followed by protective group removal - known to those skilled in the art - and hydride reduction (e.g. LiAIH₄, DiBAL, LiBH₄, or AIH₃) of the carboxylate affords (VIIIH) which undergoes intramolecular cyclization catalysed with e.g. Me₃P⁺CH₂CN(I⁻) in a solvent (e.g. propionitrile) yielding ketones of formula (IA); wherein A is a cyclic amine (e.g. pyrrolidine, piperidine or morpholine) and R₁, R₃, R₄, R₉ and R₁₀ are as defined above.

Preferred prodrug classes for the present compounds include acyloxymethyl esters or acyloxymethyl carbamates of the compounds of the present invention which may be prepared by the following general procedure (C.Schultz *et al, J. Biol. Chem*., **1993**, *268*: 6316-6322.) and (Alexander, J. *et al*, *J. Med. Chem.* **1991**, *34*: 78-81).

A carboxylic acid (1 equivalent) is suspended in dry acetonitrile (2 ml per 0.1 mmol). Diisopropyl amine (3.0 equivalents) is added followed by bromomethyl acetate (1.5 equivalents). The mixture is stirred under nitrogen overnight at room temperature. Acetonitrile is removed under reduced pressure to yield an oil which is diluted in ethyl acetate and washed with water (3 x). The organic layer is dried over anhydrous magnesium sulfate. Filtration followed by solvent removal under reduced pressure affords a crude oil. The product is purified by column chromatography on silica gel, using an appropriate solvent system.

A number of procedures, well known to those skilled in the art, may be used to verify that the attached chemical groups have been removed or that the cyclic compound has been hydrolysed after uptake in cells or mammals. An example, which is not intended in any way to limit the scope of the invention, is given in the following. A mammalian cell line, which can be obtained from the American Tissue Type Collection or other similar govemmental or commercial sources, is incubated with said modified compound. After incubation at conditions well known to those skilled in the art, the cells are washed appropriately, lysed and the lysate is isolated. Appropriate controls, well known to those skilled in the art, must be included. A number of different procedures, well known to those skilled in the art, may in turn be used to extract and purify said compound from said lysate. Said compound may or may not retain the attached chemical group or said cyclic compound may or may not have been hydrolysed. Similarly, a number of different procedures - well known to those skilled in the art - may be used to characterize said purified compound structurally and chemically. Since said purified compound has been isolated from said cell lysate and hence has been taken up by said cell line, a comparison of said structurally and chemically characterized compound with that of the original unmodified compound (i.e. without said attached chemical group or said non-cyclic compound) will immediately provide to those skilled in the art information on whether the attached chemical group as been removed in the cell or whether the cyclic compound has been hydrolyzed. As a further analysis, said purified compound may be subjected to enzyme kinetic analysis as described in detail in the present invention. If the kinetic profile is similar to that of the original compound without said attached chemical group, but different from said modified compound, this confirms that said chemical group has been removed or said cyclic compounds has been hydrolysed. Similar techniques may be used to analyze compounds of the invention in whole animals and mammals.

Pharmaceutically acceptable salts of the compounds of Formula 1, where a basic or acidic group is present in the structure, are also included within the scope of this invention. When an acidic substituent is present, such as -COOH, 5-tetrazolyl or -P(O)(OH)₂, there can be formed the ammonium, morpholinium, sodium, potassium, barium, calcium salt, and the like, for use as the dosage form. When a basic group is present, such as amino or a basic heteroaryl radical, such as pyridyl, an acidic salt, such as hydrochloride, hydrobromide, phosphate, sulfate, trifluoroacetate, trichloroacetate, acetate, oxalate, maleate, pyruvate, malonate, succinate, citrate, tartarate, fumarate, mandelate, benzoate, cinnamate, methanesulfonate, ethane sulfonate, picrate and the like, and include acids related to the pharmaceutically acceptable salts listed in Journal of Pharmaceutical Science, 66, 2 (1977) and incorporated herein by reference, can be used as the dosage form.
Also, in the case of the -COOH or -P(O)(OH)₂ being present, pharmaceutically acceptable esters can be employed, e.g., methyl, *tert*-butyl, pivaloyloxymethyl, and the like, and those esters known in the art for modifying solubility or hydrolysis characteristics for use as sustained release or prodrug formulations.

In addition, some of the compounds of the present invention may form solvates with water or common organic solvents. Such solvates are encompassed within the scope of the invention.

The present invention also has the objective of providing suitable topical, oral, and parenteral pharmaceutical formulations for use in the novel methods of treatment of the present invention. The compounds of the present invention may be administered orally as tablets, aqueous or oily suspensions, lozenges, troches, powders, granules, emulsions, capsules, syrups or elixirs. The composition for oral use may contain one or more agents selected from the group of sweetening agents, flavouring agents, colouring agents and preserving agents in order to produce pharmaceutically elegant and palatable preparations. The tablets contain the acting ingredient in admixture with non-toxic pharmaceutically acceptable excipients, which are suitable for the manufacture of tablets. These excipients may be, for example, (1) inert diluents, such as calcium carbonate, lactose, calcium phosphate or sodium phosphate; (2) granulating and disintegrating agents, such as corn starch or alginic acid; (3) binding agents, such as starch, gelatin or acacia; and (4) lubricating agents, such as magnesium stearate, stearic acid or talc. These tablets may be uncoated or coated by known techniques to delay disintegration and absorption in the gastrointestinal tract and thereby provide a sustained action over a longer period. For example, a time delay material such as glyceryl monostearate or glyceryl distearate may be employed. Coating may also be performed using techniques described in the U.S. Patent Nos. 4,256,108; 4,160,452; and 4,265,874 to form osmotic therapeutic tablets for control release.
Formulations for oral use may be in the form of hard gelatin capsules wherein the active ingredient is mixed with an inert solid diluent, for example, calcium carbonate, calcium phosphate or kaolin. They may also be in the form of soft gelatin capsules wherein the active ingredient is mixed with water or an oil medium, such as peanut oil, liquid paraffin or olive oil.
Aqueous suspensions normally contain the active materials in admixture with excipients suitable for the manufacture of aqueous suspension. Such expicients may be (1) suspending agent such as sodium carboxymethyl cellulose, methyl cellulose, hydroxypropylmethyl-cellulose, sodium alginate, polyvinylpyrrolidone, gum tragacanth and gum acacia; (2) dispersing or wetting agents which may be (a) naturally occurring phosphatide such as lecithin; (b) a condensation product of an alkylene oxide with a fatty acid, for example, polyoxyethylene stearate; (c) a condensation product of ethylene oxide with a long chain aliphatic alcohol, for example, heptadecaethylen-oxycetanol; (d) a condensation product of ethylene oxide with a partial ester derived from a fatty acid and hexitol such as polyoxyethylene sorbitol monooleate, or (e) a condensation product of ethylene oxide with a partial ester derived from fatty acids and hexitol anhydrides, for example polyoxyethylene sorbitan monooleate.
The pharmaceutical compositions may be in the form of a sterile injectable aqueous or oleagenous suspension. This suspension may be formulated according to known methods using those suitable dispersing or wetting agents and suspending agents, which have been mentioned above. The sterile injectable preparation may also a sterile injectable solution or suspension in a non-toxic parenterally-acceptable diluent or solvent, for example, as a solution in 1,3-butanediol. Among the acceptable vehides and solvents that may be employed are water, Ringer's solution, and isotonic sodium chloride solution. In addition, sterile, fixed oils are conventionally employed as a solvent or suspending medium. For this purpose, any bland fixed oil may be employed including synthetic mono- or diglycerides. In addition, fatty acids such as oleic acid find use in the preparation of injectables.
The compounds of the invention may also be administered in the form of suppositories for rectal administration. These compositions can be prepared by mixing the drug with a suitable non-irritating excipient which is solid at ordinary temperature but liquid at the rectal temperature and will therefore melt in the rectum to release the drug. Such materials are cocoa butter and polyethylene glycols.
The compounds of the present invention may also be administered in the form of liposome delivery systems, such as small unilamellar vesicles, large unilamellar vesicles, and multilamellar vesicles. Liposomes can be formed from a variety of phospholipids, such as cholesterol, stearylamine, or phosphatidyl-cholines.
For topical use, creams, ointments, jellies, solutions or suspensions, etc., containing the compounds of Formula 1 are employed.
Dosage levels of the compounds of the present invention are of the order of about 0.5 mg to about 100 mg per kilogram body weight, with a preferred dosage range between about 20 mg to about 50 mg per kilogram body weight per day (from about 25 mg to about 5 g's per patient per day). The amount of active ingredient that may be combined with the carrier materials to produce a single dosage will vary depending upon the host treated and the particular mode of administration. For example, a formulation intended for oral administration to humans may contain 5 mg to 1 g of an active compound with an appropriate and convenient amount of carrier material, which may vary from about 5 to about 95 percent of the total composition. Dosage unit forms will generally contain between from about 5 mg to about 500 mg of active ingredient.

It will be understood, however, that the specific dose level for any particular patient will depend upon a variety of factors including the activity of the specific compound employed, the age, body weight, general health, gender, diet, time of administration, route of administration, rate of excretion, drug combination and the severity of the particular disease undergoing therapy. The dosage needs to be individualized by the clinician.

### EXAMPLES

The process for preparing compounds of Formula 1 and preparations containing them is further illustrated in the following examples, which, however, are not to be construed as limiting.

Hereinafter, TLC is thin layer chromatography, CDCl₃ is deuterio chloroform, CD₃OD is tetradeuterio methanol and DMSO-d₆ is hexadeuterio dimethylsulfoxide. The structure of the compounds is confirmed by either elemental analysis or NMR, where peaks assigned to characteristic protons in the title compounds are presented where appropriate. ¹H-NMR shifts (δ_{H}) are given in parts per million (ppm) down field from tetramethylsilane as internal reference standard. M.p.: is melting point and is given in °C and is not corrected. Column chromatography was carried out using the technique described by W.C. Still *et al., J. Org. Chem. 43:* 2923 (1978) on Merck silica gel 60 (Art. 9385). HPLC analyses are performed using 5µm C₁₈ 4 x 250 mm column eluted with various mixtures of water and acetonitrile, flow = 1 ml/min, as described in the experimental section.

Compounds used as starting material are either known compounds or compounds, which can readily be prepared by methods known per se.

### Example 1

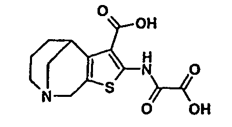

### 2-(Oxalyl-amino)-9H-4,5,6,7-tetrahydro-4,8-methano-thieno[2,3-c]azocine-3-carboxylic acid

1-Aza-bicyclo[3.3.1]nonan-4-one (1.4 g, 10 mmol) (prepared as described in *J. Med. Chem*. (1993), 683), sulphur (0.32 g, 10 mmol), morpholine, (1.5 ml) and *tert*-butylcyanoacetate (1.41 g, 10 mmol) were dissolved in ethanol (30 ml) and heated to 50°C overnight. The solvent was removed *in vacuo* and the residue was dissolved in methylene chloride and washed with an aqueous solution of sodium carbonate in water (2 M). The phases were separated, the organic phase dried (MgSO₄) and filtered. The solvent was removed *in vacuo* and the residue was chromatographed on silica (90 g) using an eluent prepared as follows:
Aqueous ammonia in water (25%) was mixed with ethanol (99.9 %) in the ratio 7: 93 making component **A**. The eluent was prepared by mixing component **A** with methylene chloride in the ratio 15:85. This afforded 900 mg (31%) of 2-amino-9*H*-4,5,6,7-tetrahydro-4,8-methano-thieno[2,3-c]azocine-3-carboxylic acid *tert* butyl ester.
¹H-NMR (400 MHz, CDCl₃): δ 6.20 (s, 2H), 4.16, 3.52 (ab-syst, 2H), 3.10-2.95 (m, 5H), 1.74 (m, 1H), 1.72 (m, 1H), 1.53 (s, 9H), 1.20 (m, 2H).

2-Amino-9*H*-4,5,6,7-tetrahydro-4,8-methano-thieno[2,3-c]azocine-3-carboxylic acid *tert* butyl ester (900 mg, 3.1 mmol) and imidazol-1-yl-oxo-acetic acid *tert* butyl ester (2 ml) were dissolved in tetrahydrofuran (10 ml) and stirred overnight. The solvent was removed *in vacuo* and the residue was chromatographed on silica (40 g) using an eluent prepared as follows: Aqueous ammonia in water (25%) was mixed with ethanol (99.9 %) in the ratio 7: 93 making component **A.** The eluent was prepared by mixing component **A** with methylene chloride in the ratio 10:90. This afforded 917 mg of 2-(*tert*-butoxyoxalylamino)-9*H*-4,5,6,7-tetrahydro-4,8-methano-thieno[2,3-c]azocine-3-carboxylic acid *tert* butyl ester. ¹H-NMR (400 MHz, CDCl₃): δ 4.33, 3.75 (ab-syst, 2H), 2.96-3.25 (m, 5H), 1.70 (m, 2H), 1.45 (m, 2H).

The above 2-(*tert*-butoxyoxalylamino)-9*H*-4,5,6,7-tetrahydro-4,8-methano-thieno[2,3-c]azocine-3-carboxylic acid *tert* butyl ester was dissolved in a mixture of trifluoroacetic acid and methylene chloride (1:1, 15 ml) and stirred for 2 hours. Evaporation of the solvent followed by coevaporation with methylene chloride 3 times (5 ml) afforded 618 mg of the title compound as a solid.
LC-MS: m/z: 311 [M+H]⁺
¹H-NMR (400 MHz, D₂O): δ 4.55, 4.25 (ab-syst, 2H), 3.81 (s, 1H), 3.3-3.45 (m, 4H), 1.51-1.72 (m, 4H).

| Calculated for C₁₃H₁₄N₂O₅S, 1.5 x C₂HF₃O₂: | | |
|---|---|---|
| C, 39.92%; | H, 3.25%; | N, 5.82%; Found |
| C, 39.87%; | H, 3.57%; | N, 6.02% |

### Example 2

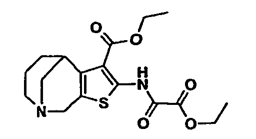

### 2-(Etoxyoxalyl-amino)-9H-4,5,6,7-tetrahydro-4,8-methano-thieno[2,3-c]azocine-3-carboxylic acid ethyl ester

The title compound was prepared analogous to Example 1 substituting *tert*-butylcyanoacetate with ethyl cyanoacetate and imidazol-1-yl-oxo-acetic acid *tert* butyl ester with ethyloxalyl chloride and finally omitting the last step.
LC-MS: m/z: 367.0 [M+H]⁺
¹H-NMR (400 MHz, D₂O): δ 1.31 (m, 6H), 1.45 (m, 1 H), 1.73 (m, 1H), 1.76 (m, 1H), 1.86 (m, 1H), 3.35 (m, 3H), 3.51 (m, 1H), 3.70 (m, 1H), 4.36 (m, 5H), 4.65 (m, 1H).

| Calculated for C₁₇H₂₂N₂O₅S, HCl: | | |
|---|---|---|
| C, 50.68%; | H, 5.75%; | N, 6.95%; Found |
| C, 50.45%; | H, 5.75%; | N, 6.85% |

### Example 3

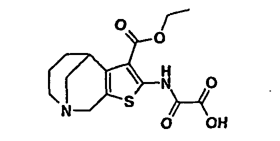

### 2-(Oxalyl-amino)-9H-4,5,6,7-tetrahydro-4,8-methano-thieno[2,3-c]azocine-3-carboxylic acid ethyl ester

The title compound was prepared analogous to Example 1 substituting *tert*-butylcyanoacetate with ethylcyanoacetate.
LC-MS: m/z: 339.2 [M+H]⁺

| Calculated for C₁₅H₁₈N₂O₅S, 2xC₂HF₃O₂: | | |
|---|---|---|
| C, 40.29%; | H, 3.56%; | N, 4.95%; Found |
| C, 40.24%; | H, 3.58%; | N, 5.10% |

### Example 4

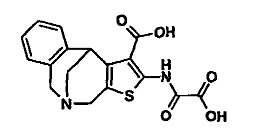

### 2-(Oxalyl-amino)-9H-4,7-dihydro-4,8-methano-benzo[f]thieno[2,3-c]azocine-3-carboxylic acid

The title compound was prepared analogous to Example **1** using 1,3,4,6-tetrahydro-2,6-methano-benzo[*c*]azocin-5-one (prepared as described in *Chem.*

*Pharm. Bull.* **17,** 434-453 (1969)) as the starting material.

An analytical sample was prepared by dissolving the compound in aqueous ammonia and applying to reverse phase silica (RP-18) using water as eluent.

The compound co-eluted with ammonium trifluoroacetate.
LC-MS: m/z: 359.2 [M+H]⁺
¹H-NMR (400 MHz, D₂O): δ 3.55 (ab-syst, 2H), 4.32 (d, 2H), 4.80, (ab-syst, 2H), 4.95 (s, 1H), 7.02 (m, 4H), 7.34 (m, 1H).

| Calculated for 2 C₁₇H₁₄N₂O₅S, H₂O, 6 C₂HF₃O₂, 7NH₃: | | |
|---|---|---|
| C, 35.92%; | H, 3.74%; | N, 10.02%; Found |
| C, 35.96%; | H, 3.95%; | N, 10.15% |

### Example 5

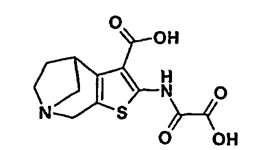

### 2-(Oxalyl-amino)-4,5,6,8-tetrahydro-4,7-methano-thieno[2,3-c]azepine-3-carboxylic acid

The title compound was prepared in a similar way as described in Example **1** using 1-aza-bicyclo[3.2.1]octan-4-one (prepared as described in *J. Org. Chem.* (1968), 4376) as the starting material.
LC-MS: m/z: 297.1 [M+H]⁺
¹H-NMR (400 MHz, DMSO-d₆): δ 4.72, 4.38 (ab-syst, 2H), 4.09 (t, 1H), 3.73 (t, 1H), 3,54 (d, 1H), 3.46 (dd, 1H), 3.38 (m, 1H), 2.21 (m, 1H), 2.11 (m, 1H).

| Calculated for C₁₂H₁₂N₂O₅S, 1.5 x C₂HF₃O₂, H₂O: | | |
|---|---|---|
| C, 37.82%; | H, 3.07%; | N, 5.88%; Found |
| C, 37.75%; | H, 2.99%; | N, 5.62% |

### Example 6

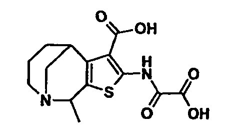

### 9-Methyl-2-(oxalyl-amino)-4,5,6,7-tetrahydro-4,8-methano-thieno[2,3-c]azocine-3-carboxylic acid

The title compound was prepared (as a diastereomeric mixture) analogous to Example 1 using 8-methyl-1-aza-bicyclo[3.3.1]nonan-4-one (prepared as described in *J. Med. Chem.* (1993), 683 and *J. Am. Chem.* Soc. (1952), 2215) as the starting material.
LC-MS: m/z: 325.1 [M+H]⁺

| Calculated for C₁₄H₁₆N₂O₅S, C₂HF₃O₂, H₂O | | |
|---|---|---|
| C, 42.11%; | H, 4.20%; | N, 6.14%; Found |
| C, 42.16%; | H, 4.04%; | N, 5.96% |

### Example 7

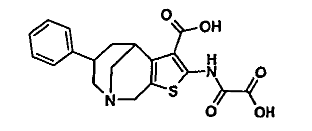

### 2-(Oxalyl-amino)-6-phenyl-9H-4,5,6,7-tetrahydro-4,8-methano-thieno[2,3-c]azocine-3-carboxylic acid

The title compound was prepared in a similar way as described in Example 1 using 7-phenyl-1-aza-bicyclo[3.3.1]nonan-4-one (prepared as described in *Eur. J. Med. Chem. Chim. Ther.* (1987), 383-392) as starting material.
LC-MS: m/z: 387.0 [M+H]⁺

| Calculated for C₁₉H₁₈N₂O₅S, C₂HF₃O₂1.5 x H₂O: | | |
|---|---|---|
| C, 47.82%; | H, 4.20%; | N, 5.31%; Found |
| C, 47.88%; | H, 3.82%; | N, 5.23% |

### Example 8

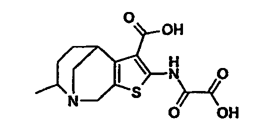

### 7-Methyl-2-(oxalyl-amino)-9H-4,5,6,7-tetrahydro-4,8-methano-thieno[2,3-c]azocine-3-carboxylic acid

### 8-Methyl-1-aza-bicyclo[3.3.1]nonan-4-one:

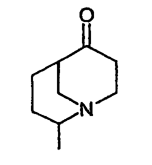

6-Methylnicotinic acid methyl ester (32.5 g, 0.2 mole), methanol (250 ml), acetic acid (50 ml) and platinum oxide (2 g) were hydrogenated 3 days at 4 atm. pressure at room temperature. The mixture was filtered, and the solvent was removed *in vacuo.* The residue was dissolved in methylene chloride, washed with 2 M aqueous sodium carbonate, dried (MgSO₄), filtered and the solvent removed *in vacuo* leaving 11.7 g of 6-methyl isonipecotinic acid as a diastereomeric mixture which was taken directly to the next step without further purification. 6-Methyl isonipecotinic acid (7.0 g) was refluxed in ethanol (70 ml) with ethyl acrylate (5.8 ml) overnight. The volatiles were removed *in vacuo* leaving a residue (11.3 g). A part of this residue (7.2 g) was dissolved in toluene (30 ml) and added dropwise over 1 hour to a refluxing solution of potassium *tert*-butoxide (8.6 g) in toluene (50 ml). Reflux was continued further 2 hours. The solvent was removed *in* vacuo. The residue was refluxed overnight in a mixture of water (100 ml) and concentrated hydrochloric acid (100 ml). The volatiles were removed *in vacuo* and the residue was dissolved in water and made basic (pH 11) with potassium carbonate. The aqueous phase was extracted with methylene chloride (3 x 200 ml) and the combined organic phases were dried (MgSO₄), filtered and the solvent removed *in vacuo.* This afforded 5.3 g of 8-methyl-1-aza-bicyclo[3.3.1]nonan-4-one as a diastereomeric mixture.
LC-MS: m/z: 154.0 [M+H]⁺

The remaining steps to the title compound were carried out in a similar way as described in Example 1.
HPLC (B1): Rₜ: 11.38 min. and 11.81 min.
LC-MS: m/z: 325.0 [M+H]⁺

| Calculated for C₁₄H₁₆N₂O₅S, 1.5 x C₂HF₃O₂ | | |
|---|---|---|
| C, 41.22%; | H, 3.56%; | N, 5.65%; Found |
| C, 41.03%; | H, 3.62%; | N, 5.56% |

### Example 9

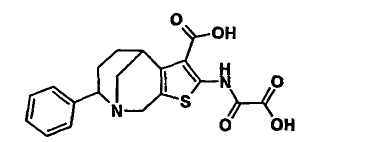

### 2-(Oxalyl-amino)-7-phenyl-9H-4,5,6,7-tetrahydro-4,8-methano-thieno[2,3-c]azocine-3-carboxylic acid

The starting material 8-phenyl-1-aza-bicyclo[3.3.1]nonan-4-one was prepared in a similar way as described in Example **8** and the remaining steps leading to the title compound were carried out in a similar way as described in Example 1.
LC-MS: m/z: 387.1 [M+H]⁺

| Calculated for C₁₉H₁₈N₂O₅S, 1.5 x C₂HF₃O₂, 0.5 x H₂O: | | |
|---|---|---|
| C, 46.65%; | H, 3.65%; | N, 4.95%; Found |
| C, 46.63%; | H, 3.77%; | N, 4.97% |

### Example 10

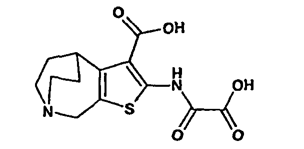

### 2-(Oxalyl-amino)-4,5,6,8-tetrahydro-4,7-ethano-thieno[2,3-c]azepine-3-carboxylic acid

The title compound was prepared analogous to Example 1 using 1-aza-bicyclo[3.2.2]nonan-4-one (prepared as described in *J. Med. Chem*., **40**; (1997), 226-235) as the starting material.
LC-MS: m/z: 311 [M+H]⁺
¹H-NMR (400 MHz, DMSO-d₆): δ 1.95 (m, 2H), 2.15 (m, 2H), 3.11 (m, 2H), 3.21 (m, 2H), 4.14 (m, 1H), 4.67 (s, 2H), 10.4 (s, 1H), 12.3 (s, 1H).

| Calculated for C₁₃H₁₄N₂O₅S, 2 x C₂HF₃O₂, 1 x H₂O | | |
|---|---|---|
| C, 36.70%; | H, 3.26%; | N, 5.03%; Found |
| C, 36.92%; | H, 3.08%; | N, 5.14% |

### Example 11

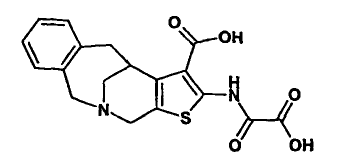

### 2-(Oxalyl-amino)-4,5,8,10-tetrahydro-4,9-methano-benzo[g]thieno[2,3-c]azonine-3-carboxylic acid

4-Oxo-piperidine-1,3-dicarboxylic acid 1-*tert*-butyl ester 3-ethyl ester (7.0 g, 26 mmol) was dissolved in acetone (150 ml) and potassium carbonate (7.1 g, 52 mmol) and dibromoxylene (13.6 g, 52 mmol) were added and the mixture was refluxed overnight. The volatiles were removed *in vacuo* and methylene chloride was added. A precipitate was filtered off and the filtrate was concentrated *in vacuo* and chromatographed on silica (38 x 5.5 cm) using methylene chloride and later ethyl acetate/methylene chloride (1:20) as eluent, which afforded 4.5 g (38 %) of 3-(2-bromomethylbenzyl)-4-oxo-piperidine-1,3-dicarboxylic acid 1-*tert*-butyl ester 3-ethyl ester as an oil.
LC-MS: m/z: 354.0 [M+H - *t*-but]⁺ and 398.0 [M+H - Boc]⁺
¹H-NMR (400 MHz, CDCl₃): δ 1.05 (t, 3H), 1.47 (s, 9H), 2.50 (bm, 1H), 2.78 (bm, 1H), 3.03 (bm, 1H), 3.18 (bm, 1H), 3.50 (d, 1H), 4.02 (bm, 2H), 4.56 (ab-syst, 2H), 4.75 (bm, 1H).

3-(2-Bromomethylbenzyl)-4-oxo-piperidine-1,3-dicarboxylic acid 1-*tert*-butyl ester 3-ethyl ester (2.5 g 5.5 mmol) was dissolved in a mixture of trifluoroacetic acid (16 ml) and methylene chloride (5 ml) and stirred 15 min. at room temperature. The solvent was removed *in vacuo* and coevaporated 2 times with methylene chloride (10 ml). The residue was dissolved in a mixture of tetrahydrofuran (150 ml) and triethylamine (15 ml) and refluxed overnight. A precipitate was filtered off and the filtrate was concentrated *in vacuo.* The residue was chromatographed on silica (90 g) using ethyl acetate as eluent, which afforded 1.4 g (94 %) of 5-oxo-1,3,4,7-tetrahydro-2,6-methano-benzo[c]azonine-6-carboxylic acid ethyl ester.
LC-MS: m/z: 274 [M+H]⁺
¹H-NMR (400 MHz, CDCl₃): δ 1.18 (m, 1H), 1.27 (t, 3H), 1.95 (dd, 1H), 3.21 (dd, 1H), 3.32 (m, 1H), 3.40 (d, 1H), 3.55 (t, 2H), 3.94 (d, 1H), 4.03 (d, 1H), 4.22 (q, 2H), 4.30 (d, 1H), 7.10-7.21 (m, 4H).

### 1,3,4,7-Tetrahydro-2,6-methano-benzo[c]azonine-5-one

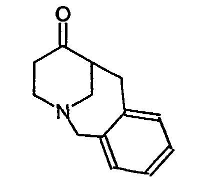

5-Oxo-1,3,4,7-tetrahydro-2,6-methano-benzo[c]azonine-6-carboxylic acid ethyl ester (1.4 g, 5.12 mmol) was dissolved in a mixture of water (15 ml) and concentrated hydrochloric acid (15 ml) and refluxed overnight. The solvent was removed *in vacuo* and the residue was dissolved in water (15 ml) and basified (pH 11) with sodium carbonate and extracted with methylene chloride (2 x 100 ml). The combined organic phases were dried (MgSO₄), filtered and the solvent evaporated *in vacuo* affording 0.76 g (74 %) of 1,3,4,7-tetrahydro-2,6-methano-benzo[c]azonine-5-one.
LC-MS: m/z: 202 [M+H]⁺, 220 [M+H₂O+H]⁺ (hydrate).
¹H-NMR (400 MHz, CDCl₃): δ 1.15 (dt, 1H), 1.85 (m, 1H), 2.74 (m, 1H), 3.12 (dd, 1H), 3.21 (m, 2H), 3.32 (d, 1H), 3.50 (s, 1H), 4.05 (d, 1H), 4.33 (d, 1H), 7.05 (m, 1H), 7.12 (m, 2H), 7.25 (m, 1H).

The remaining steps leading to the title compound were carried out in a similar way as described in Example **1**.
LC-MS: m/z: 373.4 [M+H]⁺
¹H-NMR (400 MHz, DMSO-d₆): δ 3.30 (m, 1H), 3.48 (d, 1H), 3.75 (m, 1H), 3.93 (m, 2H), 4.13 (d, 1H), 4.44 (d, 1H), 4.54 (d, 1H), 4.78 (d, 1H), 6.76 (m, 1H), 7.10 (m, 2H), 7.35 (m, 1H).

| Calculated for C₁₈H₁₆N₂O₅S, 2 x C₂HF₃O₂, 1 x H₂O: | | |
|---|---|---|
| C, 42.73%; | H, 3.26%; | N, 4.53%; Found |
| C, 43.07%; | H, 3.16%; | N, 4.37% |

### Example 12

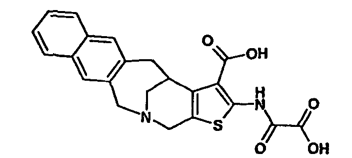

### 2-(Oxalyl-amino)-4,5,8,10-tetrahydro-4,9-methano-naphtho[2,3-q]thieno[2,3-clazonine-3-carboxylic acid

The title compound was prepared by the method described under Example **1** using 1,3,4,7-etrahydro-2,6-methano-naphtho[2,3-c]azonine-5-one as the starting material which was prepared in a similar way as described in Example **11**.
LC-MS: m/z: 423.0 [M+H]⁺

| Calculated for C₂₂H₁₈N₂O₅S, 1 x C₂HF₃O₂, 2 x H₂O: | | |
|---|---|---|
| C, 50.35%; | H, 4.05%; | N, 4.89%; Found |
| C, 50.24%; | H, 3.84%; | N, 4.94% |

### Example 13

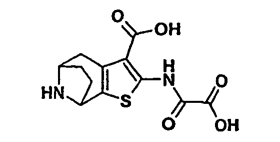

### 2-(Oxalyl-amino)-5,7-ethano-4,5,6,7-tetrahydro-thieno[2,3-c]pyridine-3-carboxylic acid

The title compound was prepared in a similar way as described under Example **1** using commercially available *N*-(*tert*-butyloxycarbonyl)-8-aza-bicyclo[3.2.1]octan-3-one as the starting material.
LC-MS: m/z: 297 [M+H]⁺
¹H-NMR (400 MHz, DMSO-d₆): δ 13.70 (bs, 1H), 12.29 (s, 1H), 9.24 (bs, 2H), 5.05 (bs, 1H), 4.32 (bs, 1H), 3.26 (dd, 1H), 2.94 (d, 1H), 2.16 (m, 3H), 1.80 (m, 1H).

| Calculated for C₁₂H₁₂N₂O₅S, 3.15 x C₂HF₃O₂: | | |
|---|---|---|
| C, 33.53%; | H, 2.33%; | N, 4.27%; Found |
| C, 33.61%; | H, 2.44%; | N, 4.25% |

### Example 14

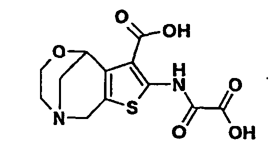

### 2-(Oxalyl-amino)-4,6,7,9-tetrahydro-4,8-methano-thieno[2,3-f][1,4]-oxazocine-3-carboxylic acid

The title compound was prepared in a similar way as described under Example **1** using 1-aza-4-oxa-bicyclo[3.3.1]nonan-6-one (prepared as described in *J. Med.*
*Chem*. (1993), **36,** 683) as the starting material.
LC-MS: m/z 313 [M+H]⁺
¹H-NMR (400 MHz, DMSO-d₆): δ 12.32 (bs, 1H), 5.52 (bs, 1H), 4.71 (d (AB), 1H), 4.58 (d (AB), 1H), 3.71 (d, 1H), 3.60-3.30 (m, 5H).

| Calculated for C₁₂H₁₂N₂O₁₀S, 0.75 x C₂HF₃O₂: | | |
|---|---|---|
| C, 40.76%; | H, 3.23%; | N, 7.04%; Found |
| C, 40.90%; | H, 3.84%; | N, 6.73% |

### Example 15

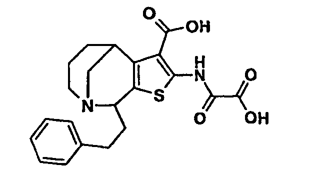

### 2-(Oxalyl-amino)-9-phenethyl-4,5,6,7-tetrahvdro-4,8-methano-thieno[2,3-c]azocine-3-carboxylic acid

The title compound was prepared by the method described under Example 1 using 1-aza-2-phenethyl-bicyclo[3.3.1]nonan-4-one (prepared by analogous procedures to the ones described in *J*. *Med. Chem*. (1993), 683 and *J*. *Am. Chem. Soc.* (1952), 2215) as the starting material.

### Example 16

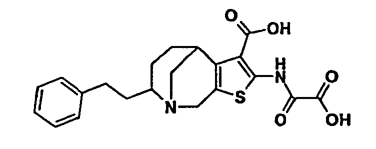

### 2-(Oxalyl-amino)-7-phenethyl-9H-4,5,6,7-tetrahydro-4,8-methano-thieno[2,3-c]azocine-3-carboxylic acid

### 8-Phenethyl-1-aza-bicyclo[3.3.1]nonan-4-one

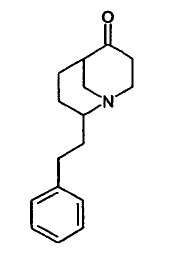

6-Methylnicotinic acid methyl ester (3 g, 0.02 mole), benzaldehyde (6.3 g, 0.06 mole), acetic acid anhydride (6 ml) and 1,3-xylene (16 ml) were heated at reflux for 2 days. The volatiles were removed *in vacuo,* and the residue was crystallised from ethyl acetate/petroleum benzene (1:9, 20 ml). This afforded 3.15 g of 6-styrylnicotinic acid methyl ester, which was taken directly to the next step without further purification. 6-Styrylnicotinic acid methyl ester (3 g, 0.012 mole), acetic acid (150 ml) and platinum oxide (0.6 g) were hydrogenated 3 days at 4 atm. pressure at room temperature. The mixture was filtered, and the solvent was removed *in vacuo*. The residue was dissolved in methylene chloride, washed with 2 M aqueous sodium carbonate, dried (MgSO₄), filtered and the solvent removed *in vacuo*. The residue was chromatographed on silica (90 g) using an eluent prepared as follows: Aqueous ammonia in water (25%) was mixed with methanol in the ratio 7: 93 making component **A**. The eluent was prepared by mixing component **A** with methylene chloride in the ratio 1:9. This afforded 1.6 g (52%) of 6-phenethyl isonipecotic acid methyl ester as a diastereomeric mixture which was taken directly to the next step. 6-Phenethyl isonipecotinic acid methyl ester (1.6 g) was refluxed in ethanol (25 ml) with *tert*-butyl acrylate (1 g) for 2 days. The volatiles were removed *in vacuo* leaving a residue which was chromatographed on silica (90 g) using ethyl acetate/petroleum benzene (1:4) as eluent, which afforded 1.4 g, (58%) of 1-(2-*tert*-butoxycarbonyl-ethyl)-6-phenethyl isonipecotinic acid methyl ester as a diastereomeric mixture which was taken directly to the next step. 1-(2-*tert*-Butoxy-carbonyl-ethyl)-6-phenethyl isonipecotinic acid methyl ester (1.4 g) was dissolved in toluene (15 ml) and added dropwise over 1 hour to a refluxing solution of potassium *tert*-butoxide (1.2 g) in toluene (25 ml). Reflux was continued further 5 hours. The solvent was removed *in vacuo* and the residue refluxed overnight in a mixture of water (20 ml) and concentrated hydrochloric acid (20 ml). The volatiles were removed *in vacuo* and the residue was dissolved in water and made basic (pH 11) with sodium carbonate. The aqueous phase was extracted with ethyl acetate (3 x 40 ml) and the combined organic phases were dried (MgSO₄), filtered and the solvent removed *in vacuo*. The residue was chromatographed on silica (90 g) using ethyl acetate/petroleum benzene (1:1) as eluent, which afforded 930 mg, (99%) of 8-phenethyl-1-aza-bicyclo[3.3.1]nonan-4-one as a diastereomeric mixture.
LC-MS: m/z: 244.2 [M+H]⁺

8-phenethyl-1-aza-bicyclo[3.3.1]nonan-4-one (930 mg, 3.82 mmol), sulphur (134 mg, 4.21 mmol), morpholine, (0.7 ml) and *tert*-butylcyano-acetate (594 mg, 4.21 mmol) were dissolved in ethanol (20 ml) and heated to 50°C overnight. The solvent was removed *in vacuo* and the residue was dissolved in methylene chloride and washed with an aqueous solution of sodium carbonate in water (2 M). The phases were separated, the organic phase dried (MgSO₄) and filtered. The solvent was removed *in vacuo* and the residue was chromatographed on silica (90 g) using ethyl acetate/petroleum benzene (1:5) as eluent. This afforded 650 mg (43%) of 2-amino-7-phenethyl-9*H*-4,5,6,7-tetrahydro-4,8-methano-thieno[2,3-c]azocine-3-carboxylic acid *tert*-butyl ester as a diastereomeric mixture.
LC-MS: m/z: 399.2 [M+H]⁺
¹H-NMR (300 MHz, CDCl₃): δ 7.15-7.30 (m, 5H), 6.00 (s, 2H), 4.29 (d, 1H), 3.92 (d, 1H), 3.67-3.74 (m, 1H), 3.62-3.67 (m, 1H), 3.54-3.58 (m, 2H), 3.05-3.22 (bm, 3H), 2.09-2.17 (bm, 1H), 1.63-1.90 (bm, 4H), 1.54 (s, 9H).

2-Amino-7-phenethyl-9*H*-4,5,6,7-tetrahydro-4,8-methano-thieno[2,3-c]azocine-3-carboxylic acid *tert*-butyl ester (650 mg, 1.6 mmol), *N*-ethyl-diisopropylamine (0.84 ml) in methylene chloride (10 ml) was cooled to 0°C whereupon *tert*-butyl oxalyl chloride (820 mg, 4.9 mmol) dissolved in methylene chloride (10 ml) was added to the mixture and stirred at 0°C for 1 hour and at room temperature for 1 hour. The solvent was removed *in vacuo* and the residue was chromatographed on silica (40 g) using ethyl acetate/petroleum benzene (1:3) as eluent. This afforded two compounds: (I) ethyl acetate/petroleum benzene (1:3) R_{f} 0.6; 262 mg of 2-(*tert*-butoxy-oxalyl-amino)-7-phenethyl-9*H*-4,5,6,7-tetrahydro-4,8-methano-thieno[2,3-c]azocine-3-carboxylic acid *tert*-butyl ester, as a mixture of enantiomers.
LC-MS: m/z: 527.2 [M+H]⁺
¹H-NMR (300 MHz, CDCl₃): δ 7.15-7.30 (m, 5H), 4.45 (d, 1H), 3.72 (d, 1H), 3.19-3.29 (t, 2H), 2.61-2.83 (m, 4H), 2.11-2.17 (m, 1H), 1.69-1.93 (m, 3H), 1.61 (s, 18H), 1.14-1.28 (m, 2H).

(II) R_{f} 0.4; 400 mg of 2-(*tert*-butoxyoxalyl-amino)-7-phenethyl-9*H*-4,5,6,7-tetrahydro-4,8-methano-thieno[2,3-c]azocine-3-carboxylic acid *tert*-butyl ester, as a mixture of enantiomers.
LC-MS: m/z: 527.2 [M+H]⁺
¹H-NMR (300 MHz, CDCl₃): δ 7.16-7.31 (m, 5H), 4.10 and 3.90 (ab-syst, 2H), 3.12-3.24 (m, 3H), 2.84-2.90 (m, 1H), 2.61-2.74 (m, 2H), 1.88-1.81 (m, 3H), 1.69-1.71 (m, 1H), 1.61 (s, 18H), 1.33-1.39 (m, 2H).

The above compound (I) 2-(*tert*-butoxyoxalyl-amino)-7-phenethyl-9*H*-4,5,6,7-tetrahydro-4,8-methano-thieno[2,3-c]azocine-3-carboxylic acid *tert* butyl ester was dissolved in a mixture of trifluoroacetic acid and methylene chloride (1: 1, 5 ml) and stirred for 2 hours. Diethyl ether (5 ml) was added and the precipitate was filtered off affording 210 mg of the title compound as a solid.
LC-MS: m/z: 415.2 [M+H]⁺
¹H-NMR (300 MHz, DMSO-d₆): δ 7.19-7.85 (m, 5H), 4.70 and 4.48 (ab-syst, 2H), 3.69 (s, 2H), 3.55-3.63 (bm, 1H), 3.48 (d, 2H), 2.56-2.78 (bm, 2H), 2.17-2.32 (bm, 1H), 1.93-2.12(bm, 2H), 1.50-1.66 (bm, 2H).

### Example 17

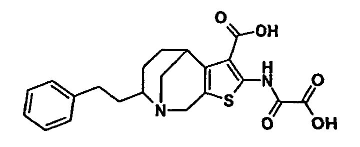

### 2-(Oxalyl-amino)-7-phenethyl-9H-4,5,6,7-tetrahydro-4,8-methano-thieno[2,3-c]azocine-3-carboxylic acid

Compound (**II**) from Example **16,** 2-(*tert*-butoxyoxalyl-amino)-7-phenethyl-9*H*-4,5,6,7-tetrahydro-4,8-methano-thieno[2,3-c]azocine-3-carboxylic acid *tert* butyl ester was dissolved in a mixture of trifluoroacetic acid and methylene chloride (1:1, 5 ml) and stirred for 2 hours. Diethyl ether (5 ml) was added; filtration afforded 83 mg of solid title compound, which is a stereoisomer of the title compound in Example 16.
LC-MS: m/z: 415.2 [M+H]⁺
¹H-NMR (300 MHz, DMSO-d₆): δ 7.15-7.34 (m, 5H), 4.60, 4.47 (ab-syst, 2H), 3.75 (s, 2H), 3.55-3.63 (bm, 3H), 2.56-2.70 (bm, 2H), 1.75-1.98 (bm, 4H), 1.20-1.28 (bm, 1H).

### Example 18

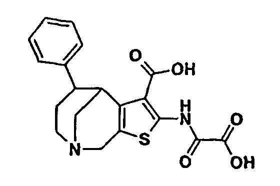

### 2-(Oxalyl-amino)-5-phenyl-9H-4,5,6,7-tetrahydro-4,8-methano-thieno[2,3-c]azocine-3-carboxylic acid

### 6-Phenethyl-1-aza-bicyclo[3.3.1]nonan-4-one

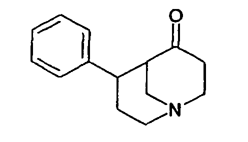

Methyl piperidone-3-carboxylate (25 g, 0.13 mol), sodium hydrogen-carbonate (24 g, 0.28 mol) and di-*tert*-butyl dicarbonate (34 g, 0.155 mol) dissolved in 1,4-dioxane/water (1:1, 320 ml) was stirred overnight. Water (160 ml) was added and the organics extracted with ethyl acetate (3 x 200 ml). The organics were dried (MgSO₄), filtered and the solvents removed *in vacuo.* The residue was chromatographed on silica (250 g) using ethyl acetate/petroleum benzene (1:6) as eluent. This afforded 32.9 g (99%) 4-oxo-piperidine-1,3-dicarboxylic acid 1-*tert*-butyl ester 3-methyl ester.
LC-MS: m/z: 280.0 [M+Na]⁺
¹H-NMR (300 MHz, CDCl₃): δ 11.97 (s, 1H), 4.06 (s, 2H), 3.78 (s, 3H), 3.57 (t, 2H), 2.37 (t, 2H), 1.49 (s, 9H).

4-oxo-piperidine-1,3-dicarboxylic acid 1-*tert*-butyl ester 3-methyl ester (5 g, 19.4 mmol) and *N*-ethyldiisopropylamine (23 ml) in methylene chloride (40 ml) was cooled to -78°C whereupon trifluoromethanesulphonic acid anhydride (13.7 g, 48 mmol) in methylene chloride was added over 1 hour. The mixture was stirred at - 78°C for 2 hours, the cooling bath was removed and the reaction allowed warming to room temperature. The solvents were removed *in vacuo* and the residue was chromatographed on silica (90 g) using ethyl acetate/petroleum benzene (1:4) as eluent. This afforded 7.1 g of 4-trifluoromethanesulphonyloxy-5,6-dihydro-2*H*-pyridine-1,3-dicarboxylic acid 1-*tert*-butyl ester 3-methyl ester.
LC-MS: m/z: 412.1 [M+Na]⁺

4-trifluoromethanesulphonyloxy-5,6-dihydro-2*H*-pyridine-1,3-dicarboxylic acid 1-*tert*-butyl ester 3-methyl ester (7.1g, 18.3 mmol), palladium tetrakis(triphenylphosphine) (640 mg, 0.55 mmol), triethylamine (3.1 ml) and phenylboronic acid (2.9 g, 23 mmol) dissolved in *N,N*-dimethyl-formamide (50 ml) was stirred for 3 hours at 100°C. Cooled and water (50 ml) added, organics extracted out with ethyl acetate (3 x 100 ml), organic phases dried (MgSO₄), filtered and the solvents were removed *in vacuo.* The residue was chromatographed on silica (90 g) using ethyl acetate/petroleum benzene (1:8) as eluent. This afforded 3.18 g (55%) of 4-phenyl-5,6-dihydro-2*H*-pyridine-1,3-dicarboxylic acid 1-*tert*-butyl ester 3-methyl ester.
¹H-NMR (400 MHz, DMSO-d₆): δ 7.26-7.35 (m, 3H), 7.12-7.14 (m, 2H), 4.25 (s, 2H), 3.61 (t, 2H), 2.51 (bt, 2H), 1.51 (s, 9H).

4-Phenyl-5,6-dihydro-2*H*-pyridine-1,3-dicarboxylic acid 1-*tert*-butyl ester 3-methyl ester (3.17 g, 10 mmol) was dissolved in a mixture of trifluoroacetic acid and methylene chloride (1:1, 10 ml) and stirred for 2 hours. Evaporation of the solvent followed by co-evaporation with methylene chloride afforded 4-phenyl-5,6-dihydro-2*H*-pyridine-1,3-dicarboxylic acid 3-methyl ester which was taken directly to the next step without further purification. 4-Phenyl-5,6-dihydro-2*H*-pyridine-1,3-dicarboxylic acid 3-methyl ester was refluxed in ethanol (25 ml) with ethyl acrylate (1.2 g) for 4 days. The volatiles were removed *in vacuo* leaving a residue which was chromatographed on silica (50 g) using ethyl acetate/petroleum benzene (1:4) as eluent, which afforded 1.42 g, (45%) of 1-(2-ethoxycarbonyl-ethyl)-4-phenyl-1,2,5,6-tetrahydropyridine-3-carboxylic acid methyl ester.
LC-MS: m/z: 318.3 [M+H]⁺
¹H-NMR (300 MHz, CDCl₃): δ 7.28-7.35 (m, 3H), 7.12-7.14 (m, 2H), 4.16 (q, 2H), 3.45 (s, 3H), 3.39 (t, 2H), 2.89 (t, 2H), 2.72 (t, 2H), 2.53-2.63 (m, 4H), 1.27 (t, 3H).

1-(2-ethoxycarbonyl-ethyl)-4-phenyl-1,2,5,6-tetrahydropyridine-3-carboxylic acid methyl ester (1.42 g, 4.5 mmol) and palladium 10% on carbon (1 g, 50% wet with water) in methanol/formic acid (30 ml, 9:1) was stirred for 3 days, filtered and the solvents removed *in vacuo.* The residue was chromatographed on silica (50 g) using ethyl acetate/petroleum benzene (1:2) as eluent. This afforded 682 mg (47%) 1-(2-ethoxycarbonyl-ethyl)-4-phenyl-piperidine-3-carboxylic acid methyl ester.
LC-MS: m/z: 320.2 [M+H]⁺
¹H-NMR (300 MHz, CDCl₃): δ 7.26-7.29 (m, 3H), 7.14-7.20 (m, 2H), 4.08-4.18 (m, 2H), 3.49 (s, 3H), 3.23-3.28 (m, 1H), 2.99-3.02 (m, 2H), 2.57-2.89 (m, 4H), 2.44-2.52 (m, 3H), 2.19-2.27 (m, 1H), 1.80-1.86 (m, 1H), 1.21-1.30 (m, 3H).

1-(2-ethoxycarbonyl-ethyl)-4-phenyl-piperidine-3-carboxylic acid methyl ester (682 mg, 2.13 mmol) was dissolved in toluene (10 ml) and added dropwise over 1 hour to a refluxing solution of potassium *tert*-butoxide (659 mg) in toluene (10 ml). Reflux was continued further 16 hours. The solvent was removed *in vacuo.* The residue was refluxed overnight in a mixture of water (10 ml) and concentrated hydrochloric acid (10 ml). The volatiles were removed *in vacuo* and the residue was dissolved in water and made basic (pH 11) with sodium carbonate. The aqueous phase was extracted with ethyl acetate (3 x 40 ml) and the combined organic phases were dried (MgSO₄), filtered and the solvent removed *in vacuo*. This afforded 393 mg of 6-phenethyl-1-aza-bicyclo[3.3.1]nonan-4-one which was taken directly to the next step without further purification.

The remaining steps leading to the title compound were carried out in a similar way as described in Example 1.
LC-MS: m/z: 387.1 [M+H]⁺
¹H-NMR (300 MHz, DMSO-d₆): δ 7.27-7.58 (m, 5H), 4.76 and 4.54 (ab-syst, 2H), 4.09 (s, 1H), 3.53-3.62 (m, 2H), 3.11-3.62 (m, 4H), 2.15-2.18 (m, 1H), 1.91-2.02 (m, 1H).

| Calculated for C₂₁H₂₂N₂O₅S, 1 x C₂HF₃O₂: | | |
|---|---|---|
| C, 50.40%; | H, 3.83%; | N, 5.60%; Found |
| C, 50.43%; | H, 3.52%; | N, 5.24% |

### Example 19

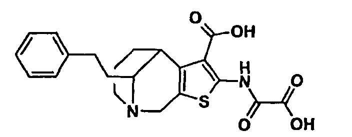

### 2-(Oxalyl-amino)-10-phenethyl-9H-4,5,6,7-tetrahydro-4,8-methano-thieno[2,3-c]azocine-3-carboxylic acid

To ethyl-2-chloronicotinate (3.7 g, 20 mmol) and bis(triphenylphosphine)-nickel(II)chloride (981 mg, 0.3 mmol) in dry tetrahydrofuran (10 ml) under a nitrogen atmosphere was added a solution of phenethyl zinc bromide (0.5 M in THF, 40 ml), the mixture was stirred for 3 days. A saturated solution of ammonium chloride (20 ml) was added and the aqueous phase was extracted with ethyl acetate (3 x 30 ml), the organic phases were dried (MgSO₄) and filtered. The solvent was removed *in vacuo* and the residue was chromatographed on silica (90 g) using ethyl acetate/petroleum benzene (1:4) as eluent, this afforded 3.81 g (75%) of ethyl 2-phenethylnicotinate.
LC-MS: m/z: 415.2 [M+H]⁺

The remaining steps leading to 9-phenethyl-1-aza-bicyclo[3.3.1]nonan-4-one and the title compound as a diastereomeric mixture were carried out in a similar way as described in Example **16.**
LC-MS: m/z: 415.1 [M+H]⁺
¹H-NMR (300 MHz, DMSO-d₆): δ 7.17-7.33 (m, 5H), 4.79 and 4.50 (ab-syst, 2H), 3.71 (s, 2H), 3.24-3.63 (bm, 3H), 2.69-2.74 (t, 2H), 2.32-2.42 (m, 1H), 1.96-2.19 (m, 2H), 1.42-1.59 (bm, 2H).

| Calculated for C₂₁H₂₂N₂O₅S, 0.4 x C₂HF₃O₂, 0.8 x H₂O: | | |
|---|---|---|
| C, 54.83%; | H, 5.08%; | N, 5.85%; Found |
| C, 54.77%; | H, 5.35%; | N, 5.80% |

### Example 20

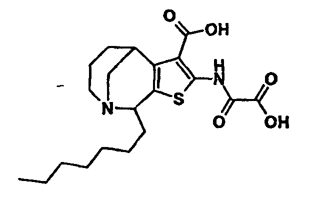

### 9-Heptyl-2-(oxalyl-amino)-4,5,6,7-tetrahydro-4,8-methano-thieno[2,3-c]azocine-3-carboxylic acid

The title compound was prepared as an enantiomeric mixture which was a diastereomere relative to Example **21** prepared by the method described under Example **1** using 1-aza-2-heptyl-bicyclo[3.3.1]nonan-4-one (prepared by analogous procedures to the ones described in *J. Med. Chem*. (1993), 683 and *J. Am. Chem. Soc.* (1952), 2215) as the starting material which was separated into diastereomers in a similar was as described in Example **16** using alumina and dichloromethane/heptane 1:1 as eluent.
HPLC (A1): Rₜ=24.20 min (100 %)
HPLC (B1): Rₜ=29.03 min
LC-MS: m/z: 409 [M+H]⁺

### Example 21

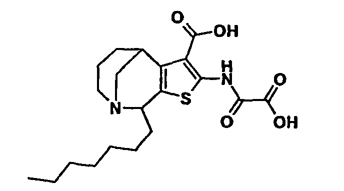

### 9-Hepthyl-2-(oxalyl-amino)-4,5,6,7-tetrahydro-4,8-methano-thieno[2,3-c]azocine-3-carboxylic acid

The title compound was a stereoisomer of Example **20** and prepared by the method described under Example **1** using 1-aza-2-heptyl-bicyclo[3.3.1]nonan-4-one (prepared by analogous procedures to the ones described in *J. Med. Chem*. (1993), 683 and *J. Am. Chem. Soc.* (1952), 2215) as the starting material.
HPLC (A1): Rₜ=22.86 min (100 %)
LC-MS: m/z: 409 [M+H]⁺

### Example 22

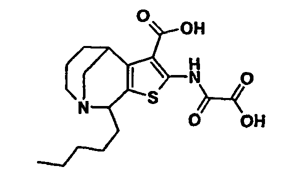

### 2-(Oxalyl-amino)-9-pentyl-4,5,6,7-tetrahydro-4,8-methano-thieno[2,3-c]azocine-3-carboxylic acid

The title compound was prepared in a similar way as described under Example **1** using 1-aza-2-pentyl-bicyclo[3.3.1]nonan-4-one (prepared by analogous procedures to the ones described in *J. Med. Chem*. (1993), 683 and *J*. *Am, Chem.* Soc. (1952), 2215) as the starting material.
LC-MS: m/z: 381.2 [M+H]⁺

| Calculated for C₁₈H₂₄N₂O₅S, 1.3 x C₂HF₃O₂, 1 x H₂O: | | |
|---|---|---|
| C, 45.09%; | H, 5.00%; | N, 5.09%; Found |
| C, 45.25%; | H, 4.91%; | N, 5.09% |

### Example 23

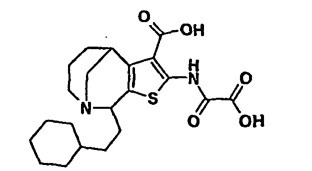

### 9-(2-Cyclohexyl-ethyl)-2-(oxalyl-amino)-4,5,6,7-tetrahydro-4,8-methano-thieno[2,3-c]azocine-3-carboxylic acid

The title compound was prepared in a similar way as described under Example **1** using 1-aza-2-(2-cyclohexyl-ethyl)-bicyclo[3.3.1]nonan-4-one (prepared by analogous procedures to the ones described in *J. Med. Chem.* (1993), 683 and *J.*
*Am. Chem.* Soc. (1952), 2215) as the starting material.
LC-MS: m/z: 421.2 [M+H]⁺

| Calculated for C₂₁H₂₈N₂O₅S, 1 x C₂HF₃O₂, 1 x H₂O: | | |
|---|---|---|
| C, 49.99%; | H, 5.65%; | N, 5.07%; Found |
| C, 49.68%; | H, 5.42%; | N, 5.32% |

### Example 24

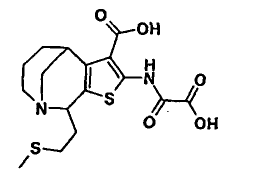

### 9-(2-Methylsulfanyl-ethyl)-2-(oxalyl-amino)-4,5,6,7-tetrahydro-4,8-methano-thieno[2,3-c]azocine-3-carboxylic acid

The title compound was prepared in a similar way as described under Example **1** using 1-aza-2-(2-methylsulfanyl-ethyl)-bicyclo[3.3.1]nonan-4-one (prepared by analogous procedures to the ones described in *J. Med. Chem*. (1993), 683 and *J. Am. Chem. Soc*. (1952), 2215) as the starting material.
LC-MS: m/z: 386.3 [M+H]⁺

| Calculated for C₁₆H₂₀N₂O₅S, 1 x C₂HF₃O₂: | | |
|---|---|---|
| C, 43.39%; | H, 4.54%; | N, 5.75%; Found |
| C, 443.37%; | H, 4.25%; | N, 5.62% |

### Example 25

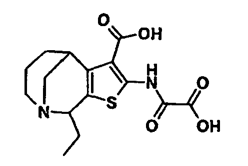

### 9-(Ethyl)-2-(oxalyl-amino)-4,5,6,7-tetrahydro-4,8-methano-thieno[2,3-c]azocine-3-carboxylic acid

The title compound was prepared by the method described under Example **1** using 1-aza-2-ethyl-bicyclo[3.3.1]nonan-4-one (prepared by analogous procedures to the ones described in *J. Med. Chem.* (1993), 683 and *J. Am. Chem. Soc.* (1952), 2215) as the starting material.
LC-MS: m/z: 339 [M+H]⁺
¹H-NMR (400 MHz, DMSO-d₆): δ 12.41 (bs, 1H), 10.21 (bs, 1 H), 4.79 (dd, 1H), 3.75 (bs, 1H), 3.65-3.15 (m, 4H), 2.11 (m, 1H), 1.96 (m, 1H), 1.87 (m, 1H), 1.73 (m, 1H), 1.59 (m, 1H), 1.35 (m, 1H), 1.12 (t, 3H).

| Calculated for C₁₅H₁₈N₂O₅S, 0.8 x C₂HF₃O₂: | | |
|---|---|---|
| C, 46.41%; | H, 4.41%; | N, 6.52%; Found |
| C, 46.25%; | H, 4.80%; | N, 6.80% |

### Example 26

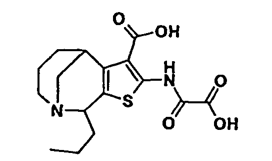

### 2-(Oxalyl-amino)-9-(propyl)-4,5,6,7-tetrahydro-4,8-methano-thieno[2,3-c]azocine-3-carboxylic acid

The title compound was prepared (as a diastereomeric mixture) by the method described under Example **1** using 1-aza-2-propyl-bicyclo[3.3.1]nonan-4-one (prepared by analogous procedures to the ones described in *J. Med. Chem.* (1993), 683 and *J. Am. Chem.* Soc. (1952), 2215) as the starting material.
LC-MS: m/z: 353 [M+H]⁺
¹H-NMR (400 MHz, DMSO-d₆): δ 12.28 (bs, 1H), 10.19 (bs, 1H), 4.85 (dd, 1H), 3.74 (bs, 1H), 3.60-3.15 (m, 4H), 2.00-0.90 (m, 11H).

| Calculated for C₁₆H₂₀N₂O₅S, 0.65 x C₂HF₃O₂: | | |
|---|---|---|
| C, 48.72%; | H, 4.88%; | N, 6.57%; Found |
| C, 48.80%; | H, 5.25%; | N, 7.60% |

### Example 27

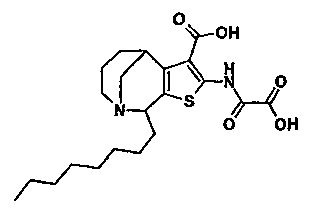

### 2-(Oxalyl-amino)-9-(octyl)-4,5,6,7-tetrahydro-4,8-methano-thieno[2,3-c]azocine-3-carboxylic acid

The title compound was prepared (as a diastereomeric mixture) by the method described under Example **1** using 1-aza-2-octyl-bicyclo[3.3.1]nonan-4-one (prepared by analogous procedures to the ones described in *J*. *Med. Chem.* (1993), 683 and *J*. *Am. Chem. Soc.* (1952), 2215) as the starting material.
LC-MS: m/z: 424 [M+H]⁺

| Calculated for C₂₁H₃₀N₂O₅S, 1.0 x C₂HF₃O₂, 1.7 x H₂O: | | |
|---|---|---|
| C, 48.71%; | H, 6.11%; | N, 4.94%; Found |
| C, 48.29%; | H, 5.93%; | N, 5.54% |

### Example 28

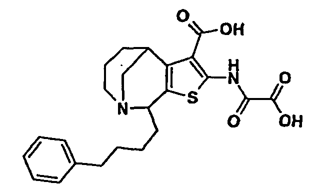

### 2-(Oxalyl-amino)-9-(4-phenyl-butyl))-4,5,6,7-tetrahydro-4,8-methano-thieno[2,3-c]azocine-3-carboxylic acid

The title compound was prepared (as a diastereomeric mixture) by the method described under Example **1** using 1-aza-2-(4-phenyl-butyl)-bicyclo[3.3.1]nonan-4-one (prepared by analogous procedures to the ones described in *J. Med. Chem*. (1993), 683 and *J. Am. Chem. Soc.* (1952), 2215) as the starting material.
LC-MS: m/z: 444 [M+H]⁺

### Example 29

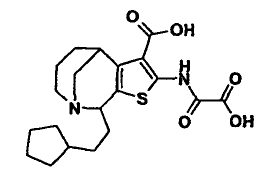

### 2-(Oxalyl-amino)-9-(2-cyclopentyl-ethyl)-4,5,6,7-tetrahydro-4,8-methano-thieno[2,3-c]azocine-3-carboxylic acid

The title compound was prepared (as a diastereomeric mixture) by the method described under Example **1** using 1-aza-2-(2-cyclopentyl-ethyl)-bicyclo[3.3.1]nonan-4-one (prepared by analogous procedures to the ones described in *J. Med. Chem*. (1993), 683 and *J. Am. Chem. Soc.* (1952), 2215) as the starting material.
LC-MS: m/z: 408 [M+H]⁺

### Example 30

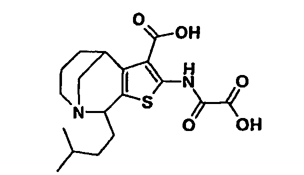

### 9-(3-Methyl-butyl)-2-(oxalyl-amino)-4,5,6,7-tetrahydro-4,8-methano-thieno[2,3-c]azocine-3-carboxylic acid

The title compound was prepared (as a diastereomeric mixture) by the method described under Example **1** using 1-aza-2-(3-methyl-butyl)-bicyclo[3.3.1]nonan-4-one (prepared by analogous procedures to the ones described in *J. Med. Chem*. (1993), 683 and *J. Am. Chem. Soc.* (1952), 2215) as the starting material.
LC-MS: m/z: 382 [M+H]⁺
¹H-NMR (400 MHz, DMSO-d₆): δ 12.28 (bs, 1H), 4.6 (bs, 1H), 3.77-3.5 (m, 4H), 1.92 (m, 2H), 1.82 (m, 1H), 1.69 (m, 1H), 1.58-1.54 (m, 2H), 1.44-1.34 (m, 2H), 1.24 (m, 1H), 1.09 (t, 1H), 0.89 (m, 6H).

### Example 31

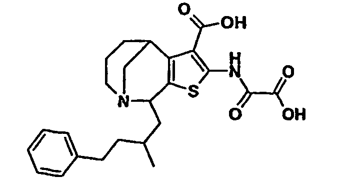

### 2-(Oxalyl-amino)-9-(4-phenyl-2-methyl-butyl)-4,5,6,7-tetrahydro-4,8-methano-thieno[2,3-c]azocine-3-carboxylic acid

The title compound was prepared (as a diastereomeric mixture) by the method described under Example **1** using 1-aza-2-(4-phenyl-2-methyl-butyl)-bicyclo[3.3.1]nonan-4-one (prepared by analogous procedures to the ones described in *J. Med. Chem.* (1993), 683 and *J. Am. Chem. Soc.* (1952), 2215) as the starting material.
LC-MS: m/z: 458 [M+H]⁺

| Calculated for C₂₄H₂₈N₂O₅S, 1.5 x C₂HF₃O₂: | | |
|---|---|---|
| C, 51.67%; | H, 4.74%; | N, 4.46%; Found |
| C, 51.45%; | H, 5.02%; | N, 4.56% |

### Example 32

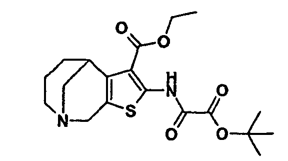

### 2-(tert-Butoxyoxalylamino)-4,5,6,7-tetrahydro-4,8-methano-thieno[2,3-c]azocine-3-carboxylic acid ethyl ester

The title compound was prepared in a similar way as described in Example **1** substituting *tert*-butyl cyanoacetate with ethyl cyanoacetate and omitting the final deprotection step.
LC-MS: m/z: 395.2 [M+H]⁺
¹H-NMR (400 MHz, DMSO-d₆) δ 1.20 (m, 1H), 1.25 (t, 3H), 1.48 (m, 3H), 1.58 (s, 9H), 3.01-3.25 (m, 5H), 3.74 (d, 1H), 4.29 (d, 1H), 4.25 (m, 2H), 12.5 (s, 1H).

| Calculated for C₁₉H₂₆N₂O₅S, 0.5 x H₂O: | | |
|---|---|---|
| C, 56.56%; | H, 6.74%; | N, 6.94%; Found |
| C, 56.56%; | H, 6.77%; | N, 6.90% |

### Example 33

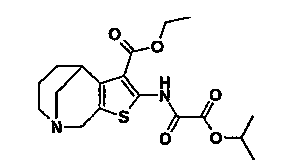

### 2-(Isopropoxyoxalylamino)-4,5,6,7-tetrahydro-4,8-methano-thieno[2,3-c]azocine-3-carboxylic acid ethyl ester

The title compound was prepared in a similar way as described in Example **1** substituting *tert*-butyl cyanoacetate with ethyl cyanoacetate and imidazol-1-yl-oxo-acetic acid *tert* butyl ester with imidazol-1-yl-oxo-acetic acid isopropyl ester and omitting the final deprotection step.
LC-MS: m/z: 381.0 [M+H]⁺

### Example 34

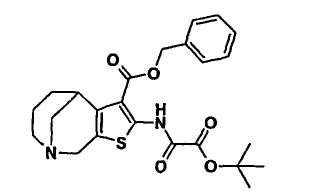

### 2-(tert-Butoxyoxalylamino)-4,5,6,7-tetrahydro-4,8-methano-thieno[2,3-c]azocine-3-carboxylic acid benzyl ester

The title compound was prepared in a similar way as described in Example **1** using benzyl cyanoacetate instead of *tert*-butyl cyanoacetate and omitting the final deprotection step.
¹H-NMR (300 MHz, CDCl₃): δ 1.22 (bt, 1H), 1.40 (m, 1H), 1.62 (s, 9H, *t*-Bu), 1.66 (t, 1H), 1.69-1.75 (m, 1H), 2.95-3.13 (m, 5H), 3.74 (d, 1H), 4.31 (d, 1H), 5.35 (m, 2H, COOC*H*₂Ph), 7.41 (m, 5H), 12.53 (s, 1H, -N*H*COCO*t*-Bu).

### Example 35

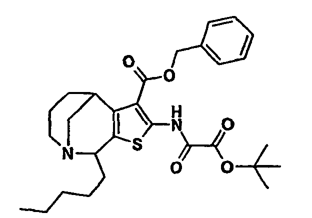

### 2-(tert-Butoxyoxalylamino)-9-pentyl-4,5,6,7-tetrahydro-4,8-methano-thieno[2,3-c]azocine-3-carboxylic acid benzyl ester

The remaining steps leading to the title compound were carried out in a similar way as described in Example **1.**
LC-MS: m/z: 527.2 [M+H]⁺
¹H-NMR: (400 MHz, CDCl₃) δ 0.89 (t, 3H), 1.20-1.70 (m, 21H), 2.90-3.10 (m, 3H), 3.55 (dd, 1H), 3.81 (dd, 1H), 4.14 (m, 1H), 5.32 (ab-syst, 2H), 7.30-7.45 (m, 5H), 12.6 (s, 1H).

### Example 36

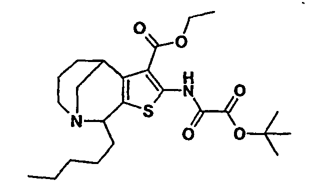

### 2-(tert-Butoxyoxalylamino)-9-pentyl-4,5,6,7-tetrahydro-4,8-methano-thieno[2,3-c]azocine-3-carboxylic acid ethyl ester

The remaining steps leading to the title compound were carried out in a similar way as described in Example **1.**
LC-MS: m/z: 465.2 [M+H]⁺
¹H-NMR: (400 MHz, CDCl₃): δ 0.90 (t, 3H), 1.20-1.82 (m, 27H), 2.9-3.23 (m, 4H), 3.58 (dd, 1H), 4.37 (m, 2H), 12.5 (s, 1H).

### Example 37

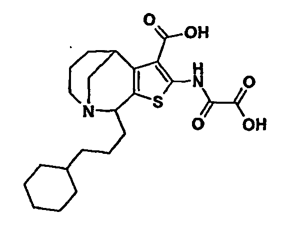

### 9-(3-Cyclohexyl-propyl)-2-(oxalyl-amino)-4,5,6,7-tetrahrdro-4,8-methano-thieno[2,3-c]azocine-3-carboxylic acid

The title compound was prepared (as a diastereomeric mixture) by the method described under Example **1** using 1-aza-2-(3-cyclohexyl-propyl)-bicyclo[3.3.1]nonan-4-one (prepared by analogous procedures to the ones described in *J. Med. Chem.* (1993), 683 and *J. Am. Chem.* Soc. (1952), 2215) as the starting material.
LC-MS: m/z: 435 [M+H]⁺

| Calculated for C₂₂H₃₀N₂O₅S, 1.5 x H₂O: | | |
|---|---|---|
| C, 50.08%; | H, 5.95%; | N, 4.87%; Found |
| C, 49.63%; | H, 5.49%; | N, 4.81% |

### Example 38

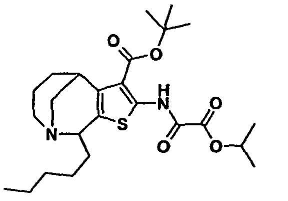

### 9-Pentyl-2-(iso-propoxyoxalyl-amino)-4,5,6,7-tetrahydro-4,8-methano-thieno[2,3-c]azocine-3-carboxylic acid tert-butyl ester

The title compound was prepared in a similar way as described in Example **1** substituting imidazol-1-yl-oxo-acetic acid *tert*-butyl ester with imidazol-1-yl-oxo-acetic acid isopropyl ester and omitting the final deprotection step. Representative peaks are shown:
¹H-NMR (400 MHz, DMSO-d₆) δ 0.92 (t, 3H), 1.20 -1.28 (m, 3H), 1.38 (m, 3H), 1.42 (d, 6H), 1.60 (s, 9H), 1.81 (m, 3H), 2.84 (m, 1H), 3.11-3.32 (m, 3H), 4.09-4.19 (m, 1H), 5.19 - 5.30 (m, 1H), 12.6 (bs, 1H).

### Example 39

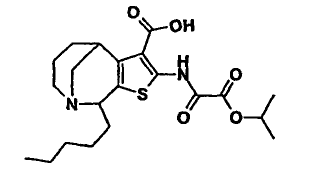

### 9-Pentyl-2-(iso-propoxyoxalyl-amino)-4,5,6,7-tetrahydro-4,8-methano-thieno[2,3-c]azocine-3-carboxylic acid

The title compound was prepared in a similar way as described in Example **1** substituting imidazol-1-yl-oxo-acetic acid *tert*-butyl ester with imidazol-1-yl-oxo-acetic acid isopropyl ester.
LC-MS: m/z: 423 [M+H]⁺
¹H-NMR (400 MHz, DMSO-d₆) δ 0.92 (t, 3H), 1.25 (m, 1H), 1.37 - 1.45 (m, 12H), 1.69 (m, 3H), 1.85 - 2.10 (m, 3H), 3.30 - 3.70 (m, 3H), 3.81 (s, 1H), 4.93 (m, 1H), 5.25 (m, 1H), 2.37 (bs, 1H).

## Claims

1. A compound of Formula 1 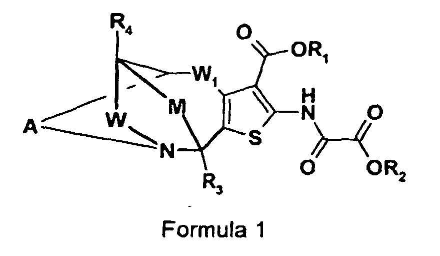 wherein
R₁ and R₂ are independently hydrogen or a functional group that can be converted to hydrogen *in vivo;*
R₃ and R₄ are independently hydrogen, C₁-C₁₀alkyl, C₂-C₁₀alkenyl, C₂- C₁₀alkynyl, aryl, arylC₁-C₁₀alkyl, C₁-C₁₀alkyloxyC₁-C₁₀alkyl, aryloxyC₁-C₁₀alkyl, arylC₁-C₁₀alkyloxyC₁-C₁₀alkyl, C₁-C₁₀alkylaminoC₁-C₁₀alkyl, C₁-C₁₀alkylthioC₁-C₁₀alkyl, arylC₁-C₁₀alkyl-aminoC₁-C₁₀alkyl, di(arylC₁-C₁₀alkyl)aminoC₁-C₁₀alkyl, C₁-C₁₀alkylcarbonyl-aminoC₁-C₁₀alkyl, arylC₁-C₁₀alkylcarbonylaminoC₁-C₁₀alkyl, CONR₅R₆, wherein the alkyl, alkenyl, alkynyl and aryl groups are optionally substituted by one or more cyano, nitro, halo, hydroxy, trihalomethyl, C₁-C₁₀alkyl, C₁-C₁₀alkoxy, or aryl independently;
R₅ and R₆ are independently selected from hydrogen, C₁-C₁₀alkyl, C₂-C₁₀alkenyl, C₂-C₁₀alkynyl, aryl, arylC₁-C₁₀alkyl, C₁-C₁₀alkylcarbonyl, C₁-C₁₀alkyloxocarbonyl, arylcarbonyl, aryloxocarbonyl, arylC₁-C₁₀alkyl-carbonyl, arylC₁-C₁₀alkyloxocarbonyl, wherein the alkyl, alkenyl, alkynyl, and aryl groups are optionally substituted by one or more cyano, nitro, halo, hydroxy, trihalomethyl, C₁-C₁₀alkyl, C₁-C₁₀alkoxy, or aryl independently; or
R₅ and R₆ may form a saturated, partially saturated or aromatic cyclic, bicyclic or tricyclic ring system containing from 3 to 14 carbon atoms and from 0 to 3 additional heteroatoms selected from nitrogen, oxygen or sulphur with the nitrogen to which they are attached, the ring system can optionally be substituted with at least one C₁-C₁₀alkyl, C₂-C₁₀alkenyl, C₂-C₁₀alkynyl, aryl, arylC₁-C₁₀alkyl, hydroxy, oxo, C₁-C₁₀alkyloxy, arylC₁-C₁₀alkyloxy, C₁-C₁₀alkyloxyC₁-C₁₀alkyl, NR₇R₈ or C₁-C₁₀alkylaminoC₁-C₁₀alkyl, wherein R₇ and R₈ are independently selected from hydrogen, C₁-C₁₀alkyl, C₂-C₁₀alkenyl, C₂-C₁₀alkynyl, aryl, arylC₁-C₁₀alkyl, C₁-C₁₀alkyl-carbonyl, arylcarbonyl, arylC₁-C₁₀alkylcarbonyl, C₁-C₁₀alkylcarboxy or arylC₁-C₁₀alkylcarboxy; wherein the alkyl, alkenyl, alkynyl, and aryl groups are optionally substituted by one or more cyano, nitro, halo, hydroxy, trihalomethyl, C₁-C₁₀alkyl, C₁-C₁₀alkoxy, or aryl independently; or
R₅ and R₆ are independently a saturated or partial saturated cyclic 5, 6 or 7 membered amine, imide or lactam;
A is absent or -[C(R₉R₁₀)]ᵢ-, -[C(R₁₁R₁₂)]ⱼ-C(R₁₃)=C(R₁₄)-[C(R₁₅R₁₆)]ₖ-, -[C(R₁₇R₁₈)]_{y}-(X)-[C(R₁₉R₂₀)]_{z}-; wherein X is O, NR₂₁ or S; i is 1, 2, 3 or 4; y and z are independently 0, 1, 2 or 3; j and k are independently 0, 1 or 2;
M is absent or -[C(R₂₉R₃₀)]ₚ-; wherein p is 1, 2 or 3;
with the proviso that A and M cannot both be absent;
W is a valence bond or -[C(R₃₁R₃₂)]_{q}-; wherein q is 1 or 2;
W₁ is a valence bond or -[C(R₃₃R₃₄)]_{qq}; wherein qq is 1 or 2;
R₉, R₁₀, R₁₁, R₁₂, R₁₃, R₁₄, R₁₅, R₁₆, R₁₈, R₁₉, R₂₀, R₂₁ R₃₁, R₃₂, R₃₃ and R₃₄ are independently selected from hydrogen, C₁-C₄alkyl, C₂-C₁₀alkenyl, C₂-C₁₀alkynyl, aryl, and arylC₁-C₄alkyl; wherein the alkyl, alkenyl, alkynyl, and aryl groups are optionally substituted by one or more cyano, nitro, halo, hydroxy, trihalomethyl, C₁-C₁₀alkyl, C₁-C₁₀alkoxy, or aryl independently;
or a salt thereof with a pharmaceutically acceptable acid or base, or any optical isomer or mixture of optical isomers, including a racemic mixture, or any tautomeric forms.

2. A compound according to claim 1 wherein R₁ and R₂ are independently hydrogen, C₁-C₁₀alkyl, C₂-C₁₀alkenyl, C₂-C₁₀alkynyl, aryl, arylC₁-C₁₀alkyl, C₁-C₁₀alkyloxy, C₁-C₁₀alkyloxyC₁-C₁₀alkyloxy, aryloxy, and arylC₁-C₁₀alkyl-oxy; wherein the alkyl, alkenyl, alkynyl and aryl groups are optionally substituted by one or more cyano, nitro, halo, hydroxy, trihalomethyl, C₁-C₁₀alkyl, C₁-C₁₀alkoxy, or aryl independently.

3. A compound according to any one of the claims 1 to 2 wherein A is absent or -[C(R₉R₁₀)]ᵢ-, -[C(R₁₇R₁₈)]_{y}-(X)-[C(R₁₉R₂₀)]_{z}-; wherein X is O, NR₂₁ or S; i is 1, 2, 3 or 4; y and z are independently 0, 1, 2 or 3.

4. A compound according to claim 3 wherein A is -[C(R₉R₁₀)]ᵢ-, wherein i is 1, 2, 3 or 4

5. A compound according to any one of the claims 1 to 4 wherein M is -[C(R₂₉R₃₀)]ₚ-; wherein p is 1, 2 or 3.

6. A compound according to any one of the claims 1 to 4 wherein M is absent.

7. A compound according to any one of the claims 1 to 6 wherein W is a valence bond.

8. A compound according to any one of the claims 1 to 6 wherein W is -[C(R₃₁R₃₂)]_{q}-; wherein q is 1 or 2.

9. A compound according to any one of the claims 1 to 8 wherein W₁ is a valence bond.

10. A compound according to any one of the claims 1 to 8 wherein W₁ is -[C(R₃₃R₃₄)]_{qq}; wherein qq is 1 or 2.

11. A compound according to any one of the claims 1 to 10 wherein R₁ is hydrogen, C₁-C₁₀alkyl, C₂-C₁₀alkenyl, C₂-C₁₀alkynyl, aryl, arylC₁-C₁₀alkyl, C₁-C₁₀alkyloxy; wherein the alkyl, alkenyl, alkynyl and aryl groups are optionally substituted by one or more cyano, nitro, halo, hydroxy, trihalomethyl, C₁-C₁₀alkyl, C₁-C₁₀alkoxy, or aryl independently.

12. A compound according to claim 11 wherein R₁ is hydrogen, C₁-C₁₀alkyl, C₂-C₁₀alkenyl, C₂-C₁₀alkynyl, aryl, or arylC₁-C₁₀alkyl; wherein the alkyl, alkenyl, alkynyl and aryl groups are optionally substituted by one or more cyano, nitro, halo, hydroxy, trihalomethyl, C₁-C₁₀alkyl, C₁-C₁₀alkoxy, or aryl independently.

13. A compound according to claim 12 wherein R₁ is hydrogen, C₁-C₁₀alkyl or arylC₁-C₁₀alkyl; wherein the alkyl groups are optionally substituted by one or more cyano, nitro, halo, hydroxy, trihalomethyl, C₁-C₁₀alkyl, C₁-C₁₀alkoxy, or aryl independently.

14. A compound according to claim 13 wherein R₁ is hydrogen or C₁-C₁₀alkyl; wherein the alkyl group is optionally substituted by one or more cyano, nitro, halo, hydroxy, trihalomethyl, C₁-C₁₀alkyl, C₁-C₁₀alkoxy, or aryl independently.

15. A compound according to any one of the claims 1 to 14 wherein R₂ is hydrogen, C₁-C₁₀alkyl, C₂-C₁₀alkenyl, C₂-C₁₀alkynyl, aryl, arylC₁-C₁₀alkyl, C₁-C₁₀alkyloxy; wherein the alkyl, alkenyl, alkynyl and aryl groups are optionally substituted by one or more cyano, nitro, halo, hydroxy, trihalomethyl, C₁-C₁₀alkyl, C₁-C₁₀alkoxy, or aryl independently.

16. A compound according to claim 15 wherein R₂ is hydrogen, C₁-C₁₀alkyl, C₂-C₁₀alkenyl, C₂-C₁₀alkynyl, aryl; wherein the alkyl, alkenyl, alkynyl and aryl groups are optionally substituted by one or more cyano, nitro, halo, hydroxy, trihalomethyl, C₁-C₁₀alkyl, C₁-C₁₀alkoxy, or aryl independently.

17. A compound according to claim 16 wherein R₂ is hydrogen or C₁-C₁₀alkyl; wherein the alkyl group is optionally substituted by one or more cyano, nitro, halo, hydroxy, trihalomethyl, C₁-C₁₀alkyl, C₁-C₁₀alkoxy, or aryl independently.

18. A compound according to claim 17 wherein R₂ is hydrogen or C₁-C₁₀alkyl.

19. A compound according to any one of the claims 1 to 18 wherein R₃ is hydrogen, C₁-C₁₀alkyl, C₂-C₁₀alkenyl, C₂-C₁₀alkynyl, aryl, arylC₁-C₁₀alkyl, aryloxyC₁-C₁₀alkyl, C₁-C₁₀alkylthioC₁-C₁₀alkyl, or arylC₁-C₁₀alkyloxyC₁-C₁₀alkyl, wherein the alkyl, alkenyl, alkynyl and aryl groups are optionally substituted by one or more cyano, nitro, halo, hydroxy, trihalomethyl, C₁-C₁₀alkyl, C₁-C₁₀alkoxy, or aryl independently.

20. A compound according to claim 19 wherein R₃ is hydrogen, C₁-C₁₀alkyl, C₂-C₁₀alkenyl, C₂-C₁₀alkynyl, aryl, C₁-C₁₀alkylthioC₁-C₁₀alkyl, or arylC₁-C₁₀alkyl, wherein the alkyl, alkenyl, alkynyl and aryl groups are optionally substituted by one or more cyano, nitro, halo, hydroxy, trihalomethyl, C₁-C₁₀alkyl, C₁-C₁₀alkoxy, or aryl independently.

21. A compound according to claim 20 wherein R₃ is hydrogen or C₁-C₁₀alkyl, wherein the alkyl group is optionally substituted by one or more cyano, nitro, halo, hydroxy, trihalomethyl, C₁-C₁₀alkyl, C₁-C₁₀alkoxy, or aryl independently.

22. A compound according to claim 21 wherein R₃ is hydrogen or C₁-C₁₀alkyl.

23. A compound according to any one of the claims 1 to 22 wherein R₄ is hydrogen, C₁-C₁₀alkyl, C₂-C₁₀alkenyl, C₂-C₁₀alkynyl, aryl, arylC₁-C₁₀alkyl, aryloxyC₁-C₁₀alkyl, or arylC₁-C₁₀alkyloxyC₁-C₁₀alkyl, wherein the alkyl, alkenyl, alkynyl and aryl groups are optionally substituted by one or more cyano, nitro, halo, hydroxy, trihalomethyl, C₁-C₁₀alkyl, C₁-C₁₀alkoxy, or aryl independently.

24. A compound according to claim 23 wherein R₄ is hydrogen, C₁-C₁₀alkyl, C₂-C₁₀alkenyl, C₂-C₁₀alkynyl, aryl, or arylC₁-C₁₀alkyl, wherein the alkyl, alkenyl, alkynyl and aryl groups are optionally substituted by one or more cyano, nitro, halo, hydroxy, trihalomethyl, C₁-C₁₀alkyl, C₁-C₁₀alkoxy, or aryl independently.

25. A compound according to claim 24 wherein R₄ is hydrogen or C₁-C₁₀alkyl, wherein the alkyl group is optionally substituted by one or more cyano, nitro, halo, hydroxy, trihalomethyl, C₁-C₁₀alkyl, C₁-C₁₀alkoxy, or aryl independently.

26. A compound according to claim 25 wherein R₄ is hydrogen or C₁-C₁₀alkyl.

27. A compound according to any one of the claims 1 to 26 wherein R₉ or R₁₀ are independently selected from hydrogen, C₁-C₄alkyl or aryl, wherein the alkyl and aryl groups are optionally substituted by one or more cyano, nitro, halo, hydroxy, trihalomethyl, C₁-C₁₀alkyl, C₁-C₁₀alkoxy, or aryl independently.

28. A compound according to claim 27 wherein R₉ or R₁₀ are independently selected from hydrogen, C₁-C₄alkyl or aryl.

29. A compound according to any one of the preceding claims selected from the following:
2-(Oxalyl-amino)-9*H*-4,5,6,7-tetrahydro-4,8-methano-thieno[2,3-c]azocine-3-carboxylic acid;
2-(Etoxyoxalyl-amino)-9*H*-4,5,6,7-tetrahydro-4,8-methano-thieno[2,3-c]azocine-3-carboxylic acid ethyl ester;
2-(Oxalyl-amino)-9*H*-4,5,6,7-tetrahydro-4,8-methano-thieno[2,3-c]azocine-3-carboxylic acid ethyl ester;
2-(Oxalyl-amino)-9*H*-4,7-dihydro-4,8-methano-benzo[f]thieno[2,3-c]azocine-3-carboxylic acid;
2-(Oxalyl-amino)-4,5,6,8-tetrahydro-4,7-methano-thieno[2,3-c]azepine-3-carboxylic acid;
9-Methyl-2-(oxalyl-amino)-4,5,6,7-tetrahydro-4,8-methano-thieno[2,3-c]azocine-3-carboxylic acid;
2-(Oxalyl-amino)-6-phenyl-9*H*-4,5,6,7-tetrahydro-4,8-methano-thieno[2,3-c]azocine-3-carboxylic acid;
7-Methyl-2-(oxalyl-amino)-9*H*-4,5,6,7-tetrahydro-4,8-methano-thieno[2,3-c]azocine-3-carboxylic acid;
2-(Oxalyl-amino)-4,5,6,8-tetrahydro-4,7-ethano-thieno[2,3-c]azepine-3-carboxylic acid;
2-(Oxalyl-amino)-5,7-ethano-4,5,6,7-tetrahydro-thieno[2,3-c]pyridine-3-carboxylic acid;
2-(Oxalyl-amino)-1,3,4,6-tetrahydro-4,8-methano-thieno[2,3-f][1,4]-oxazocine-3-carboxylic acid;
2-(Oxalyl-amino)-9-phenethyl-4,5,6,7-tetrahydro-4,8-methano-thieno[2,3-c]azocine-3-carboxylic acid;
2-(Oxatyl-amino)-7-phenethyl-9*H*-4,5,6,7-tetrahydro-4,8-methano-thieno[2,3-c]azocine-3-carboxylic acid;
2-(Oxalyl-amino)-9-phenethyl-9*H*-4,5,6,7-tetrahydro-4,8-methano-thieno[2,3-c]azocine-3-carboxylic acid;
2-(Oxalyl-amino)-5-phenyl-9*H*-4,5,6,7-tetrahydro-4,8-methano-thieno[2,3-c]azocine-3-carboxylic acid;
2-(Oxalyl-amino)-10-phenethyl-9*H*-4,5,6,7-tetrahydro-4,8-methano-thieno[2,3-c]azocine-3-carboxylic acid;
9-Hepthyl-2-(oxalyl-amino)-4,5,6,7-tetrahydro-4,8-methano-thieno[2,3-c]azocine-3-carboxylic acid;
9-Hepthyl-2-(oxalyl-amino)-4,5,6,7-tetrahydro-4,8-methano-thieno[2,3-c]azocine-3-carboxylic acid;
2-(Oxalyl-amino)-7-phenyl-9*H*-4,5,6,7-tetrahydro-4,8-methano-thieno[2,3-c]azocine-3-carboxylic acid;
2-(Oxalyl-amino)-9-pentyl-4,5,6,7-tetrahydro-4,8-methano-thieno[2,3-c]azocine-3-carboxylic acid;
9-(2-Cyclohexyl-ethyl)-2-(oxalyl-amino)-4,5,6,7-tetrahydro-4,8-methano-thieno[2,3-c]azocine-3-carboxylic acid;
9-(2-Methylsulfanyl-ethyl)-2-(oxalyl-amino)-4,5,6,7-tetrahydro-4,8-methano-thieno[2,3-c]azocine-3-carboxylic acid;
9-(Ethyl)-2-(oxalyl-amino)-4,5,6,7-tetrahydro-4,8-methano-thieno[2,3-c]azocine-3-carboxylic acid;
2-(Oxalyl-amino)-9-(propyl)-4,5,6,7-tetrahydro-4,8-methano-thieno[2,3-c]azocine-3-carboxylic acid;
2-(*tert*-Butoxyoxalylamino)-4,5,6,7-tetrahydro-4,8-methano-thieno[2,3-c]azocine-3-carboxylic acid ethyl ester;
2-(Isopropoxyoxalylamino)-4,5,6,7-tetrahydro-4,8-methano-thieno[2,3-c]azocine-3-carboxylic acid ethyl ester;
2-(Benzoxyoxalylamino)-4,5,6,7-tetrahydro-4,8-methano-thieno[2,3-c]azocine-3-carboxylic acid ethyl ester;
2-(Benzoxyoxalylamino)-4,5,6,7-tetrahydro-4,8-methano-thieno[2,3-c]azocine-3-carboxylic acid benzyl ester;
2-(*tert*-Butoxyoxalylamino)-4,5,6,7-tetrahydro-4,8-methano-thieno[2,3-c]azocine-3-carboxylic acid benzyl ester;
2-(*tert*-Butoxyoxalylamino)-9-pentyl-4,5,6,7-tetrahydro-4,8-methano-thieno[2,3-c]azocine-3-carboxylic acid benzyl ester;
2-(*tert*-Butoxyoxalylamino)-9-pentyl-4,5,6,7-tetrahydro-4,8-methano-thieno[2,3-c]azocine-3-carboxylic acid ethyl ester;
2-(Oxalyl-amino)-9-(octyl)-4,5,6,7-tetrahydro-4,8-methano-thieno[2,3-c]azocine-3-carboxylic acid;
2-(Oxalyl-amino)-9-(4-phenyl-butyl))-4,5,6,7-tetrahydro-4,8-methano-thieno[2,3-c]azocine-3-carboxylic acid;
2-(Oxalyl-amino)-9-(2-cyclopentyl-ethyl)-4,5,6,7-tetrahydro-4,8-methano-thieno[2,3-c]azocine-3-carboxylic acid;
9-(3-Methyl-butyl)-2-(oxalyl-amino)-4,5,6,7-tetrahydro-4,8-methano-thieno[2,3-c]azocine-3-carboxylic acid;
2-(Oxalyl-amino)-9-(4-phenyl-2-methyl-butyl)-4,5,6,7-tetrahydro-4,8-methano-thieno[2,3-c]azocine-3-carboxylic acid;
2-(*tert*-Butoxyoxalylamino)-4,5,6,7-tetrahydro-4,8-methano-thieno[2,3-c]azocine-3-carboxylic acid ethyl ester;
2-(Isopropoxyoxalylamino)-4,5,6,7-tetrahydro-4,8-methano-thieno[2,3-c]azocine-3-carboxylic acid ethyl ester;
2-(*tert*-Butoxyoxalylamino)-4,5,6,7-tetrahydro-4,8-methano-thieno[2,3-c]azocine-3-carboxylic acid benzyl ester;
2-(*tert*-Butoxyoxalylamino)-9-pentyl-4,5,6,7-tetrahydro-4,8-methano-thieno[2,3-c]azocine-3-carboxylic acid benzyl ester;
2-(*tert*-Butoxyoxalylamino)-9-pentyl-4,5,6,7-tetrahydro-4,8-methano-thieno[2,3-c]azocine-3-carboxylic acid ethyl ester;
9-(3-Cyclohexyl-propyl)-2-(oxalyl-amino)-4,5,6,7-tetrahydro-4,8-methano-thieno[2,3-c]azocine-3-carboxylic acid;
9-Pentyl-2-(*iso*-propoxyoxalyl-amino)-4,5,6,7-tetrahydro-4,8-methano-thieno[2,3-c]azocine-3-carboxylic acid *tert*-butyl ester;
9-Pentyl-2-(*iso*-propoxyoxalyl-amino)-4,5,6,7-tetrahydro-4,8-methano-thieno[2,3-c]azocine-3-carboxylic acid;
9-(3-Cyclohexyl-propyl)-2-(oxalyl-amino)-4,5,6,7-tetrahydro-4,8-methano-thieno[2,3-c]azocine-3-carboxylic acid;
9-Pentyl-2-(*iso*-propoxyoxalyl-amino)-4,5,6,7-tetrahydro-4,8-methano-thieno[2,3-c]azocine-3-carboxylic acid *tert*-butyl ester;
9-Pentyl-2-(*iso*-propoxyoxalyl-amino)-4,5,6,7-tetrahydro-4,8-methano-thieno[2,3-c]azocine-3-carboxylic acid.

30. A pharmaceutical composition comprising a compound according to any one of the claims 1 to 29 or a pharmaceutically acceptable salt thereof with a pharmaceutically acceptable acid or base, or any optical isomer or mixture of optical isomers, including a racemic micture, or any tautomeric forms together with one or more pharmaceutically acceptable carriers or diluents.

31. The pharmaceutical composition according to claim 30 suitable for treating, managing or preventing type 1 diabetes, type 2 diabetes, impaired glucose tolerance, insulin resistance, leptin resistance and/or obesity.

32. A use of a compound according to any one of the claims 1 to 29 or a pharmaceutically acceptable salt thereof with a pharmaceutically acceptable acid or base, or any optical isomer or mixture of optical isomers, including a racemic micture, or any tautomeric forms, together with one or more pharmaceutically acceptable carriers or diluents as a medicament.

33. A use of a compound according to any one of the claims 1 to 29 or a pharmaceutically acceptable salt thereof with a pharmaceutically acceptable acid or base, or any optical isomer or mixture of optical isomers, including a racemic micture, or any tautomeric forms, together with one or more pharmaceutically acceptable carriers or diluents for preparing a medicament.

34. The use according to claim 33 for the preparation of a medicament suitable for the treatment of immune dysfunctions including autoimmunity, diseases with dysfunctions of the coagulation system, allergic diseases, osteoporosis, proliferative disorders including cancer and psoriasis, diseases with decreased or increased synthesis or effects of growth hormone, diseases with decreased or increased synthesis of hormones or cytokines that regulate the release of/or response to growth hormone, diseases of the brain including Alzheimer's disease and schizophrenia, and infectious diseases.

## Patentansprüche

1. Verbindung der Formel 1 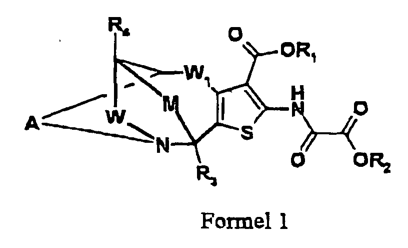 wobei
R₁ und R₂ unabhängig Wasserstoff oder eine funktionelle Gruppe, die in vivo zu Wasserstoff umgewandelt werden kann, sind,
R₃ und R₄ unabhängig Wasserstoff, C₁-C₁₀-Alkyl, C₂-C₁₀-Alkenyl, C₂-C₁₀-Alkinyl, Aryl, Aryl-C₁-C₁₀-alkyl, C₁-C₁₀-Alkyloxy-C₁-C₁₀-alkyl, Aryloxy-C₁-C₁₀-alkyl, Aryl-C₁-C₁₀-alkyloxy-C₁-C₁₀-alkyl, C₁-C₁₀-Alkylamino-C₁-C₁₀-alkyl, C₁-C₁₀-Alkylthio-C₁-C₁₀-alkyl, Aryl-C₁-C₁₀-alkylaminoC₁-C₁₀-alkyl, Di(aryl-C₁-C₁₀-alkyl)amino-C₁-C₁₀-alkyl, C₁-C₁₀-Alkylcarbonylamino-C₁-C₁₀-alkyl, Aryl-C₁-C₁₀-alkylcarbonylamino-C₁-C₁₀-alkyl, CONR₅R₆ sind, wobei die Alkyl-, Alkenyl-, Alkinyl- und Arylgruppen gegebenenfalls unabhängig substituiert sind durch ein oder mehrere Cyano, Nitro, Halogen, Hydroxy, Trihalogenmethyl, C₁-C₁₀-Alkyl, C₁-C₁₀-Alkoxy oder Aryl,
R₅ und R₆ unabhängig ausgewählt sind aus Wasserstoff, C₁-C₁₀-Alkyl, C₂-C₁₀-Alkenyl, C₂-C₁₀-Alkinyl, Aryl, Aryl-C₁-C₁₀-alkyl, C₁-C₁₀-Alkylcarbonyl, C₁-C₁₀-Alkyloxocarbonyl, Arylcarbonyl, Aryloxocarbonyl, Aryl-C₁-C₁₀-alkylcarbonyl, Aryl-C₁-C₁₀-alkyloxocarbonyl, wobei die Alkyl-, Alkenyl-, Alkinyl- und Arylgruppen gegebenenfalls unabhängig substituiert sind durch ein oder mehrere Cyano, Nitro, Halogen, Hydroxy, Trihalogenmethyl, C₁-C₁₀-Alkyl, C₁-C₁₀-Alkoxy oder Aryl, oder
R₅ und R₆ ein gesättigtes, teilweise gesättigtes oder aromatisches cyclisches, bicyclisches oder tricyclisches Ringsystem, enthaltend 3 bis 14 Kohlenstoffatome und 0 bis 3 zwätzliche Heteroatome, ausgewählt aus Stickstoff, Sauerstoff oder Schwefel, mit dem Stickstoffatom, an welches sie gebunden sind, bilden können, wobei das Ringsystem wahlweise substituiert ist mit mindestens einem C₁-C₁₀-Alkyl, C₂-C₁₀-Alkenyl, C₂-C₁₀-Alkinyl, Aryl, Aryl-C₁-C₁₀-alkyl, Hydroxy, Oxo, C₁-C₁₀-Alkyloxy, Aryl-C₁-C₁₀-alkyloxy, C₁-C₁₀-Alkyloxy-C₁-C₁₀-alkyl, NR₇R₈ oder C₁-C₁₀-Alkylamino-C₁-C₁₀-alkyl, wobei R₇ und R₈ unabhängig ausgewählt sind aus Wasserstoff, C₁-C₁₀-Alkyl, C₂-C₁₀-Alkenyl, C₂-C₁₀-Alkinyl, Aryl, Aryl-C₁-C₁₀-alkyl, C₁-C₁₀-Alkylcarbonyl, Arylcarbonyl, Aryl-C₁-C₁₀-alkylcarbonyl, C₁-C₁₀-Alkylcarboxy oder Aryl-C₁-C₁₀-alkylcarboxy, wobei die Alkyl-, Alkenyl-, Alkinyl- und Arylgruppen gegebenenfalls unabhängig substituiert sind durch ein oder mehrere Cyano, Nitro, Halogen, Hydroxy, Trihalogenmethyl, C₁-C₁₀-Alkyl, C₁-C₁₀-Alkoxy oder Aryl, oder
R₅ und R₆ unabhängig ein gesättigtes oder teilweise gesättigtes cyclisches 5-, 6- oder 7-gliedriges Amin, Imid oder Lactam sind,
A fehlt oder -[C(R₉R₁₀)]ᵢ-, -[C(R₁₁R₁₂)]ⱼ-C(R₁₃)=C(R₁₄)-[C(R₁₅R₁₆)]ₖ--[C(R₁₇R₁₈)]_{y}-(X)-[C(R₁₉R₂₀)]_{z}- ist, wobei X O, NR₂₁ oder S ist, i 1, 2, 3 oder 4 ist, y und z; unabhängig 0, 1, 2 oder 3 sind, j und k unabhängig 0, 1 oder 2 sind,
M fehlt oder -[C(R₂₉R₃₀)]ₚ- ist, wobei p 1, 2 oder 3 ist,
mit der Maßgabe, dass A und M nicht zusammen fehlen dürfen,
W ein Valenzbindung oder -[C(R₃₁R₃₂)]_{q}- ist, wobei q 1 oder 2 ist,
W₁ ein Valenzbindung oder -[C(R₃₃R₃₄)]_{qq} ist, wobei qq 1 oder 2 ist,
R₉, R₁₀, R₁₁, R₁₂, R₁₃, R₁₄, R₁₅, R₁₆, R₁₈, R₁₉, R₂₀, R₂₁ R₃₁, R₃₂, R₃₃ und R₃₄ unabhängig ausgewählt sind aus Wasserstoff, C₁-C₄-Alkyl, C₂-C₁₀-Alkenyl, C₂-C₁₀-Alkinyl, Aryl und Aryl-C₁-C₄-alkyl, wobei die Alkyl-, Alkenyl-, Alkinyl- und Arylgruppen gegebenenfalls unabhängig substituiert sind durch ein oder mehrere Cyano, Nitro, Halogen, Hydroxy, Trihalogenmethyl, C₁-C₁₀-Alkyl, C₁-C₁₀-Alkoxy oder Aryl,
oder ein Salz davon mit einer pharmazeutisch verträglichen Säure oder Base oder ein beliebiges Isomer oder Gemisch aus optischen Isomeren, einschließlich eines racemischen Gemischs, oder beliebige tautomere Formen davon,

2. Verbindung nach Anspruch 1 wobei R₁ und R₂ unabhängig Wasserstoff, C₁-C₁₀-Alkyl, C₂-C₁₀-Alkenyl, C₂-C₁₀-Alkinyl, Aryl, Aryl-C₁-C₁₀-alkyl, C₁-C₁₀-Alkyloxy, C₁-C₁₀-Alkyloxy-C₁-C₁₀-alkyloxy, Aryloxy und Aryl-C₁-C₁₀-alkyloxy sind, wobei die Alkyl-, Alkenyl-, Alkinyl- und Arylgruppen gegebenenfalls unabhängig substituiert sind durch ein oder mehrere Cyano, Nitro, Halogen, Hydroxy, Trihalogenmethyl, C₁-C₁₀-Alkyl, C₁-C₁₀-Alkoxy oder Aryl.

3. Verbindung nach einem der Ansprüche 1 oder 2, wobei A fehlt oder -[C(R₉R₁₀)]ᵢ-, -[C(R₁₇R₁₈)]_{y}-(X)-[C(R₁₉R₂₀)]_{z}- ist, wobei X O, NR₂₁ oder S ist, i 1, 2, 3 oder 4 ist, y und z unabhängig 0, 1, 2 oder 3 sind.

4. Verbindung nach Anspruch 3, wobei A is -[C(R₉R₁₀)]ᵢ-, wobei i 1, 2, 3 oder 4 ist.

5. Verbindung nach einem der Ansprüche 1 bis 4, wobei M is -[C(R₂₉R₃₀)]ₚ-, wobei p 1, 2 oder 3 ist.

6. Verbindung nach einem der Ansprüche 1 bis 4, wobei M fehlt.

7. Verbindung nach einem der Ansprüche 1 bis 6, wobei W eine Valenzbindung ist.

8. Verbindung nach einem der Ansprüche 1 bis 6, wobei W -[C(R₃₁R₃₂)]_{q}- ist, wobei q 1 oder 2 ist.

9. Verbindung nach einem der Ansprüche 1 bis 8, wobei W₁ eine Valenzbindung ist.

10. Verbindung nach einem der Ansprüche 1 bis 8, wobei W₁ -[C(R₃₃R₃₄)]_{qq} ist, wobei qq 1 oder 2 ist.

11. Verbindung nach einem der Ansprüche 1 bis 10, wobei R₁ Wasserstoff, C₁-C₁₀-Alkyl, C₂-C₁₀-Alkenyl, C₂-C₁₀-Alkinyl, Aryl, Aryl-C₁-C₁₀-alkyl, C₁-C₁₀-Alkyloxy ist, wobei die Alkyl-, Alkenyl-, Alkinyl- und Arylgruppen gegebenenfalls unabhängig substituiert sind durch ein oder mehrere Cyano, Nitro, Halogen, Hydroxy, Trihalogenmethyl, C₁-C₁₀-Alkyl, C₁-C₁₀-Alkoxy oder Aryl.

12. Verbindung nach Anspruch 11, wobei R₁ Wassmtoff, C₁-C₁₀-Alkyl, C₂-C₁₀-Alkenyl, C₂-C₁₀-Alkinyl, Aryl oder Aryl-C₁-C₁₀-alkyl ist, wobei die Alkyl-, Alkenyl-, Alkinyl- und Arylgruppen gegebenenfalls unabhängig substituiert sind durch ein oder mehrere Cyano, Nitro, Halogen, Hydroxy, Trihalogenmethyl, C₁-C₁₀-Alkyl, C₁-C₁₀-Alkoxy oder Aryl.

13. Verbindung nach Anspruch 12, wobei R₁ Wasserstoff, C₁-C₁₀-Alkyl oder Aryl-C₁-C₁₀-alkyl ist, wobei die Alkyl-, Alkenyl-, Alkinyl- und Arylgruppen gegebenenfalls unabhängig substituiert sind durch ein oder mehrere Cyano, Nitro, Halogen, Hydroxy, Trihalogenmethyl, C₁-C₁₀-Alkyl, C₁-C₁₀-Alkoxy oder Aryl.

14. Verbindung nach Anspruch 13, wobei R₁ Wasserstoff oder C₁-C₁₀-Alkyl ist, wobei die Alkyl-, Alkenyl-, Alkinyl- und Arylgruppen gegebenenfalls unabhängig substituiert sind durch ein oder mehrere Cyano, Nitro, Halogen, Hydroxy, Tribalogenmethyl, C₁-C₁₀-Alkyl, C₁-C₁₀-Alkoxy oder Aryl.

15. Verbindung nach einem der Ansprüche 1 bis 14, wobei R₂ Wasserstoff, C₁-C₁₀-Alkyl, C₂-C₁₀-Alkenyl, C₂-C₁₀-Alkinyl, Aryl, Aryl-C₁-C₁₀-alkyl, C₁-C₁₀-Alkyloxy ist, wobei die Alkyl-, Alkenyl-, Alkinyl- und Arylgruppen gegebenenfalls unabhängig substituiert sind durch ein oder mehrere Cyano, Nitro, Halogen, Hydroxy, Trihalogenmethyl, C₁-C₁₀-Alkyl, C₁-C₁₀-Alkoxy oder Aryl.

16. Verbindung nach Anspruch 15, wobei R₂ Wasserstoff, C₁-C₁₀-Alkyl, C₂-C₁₀-Alkenyl, C₂-C₁₀-Alkinyl, Aryl ist, wobei die Alkyl-, Alkenyl-, Alkinyl- und Arylgruppen gegebenenfalls unabhängig substituiert sind durch ein oder mehrere Cyano, Nitro, Halogen, Hydroxy, Trihalogenmethyl, C₁-C₁₀-Alkyl, C₁-C₁₀-Alkoxy oder Aryl.

17. Verbindung nach Anspruch 16, wobei R₂ Wasserstoff oder C₁-C₁₀-Alkyl ist, wobei die Alkyl-, Alkenyl-, Alkinyl- und Arylgruppen gegebenenfalls unabhängig substituiert sind durch ein oder mehrere Cyano, Nitro, Halogen, Hydroxy, Trihalogemnethyl, C₁-C₁₀-Alkyl, C₁-C₁₀-Alkoxy oder Aryl.

18. Verbindung nach Anspruch 17, wobei R₂ Wasserstoff oder C₁-C₁₀-Alkyl ist,

19. Verbindung nach einem der Ansprüche 1 bis 18, wobei R₃ Wasserstoff, C₁-C₁₀-Alkyl, C₂-C₁₀-Alkenyl, C₂-C₁₀-Alkinyl, Aryl, Aryl-C₁-C₁₀-alkyl, AryloxyC₁-C₁₀-alkyl, C₁-C₁₀-Alkylthio-C₁-C₁₀-alkyl oder Aryl-C₁-C₁₀-alkyloxy-C₁-C₁₀-alkyl ist, wobei die Alkyl-, Alkenyl-, Alkinyl- und Arylgruppen gegebenenfalls unabhängig substituiert sind durch ein oder mehrere Cyano, Nitro, Halogen, Hydroxy, Trihalogenmethyl, C₁-C₁₀-Alkyl, C₁-C₁₀-Alkoxy oder Aryl.

20. Verbindung nach Anspruch 19, wobei R₃ Wasserstoff, C₁-C₁₀-Alkyl, C₂-C₁₀-Alkenyl, C₂-C₁₀-Alkinyl, Aryl, C₁-C₁₀-Alkylthio-C₁-C₁₀-alkyl oder Aryl-C₁-C₁₀-alkyl ist, wobei die Alkyl-, Alkenyl-, Alkinyl- und Arylgruppen gegebenenfalls unabhängig substituiert sind durch ein oder mehrere Cyano, Nitro, Halogen, Hydroxy, Trihalogenmethyl, C₁-C₁₀-Alkyl, C₁-C₁₀-Alkoxy oder Aryl.

21. Verbindung nach Anspruch 20, wobei R₃ Wasserstoff oder C₁-C₁₀-Alkyl ist, wobei wobei die Alkyl-, Alkenyl-, Alkinyl- und Arylgruppen gegebenenfalls unabhängig substituiert sind durch ein oder mehrere Cyano, Nitro, Halogen, Hydroxy, Trihalogenmethyl, C₁-C₁₀-Alkyl, C₁-C₁₀-Alkoxy oder Aryl.

22. Verbindung nach Anspruch 21, wobei R₃ Wasserstoff oder C₁-C₁₀-Alkyl ist.

23. Verbindung nach einem der Ansprüche 1 bis 22 wobei R₄ Wasserstoff, C₁-C₁₀-Alkyl, C₂-C₁₀-Alkenyl, C₂-C₁₀-Alkinyl, Aryl, Aryl-C₁-C₁₀-alkyl, Aryloxy-C₁-C₁₀-alkyl oder Aryl-C₁-C₁₀-alkyloxy-C₁-C₁₀-alkyl ist, wobei die Alkyl-, Alkenyl-, Alkinyl- und Arylgruppen gegebenenfalls unabhängig substituiert sind durch ein oder mehrere Cyano, Nitro, Halogen, Hydroxy, Trihalogenmethyl, C₁-C₁₀-Alkyl, C₁-C₁₀-Alkoxy oder Aryl.

24. Verbindung nach Anspruch 23, wobei R₄ Wasserstoff, C₁-C₁₀-Alkyl, C₂-C₁₀-Alkenyl, C₂-C₁₀-Alkinyl, Aryl oder Aryl-C₁-C₁₀-alkyl ist, wobei die Alkyl-, Alkenyl-, Alkinyl- und Arylgruppen gegebenenfalls unabhängig substituiert sind durch ein oder mehrere Cyano, Nitro, Halogen, Hydroxy, Trihalogenmethyl, C₁-C₁₀-Alkyl, C₁-C₁₀-Alkoxy oder Aryl.

25. Verbindung nach Anspruch 24, wobei R₄ Wasserstoff oder C₁-C₁₀-Alkyl ist, wobei die Alkyl-, Alkenyl-, Alkinyl- und Arylgruppen gegebenenfalls unabhängig substituiert sind durch ein oder mehrere Cyano, Nitro, Halogen, Hydroxy, Trihalogenmethyl, C₁-C₁₀-Alkyl, C₁-C₁₀-Alkoxy oder Aryl.

26. Verbindung nach Anspruch 25, wobei R₄ Wasserstoff oder C₁-C₁₀-Alkyl ist.

27. Verbindung nach einem der Ansprüche 1 bis 26, wobei R₉ oder R₁₀ unabhängig ausgewählt sind aus Wasserstoff, C₁-C₄-Alkyl oder Aryl, wobei die Alkyl-, Alkenyl-, Alkinyl- und Arylgruppen gegebenenfalls unabhängig substituiert sind durch ein oder mehrere Cyano, Nitro, Halogen, Hydroxy, Trihalogenmethyl, C₁-C₁₀-Alkyl, C₁-C₁₀-Alkoxy oder Aryl.

28. Verbindung nach Anspruch 27, wobei R₉ oder R₁₀ unabhängig ausgewählt sind aus Wasserstoff, C₁-C₄-Alkyl oder Aryl.

29. Verbindung nach einem der vorangehenden Ansprüche, ausgewählt aus Folgendem:
2-(Oxalylamino)-9*H*-4,5,6,7-tetrahydro-4,8-methanthieno[2,3-c]azocin-3-carbonsäure,
2-(Etoxyoxalylamino)-9*H*-4,5,6,7-tetrahydro-4,8-methanthieno[2,3-c]acocin-3-carbonsäure ethyl ester,
2-(Oxalylamino)-9*H*-4,5,6,7-tetrahydro-4,8-methanthieno[2,3-c]acocin-3-carbonsäureethylester,
2-(Oxalylamino)-9*H*-4,7-dihydro-4,8-methano-benzo[f]thieno[2,3-c]acocin-3-carbonsäure,
2-(Oxalylamino)-4,5,6,8-tetrahydro-4,7-methanthieno[2,3-c]azepin-3-carbonsäure,
9-Methyl-2-(oxalylamino)-4,5,6,7-tetrahydro-4,8-methanthieno[2,3-c]acocin-3-carbonsäure,
2-(Oxalylamino)-6-phenyl-9*H*-4,5,6,7-tetrahydro-4,8-methanthieno[2,3-c]acocin-3-carbonsäure,
7-Methyl-2-(oxalylamino)-9*H*-4,5,6,7-tetrahydro-4,8-methanthieno[2,3-c]acocin-3-carbonsäure,
2-(Oxalylamino)-4,5,6,8-tetrahydro-4,7-ethanthieno[2,3-c]azepin-3-carbonsäure,
2-(Oxalylamino)-5,7-ethano-4,5,6,7-tetrahydrvthieno[2,3-c]pyridin-3-carbonsäure,
2-(Oxalylamino)-1,3,4,6-telrahydro-4,8-methanthieno[2,3-f][1,4]-oxacocin-3-carbonsäure,
2-(Oxalylamino)-9-phenethyl-4,5,6,7-tetrahydro-4,8-methanthieno[2,3-c]acocin-3-carbonsäure,
2-(Oxalylamino)-7-phenethyl-9*H*-4,5,6,7-tetrahydro-4,8-methanthieno[2,3-c]acocin-3-carbonsäure,
2-(Oxalylamino)-9-phenethyl-9*H*-4,5,6,7-tetrahydro-4,8-methanthieno[2,3-c]acocin-3-carbonsäure,
2-(Oxalylamino)-5-phenyl-9*H*-4,5,6,7-tetrahydro-4,8-methanthieno[2,3-c]acocin-3-carbonsäure,
2-(Oxalylamino)-10-phenethyl-9*H*-4,5,6,7-tetrahydro-4,8-methanthieno[2,3-c]acocin-3-carbonsäure,
9-Heptyl-2-(oxalylamino)-4,5,6,7-tetrahydro-4,8-methanthieno[2,3-c]acocin-3-carbonsäure,
9-Heptyl-2-(oxalylamino)-4,5,6,7-tetrahydro-4,8-methanthieno[2,3-c]acocin-3-carbonsäure,
2-(Oxalylamino)-7-phenyl-9*H*-4,5,6,7-tetrahydro-4,8-methanthieno[2,3-c]acocin-3-carbonsäure,
2-(Oxalylamino)-9-pentyl-4,5,6,7-teuahydro-4,8-methanthieno[2,3-c]acocin-3-carbonsäure,
9-(2-Cyclohexylethyl)-2-(oxalylamino)-4,5,6,7-tetrahydro-4,8-methanthieno[2,3-c]acocin-3-carbonsäure,
9-(2-Methylsulfanylethyl)-2-(oxalylamino)-4,5,6,7-tetrahydro-4,8-methanthieno[2,3-c]acocin-3-carbonsäure,
9-(Ethyl)-2-(oxalylamino)-4,5,6,7-tetrahydro-4,8-methanthieno[2,3-c]acocin-3-carbonsäure,
2-(Oxalylamino)-9-(propyl)-4,5,6,7-tetrahydro-4,8-methanthieno[2,3-c]acocin-3-carbonsäure,
2-(*tert*-Butoxyoxalylamino)-4,5,6,7-tetrahydro-4,8-methanthieno[2,3-c]acocin-3-carbonsäureethylester,
2-(Isopropoxyoxalylamino)-4,5,6,7-tetrahydro-4,8-methanthieno[2,3-c]acocin-3-carbonsäureethylester,
2-(Benzoxyoxalylamino)-4,5,6,7-tetrahydro-4,8-methonthieno[2,3-c]acocin-3-carbonsäureethylester,
2-(Benzoxyoxalylamino)-4,5,6,7-tetrahydro-4,8-methanthieno[2,3-c]acocin-3-carbonsäurebenzylester,
2-(*tert*-Butoxyoxalylamino)-4,5,6,7-tetrahydro-4,8-methanthieno[2,3-c]acocin-3-carbonsäurebenzylester,
2-(*tert*-Butoxyoxalylamino)-9-pentyl-4,5,6,7-tetrahydro-4,8-methanthieno[2,3-c]acocin-3-carbonsäurebenzylester,
2-(*tert*-Butoxyoxalylamino)-9-pentyl-4,5,6,7-tetrahydro-4,8-methanthieno[2,3-c]acocin-3-carbonsäureethylester,
2-(Oxalylamino)-9-(octyl)-4,5,6,7-tetrahydro-4,8-methanthieno[2,3-c]acocin-3-carbonsäure,
2-(Oxalylamino)-9-(4-phenylbutyl))-4,5,6,7-tetrahydro-4,8-methanthieno[2,3-c]acocin-3-carbonsäure,
2-(Oxalylamino)-9-(2-cyclopentylethyl)-4,5,6,7-tetrahydro-4,8-methanthieno[2,3-c]acocin-3-carbonsäure,
9-(3-Methylbutyl)-2-(oxalylamino)-4,5,6,7-tetrahydro-4,8-methanthieno[2,3-c]acocin-3-carbonsäure,
2-(Oxalylamino)-9-(4-phenyl-2-methylbutyl)-4,5,6,7-tetrahydro-4,8-methanthieno[2,3-c]acocin-3-carbonsäure,
2-(*tert*-Butoxyoxalylamino)-4,5,6,7-tetrahydro-4,8-methanthieno[2,3-c]acocin-3-carbonsäureethylester,
2-(Isopropoxyoxalylamino)-4,5,6,7-tetrahydro-4,8-methanthieno[2,3-c]acocin-3-carbonsäureethylester,
2-(*tert*-Butoxyoxalylamino)-4,5,6,7-tetrahydro-4,8-methanthieno[2,3-c]acocin-3-carbonsäurebenzylester,
2-(*tert*-Butoxyoxalylamino)-9-pentyl-4,5,6,7-tetrahydro-4,8-methanthieno[2,3-c]acocin-3-carbonsäurebenzylester,
2-(*tert*-Butoxyoxalylamino)-9-pentyl-4,5,6,7-tetrahydro-4,8-methanthieno[2,3-c]acocin-3-carbonsäureethylester,
9-(3-Cyclohexylpropyl)-2-(oxalylamino)-4,5,6,7-tetrahydro-4,8-methanthieno[2,3-c]acocin-3-carbonsäure,
9-Pentyl-2-(*iso*-propoxyoxalylamino)-4,5,6,7-tetrahydro-4,8-methanthieno[2,3-c]acocin-3-carbonsäure-*tert*-butylester,
9-Pentyl-2-(*iso*-propoxyoxalylamino)-4,5,6,7-tetrahydro-4,8-methanthieno[2,3-c]acocin-3-carbonsäure,
9-(3-Cyclohexylpropyl)-2-(oxalylamino)-4,5,6,7-tetrahydro-4,8-methanthieno[2,3-c]acocin-3-carbonsäure,
9-Pentyl-2-(*iso*-propoxyoxalylamino)-4,5,6,7-tetrahydro-4,8-methanthieno[2,3-c]acocin-3-carbonsäure-*tert*-butylester,
9-Pentyl-2-(*iso*-propoxyoxalylamino)-4,5,6,7-tetrahydro-4,8-methanthieno[2,3-c]acocin-3-carbonsäure.

30. Arzneimittel, umfassend eine Verbindung nach einem der Ansprüche 1 bis 10 oder ein pharmazeutisch verträgliches Salz davon mit einer pharmazeutisch verträglichen Säure oder Base oder ein beliebiges optisches Isomer oder Gemisch aus optischen Isomeren, einschließlich eines racemischen Gemischs, oder beliebige tautomere Formen davon zusammen mit einem oder mehreren pharmazeutisch verträglichen Trägern oder Verdünnungsmitteln.

31. Arzneimittel nach Anspruch 30, das zur Behandlung, Bewältigung oder Verhütung von Diabetes Typ 1, Diabetes Typ 2, beeinträchtigter Glucoseverträglichkeit, Insulinresistenz, Leptinresistenz und/oder Fettsucht geeignet ist

32. , Verwendung einer Verbindung nach einem der Ansprüche 1 bis 29 oder eines pharmazeutisch verträglichen Salzes davon mit einer pharmazeutisch verträglichen Säure oder Base oder eines beliebigen optischen Isomers oder Gemischs aus optischen Isomeren, einschließlich eines racemischen Gemischs, oder von beliebigen tautomeren Formen davon zusammen mit einem oder mehreren pharmazeutisch verträglichen Trägern oder Verdünnungsmitteln als Arzneimittel.

33. Verwendung einer Verbindung nach einem der Ansprüche 1 bis 29 oder eines pharmazeutisch verträglichen Salzes davon mit einer pharmazeutisch verträglichen Säure oder Base oder eines beliebigen optischen Isomers oder Gemischs aus optischen Isomeren, einschließlich eines racemischen Gemischs, oder von beliebigen tautomeren Formen davon zusammen mit einem oder mehreren pharmazeutisch verträglichen Trägern oder Verdünnungsmitteln zur Herstellung eines Medikaments.

34. Verwendung nach Anspruch 33 zur Herstellung eines Medikaments, das zur Behandlung von Immunfehlfunktionen, einschließlich Autoimmtmität, Erkrankungen mit Fehlfunktionen des Blutgerinnungssystems, allergischen Erkrankungen, Osteoporose, Wucherungsstörungen, einschließlich Krebs und Schuppenflechte, Erkrankungen mit verminderter oder erhöhter Synthese oder Wirkungen des Wachstumshormons, Erkrankungen mit verminderter oder erhöhter Synthese von Hormonen oder Cytokinen, die die Abgabe von oder das Ansprechverhalten auf Wachstumshormon regulieren, Erkrankungen des Gehirns, einschließlich Alzheimer-Krankheit und Schizophrenie, und Infektionserkrankungen geeignet ist.

## Revendications

1. Composé de formule 1 : 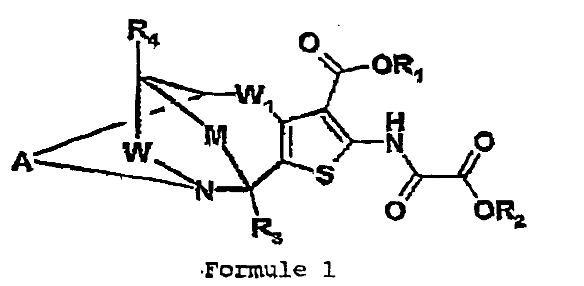 dans laquelle
R₁ et R₂ sont indépendamment l'hydrogène ou un groupe fonctionnel qui peut être converti en hydrogène in vivo ;
R₃ et R₄ sont indépendamment l'hydrogène ou un radical alkyle en C₂-C₁₀, alcényle en C₂-C₁₀, alcynyle en C₂-C₁₀,, aryle, aryl(alkyle en C₁-C₁₀), (alkyl en C₁-C₁₀)oxyalkyle en C₁-C₁₀, aryloxy(alkyle en C₁-C₁₀), aryl (alkyl en C₁-C₁₀)oxyalkyle en C₁-C₁₀, (alkyl en C₁-C₁₀)aminoalkyle en C₁-C₁₀, (alkyl en C₁-C₁₀)thioalkyle en C₁-C₁₀, aryl(alkyl en C₁-C₁₀)aminoalkyle en C₁-C₁₀, di[aryl(alkyl en C₁-C₁₀)]aminoalkyle en C₁-C₁₀, (alkyl en C₁-C₁₀) carbonylamino(alkyle en C₁-C₁₀), aryl (alkyl en C₁-C₁₀)carbonylamino(alkyle en C₁-C₁₀), CONR₅R₆, où les groupes alkyle, alcényle, alcynyle et aryle sont éventuellement substitués par un ou plusieurs radicaux cyano, nitro, halogéno, hydroxy, trihalogénométhyle, alkyle en C₁-C₁₀, alcoxy en C₁-C₁₀ ou aryle indépendamment ;
R₅ et R₆ sont indépendamment choisis parmi l'hydrogène et les radicaux alkyle en C₁-C₁₀, alcényle en C₂-C₁₀, alcynyle en C₂-C₁₀, aryle, aryl(alkyle en C₁-C₁₀), (alkyl en C₁-C₁₀)carbonyle, (alkyl en C₁-C₁₀)oxocarbonyle, arylcarbonyle, aryloxocarbonyle, aryl(alkyl en C₁-C₁₀) carbonyle, aryl (alkyl en C₁-C₁₀) oxocarbonyle, où les groupes alkyle, alcényle, alcynyle et aryle sont éventuellement substitués par un ou plusieurs radicaux cyano, nitro, halogéno, hydroxy, trihalogénométhyle, alkyle en C₁-C₁₀, alcoxy en C₁-C₁₀, ou aryle indépendamment ; ou bien
R₅ et R₆ peuvent former un système cyclique, bicyclique ou tricyclique, saturé, partiellement saturé ou aromatique, contenant de 3 à 14 atomes de carbone et de 0 à 3 hétéroatomes supplémentaires choisis parmi l'azote, l'oxygène et le soufre, avec l'azote auxquels ils sont rattachés, le système cyclique pouvant éventuellement être substitués par au moins un radical alkyle en C₁-C₁₀, alcényle en C₂-C₁₀, alcynyle en C₂-C₁₀, aryle, aryl(alkyle en C₁-C₁₀), hydroxy, oxo, alkyloxy en C₁-C₁₀, aryl(alkyloxy en C₁-C₁₀), (alkyl en C₁-C₁₀)oxyalkyle en C₁-C₁₀, NR₇R₈ ou (alkyl en C₁-C₁₀)aminoalkyle en C₁-C₁₀, où R₇ et R₈ pont indépendamment choisis parmi l'hydrogène et les radicaux alkyle en C₁-C₁₀, alcényle en C₂-C₁₀, alcynyle en C₂-C₁₀, aryle, aryl(alkyle en C₁-C₁₀), (alkyl en C₁-C₁₀)carbonyle, arylcarbonyle, aryl(alkyl en C₁-C₁₀)carbonyle, (alkyl en C₁-C₁₀)carboxy ou aryl(alkyl en C₁-C₁₀)carboxy ; où les groupes alkyle, alcényle, alcynyle et aryle sont éventuellement substitués par un ou plusieurs radicaux cyano, nitro, halogéno, hydroxy, trihalogénométhyle, alkyle en C₁-C₁₀, alcoxy en C₁-C₁₀, ou aryle indépendamment ; ou bien
R₅ et R₆ sont indépendamment une amine, un imide ou un lactarge saturé ou partiellement saturée cyclique, à 5, 6 ou 7 chaînons ;
A est absent ou est -[C(R₉R₁₀)]ᵢ-, -[(CR₁₁R₁₂)]ⱼ-C(R₁₃)=c (R₁₄)=[C (R₁₅R₁₆)]ₖ-, -[C(R₁₇R₁₈)]_{y}-(X)-[C(R₁₉R₂₀)]_{z}- ; où X est O, NR₂₁ ou S ; i vaut 1, 2, 3 ou 4 ; y et z valent indépendamment 0, 1, 2 ou 3 ; j et k valent indépendamment 0, 1 ou 2 ;
M est absent ou est -[C(R₂₉R₃₀)]ₚ- ; où p vaut 1, 2 ou 3 ; avec comme condition que A et M ne peuvent pas être tous deux absents ;
W est une liaison de valence ou -[C(R₃₁R₃₂)]_{q}- ; où q vaut 1 ou 2 ;
W₁ est une liaison de valence ou -[C(R₃₃R₃₄)]_{qq} ; où qq vaut 1 ou 2 ;
R₉, R₁₀, R₁₁, R₁₂, R₁₃, R₁₄, R₁₅, R₁₆, R₁₈, R₁₉, R₂₀, R₂₁, R₃₁, R₃₂, R₃₃ et R₃₄ sont indépendamment choisis parmi l'hydrogène et les radicaux alkyle en C₁-C₈, alcényle en C₂-C₁₀, alcynyle en C₂-C₁₀, aryle, et aryl(alkyle en C₁-C₄) ; où les groupes alkyle, alcényle, alcynyle et aryle sont éventuellement substitués par un ou plusieurs radicaux cyano, nitro, halogéno, hydroxy, trihalogenométhyle, alkyle en C₁-C₁₀, alcoxy en C₁-C₁₀, ou aryle indépendamment ;
ou un sel d'un tel composé avec un acide ou une base acceptable en pharmacie, ou n'importe quel isomère optique
ou mélange d'isomères optiques, y compris un mélange racémique, ou n'importe quelles formes tautomères.

2. , Composé selon la revendication 1, dans lequel R₁ et R₂ sont indépendamment l'hydrogène ou un radical alkyle en C₁-C₁₀, alcényle en C₂-C₁₀, alcynyle en C₂-C₁₀, aryle, aryl(alkyle en C₁-C₁₀), alkyloxy en C₁-C₁₀, (alkyl en C₁-C₁₀)oxyalkyloxy en C₁-C₁₀, aryloxy, et aryl(alkyloxy en C₁-C₁₀) ; où les groupes alkyle, alcényle, alcynyle et aryle sont éventuellement substitués par un ou plusieurs radicaux cyano, nitro, halogéno, hydroxy, trihalogénométhyle, alkyle en C₁-C₁₀, alcoxy en C₁-C₁₀, ou aryle indépendamment,

3. Composé selon l'une quelconque des revendications 1 et 2, dans lequel A est absent ou est [C(R₉R₁₀)]ᵢ-, -[C(R₁₇R₁₈)]_{y}-(X)-[C(R₁₉R₂₀)]ᵣ- ; où X est Q, NR₂₁ ou s ; i vaut 1, 2, 3 ou 4 ; y et z valent indépendamment 0, 1, 2 ou 3.

4. Composé selon la revendication 3, dans lequel A est -[C(R₉R₁₀)]ᵢ-, où i vaut 1, 2, 3 ou 4.

5. Composé selon l'une quelconque des revendications 1 à 4, dans lequel M est -[C(R₂₉R₃₀)]ₚ- ; où p vaut 1, 2 ou 3.

6. Composé selon l'une quelconque des revendications 1 à 4, dans lequel M est absent.

7. Composé selon l'une quelconque des revendications 1 à 5, dans lequel W est une liaison de valence.

8. Composé selon l'une quelconque des revendications 1 à 6, dans lequel W est -[C(R₃₁R₃₂)]_{q}- ; où q vaut 1 ou 2.

9. Composé selon l'une quelconque des revendications 1 à 8, dans lequel W₁ est une liaison de valence.

10. Composé selon l'une quelconque des revendications 1 à 8, dans lequel W₁ est -[C(R₃₃R₃₄)]_{qq} ; où qq vaut 1 ou 2.

11. Composé selon l'une quelconque des revendications 1 à 10, dans lequel R₁ est l'hydrogène ou un radical alkyle en C₁-C₁₀, alcényle en C₂-C₁₀, alcynyle en C₂-C₁₀, aryle, aryl(alkyle en C₁-C₁₀, alkyloxy en C₁-C₁₀ ; où les groupes alkyle, alcényle, alcynyle et aryle sont éventuellement substitués par un ou plusieurs radicaux cyano, nitro, halogéno, hydroxy, trihalogénométhyle, alkyle en C₁-C₁₀, alcoxy en C₁-C₁₀, ou aryle indépendamment.

12. Composé selon la revendication 11, dans lequel R₁ est l'hydrogène ou un radical alkyle en C₁-C₁₀, alcényle en C₂-C₁₀, alcynyle en C₂-C₁₀, aryle, ou aryl(alkyle en C₁-C₁₀) ; où les groupes alkyle, alcényle, alcynyle et aryle sont éventuellement substitués par un ou plusieurs radicaux cyano, nitro, halogéno, hydroxy, trihalogénométhyle, alkyle en C₁-C₁₀, alcoxy en C₁-C₁₀ ou aryle indépendamment.

13. Composé selon la revendication 12, dans lequel R₁ est l'hydrogène ou un radical alkyle en C₁-C₁₀ ou aryl (alkyle en C₁-C₁₀) ; où les groupes alkyle sont éventuellement substitués par un ou plusieurs radicaux cyano, nitro, halogéno, hydroxy, trihalogénométhyle, alkyle en C₁-C₁₀, alcoxy en C₁-C₁₀, ou aryle indépendamment.

14. Composé selon la revendication 13, dans lequel R₁ est l'hydrogène ou un radical alkyle en C₁-C₁₀ ; où le groupe alkyle est éventuellement substitué par un ou plusieurs radicaux cyano, nitro, halogéno, hydroxy, trihalogénométhyle, alkyle en C₁-C₁₀, alcoxy en C₁-C₁₀, ou aryle indépendamment.

15. Composé selon l'une quelconque des revendications 1 à 14, dans lequel R₂ est l'hydrogène ou un radical alkyle en C₁-C₁₀, alcényle en C₂-C₁₀, alcynyle en C₂-C₁₀, aryle, aryl(alkyle en C₁-C₁₀, alkyloxy en C₁-C₁₀ ; où les groupes alkyle, alcényle, alcynyle et aryle sont éventuellement substitués par un ou plusieurs radicaux cyano, nitro, halogéno, hydroxy, trihalogénométhyle, alkyle en C₁-C₁₀, alcoxy en C₁-C₁₀, ou aryle indépendamment.

16. Composé selon la revendication 15, dans lequel R₂ est l'hydrogène ou un radical alkyle en C₁-C₁₀, alcényle en C₂-C₁₀, alcynyle en C₂-C₁₀, aryle ; où les groupes alkyle, alcényle, alcynyle et aryle sont éventuellement substitués par un ou plusieurs radicaux cyano, nitro, halogéno, hydroxy, trihalogénométhyle, alkyle en C₁-C₁₀, alcoxy en C₁-C₁₀ ou aryle indépendamment.

17. Composé selon la revendication 16, dans lequel R₁ est l'hydrogène ou un radical alkyle en C₁-C₁₀ ; où le groupe alkyle est éventuellement substitué par un ou plusieurs radicaux cyano, nitro, halogéno, hydroxy, trihalogénométhyle, alkyle en C₁-C₁₀, alcoxy en C₁-C₁₀, ou aryle indépendamment.

18. Composé selon la revendication 17, dans laquelle R₂ est l'hydrogène ou un radical alkyle en C₁-C₁₀.

19. Composé selon l'une quelconque des revendications 1 à 18, dans lequel R₃ est l'hydrogène ou un radical alkyle en C₁-C₁₀, alcényle en C₂-C₁₀, alcynyle en C₂-C₁₀, aryle, aryl(alkyle en C₁-C₁₀), aryloxy(alkyle en C₁-C₁₀), (alkyl en C₁-C₁₀)thioalkyle en C₁-C₁₀, ou aryl(alkyl en C₁-C₁₀)oxyalkyle en C₁-C₁₀, où les groupes alkyle, alcényle, alcynyle et aryle sont éventuellement substitués par un ou plusieurs radicaux cyano, nitro, halogéno, hydroxy, trihalogénométhyle, alkyle en C₁-C₁₀, aleoxy en C₁-C₁₀, ou aryle indépendamment.

20. Composé selon la revendication 19, dans lequel R₃ est l'hydrogène ou un radical alkyle en C₁-C₁₀, alcényle en C₂-C₁₀, alcynyle en C₂-C₁₀, aryle, (alkyl en C₁-C₁₀)thioalkyle en C₁-C₁₀, ou aryl(alkyle en C₁-C₁₀), où les groupes alkyle, alcényle, alcynyle et aryle sont éventuellement substitués par un ou plusieurs radicaux cyano, nitro, halogéno, hydroxy, trihalogénométhyle, alkyle en C₁-C₁₀, alcoxy en C₁-C₁₀, ou aryle indépendamment.

21. Composé selon la revendication 20, dans lequel R₃ est, l'hydrogène ou un radical alkyle en C₁-C₁₀, où le groupe alkyle est éventuellement substitué par un ou plusieurs radicaux cyano, nitro, halogéno, hydroxy, trihalogénométhyle, alkyle en C₁-C₁₀, alcoxy en C₁-C₁₀, ou aryle indépendamment.

22. Composé selon la revendication 21, dans lequel R₃ est l'hydrogène ou un radical alkyle en C₁-C₁₀.

23. Composé selon l'une quelconque des revendications 1 à 22, dans lequel R₄ est l'hydrogène ou un radical alkyle en C₁-C₁₀, alcényle en C₂-C₁₀, alcynyle en C₂-C₁₀, aryle, aryl(alkyle en C₁-C₁₀), aryloxy(alkyle en C₁-C₁₀), ou aryl(alkyl en C₁-C₁₀)oxyalkyle en C₁-C₁₀, où les groupes alkyle, alcényle, alcynyle et aryle sont éventuellement substitués par un ou plusieurs radicaux cyano, nitro, halogéno, hydroxy, trihalogénométhyle, alkyle en C₁-C₁₀, alcoxy en C₁-C₁₀, ou aryle indépendamment.

24. Composé selon la revendication 23, dans lequel R₄ est l'hydrogène ou un radical alkyle en C₁-C₁₀, alcényle en C₂-C₁₀, alcynyle en C₂-C₁₀, aryle, ou aryl(alkyle en C₁-C₁₀),
où les groupes alkyle, alcényle, alcynyle et aryle sont éventuellement substitués par un ou plusieurs radicaux cyano, nitro, halogéno, hydroxy, trihalogénométhyle, alkyle en C₁-C₁₀, alcoxy en C₁-C₁₀, ou aryle indépendamment.

25. Composé selon la revendication 24, dans lequel R₄ est l'hydrogène ou un radical alkyle en C₁-C₁₀, où le groupe alkyle est éventuellement substitué par un ou plusieurs radicaux cyano, nitro, halogéno, hydroxy, trihalogénométhyle, alkyle en C₁-C₁₀, alcoxy en C₁-C₁₀, ou aryle indépendamment.

26. Composé selon la revendication 25, dans lequel R₄ est l'hydrogène ou un radical alkyle en C₁-C₁₀.

27. Composé selon l'une quelconque des revendications 1 à 26, dans lequel R₉ ou R₁₀ sont indépendamment choisis parmi l'hydrogène et les radicaux alkyle en C₁-C₄ ou aryle, où les groupes alkyle et aryle sont éventuellement substitués par un ou plusieurs radicaux cyano, nitro, halogéno, hydroxy, trihalogénométhyle, alkyle en C₁-C₁₀, alcoxy en C₁-C₁₀, ou aryle indépendamment.

28. Composé selon la revendication 27, dans lequel R₉ ou R₁₀ sont indépendamment choisis parmi l'hydrogène et les radicaux alkyle en C₁-C₄ ou aryle.

29. Composé selon l'une quelconque des revendications précédentes, choisi parmi les suivants :
acide 2-(oxalylamino)-9H-4,5,6,7-tétrahydro-4,8-méthano-thiéno[2,3-c]azocine-3-carboxylique ;
ester éthylique d'acide 2-(éthoxyoxalylamino)-9H-4,5,6,7-tétrahydro-4,8-méthano-thiéno[2,3-c]azocine-3-carboxylique ;
ester éthylique d'acide 2-(oxalylamino)-9H-4,5,6,7-tétrahydro-4,8-méthano-thiéno[2,3-c]azocine-3-carboxylique ;
acide 2-(oxalylamino)-9H-4,7-dihydro-4,8-méthano-benzo[f]thiéno[2,3-c]azocine-3-carboxylique ;
acide 2-(oxalylamino)-4,5,6,8-tétrahydro-4,7-méthano-thiéno[2,3-c]azépine-3 carboxylique ;
acide 9-méthyl-2-(oxalylamino)-4,5,6,7-tetrahydro-4,8-méthano-thiéno[2,3-c]azocine-3-carboxylique ;
acide 2-(oxalylamino)-6-phényl-9H-4,5,6,7-tétrahydro-4,8-méthano-thiéno[2,3-c]azocine-3-carboxylique ;
acide 7-méthyl-2-(oxalylamino)-9H-4,5,6,7-tétrahydro-4,8-méthano-thiéno[2,3-c]azocine-3-carboxylique ;
acide 2-(oxalylamino)-4,5,6,8-tétrahydro-4,7-éthano-thiéno[2,3-c]azépine-3 carboxylique ;
acide 2-(oxalylamino)-5,7-éthano-4,5,6,7-tétrahydro-thiéno[2,3-c]pyridine-3-carboxylique ;
acide 2-(oxalylamino)-1,3,4,6-tétrahydro-9,8-méthano-thiéno[2,3-f][1,4]-oxazocine-3-carboxylique ;
acide 2-(oxalylamino)-9-phénéthyl-4,5,6,7-tétrahydro-4,8-méthano-thiéno[2,c]azocine-3-carboxylique ;
acide 2-(oxalylamino)-7-phénéthyl-9H-4,5,6,7-tétrahydro-4,8-méthano-thiéno[2,3-c]azocine-3-carboxylique ;
acide 2-(oxalylamino)-9-phénéthyl-9H-4,5,6,7-tétrahydro-4,8-méthano-thiéno[2,3-c]azocine-3-carboxylique ;
acide 2-(oxalylamino)-5-phényl-9H-4,5,6,7-tétrahydro-4,8-méthano-thiéno[2,3-c]azocine-3-carboxylique ;
acide 2-(oxalylamino)-10-phénéthyl-9H-4,5,6,7-tétrahydro-4,8-méthano-thiéno[2,3-c]azocine-3-carboxylique ;
acide 9-heptyl-2-(oxalylamino)-4,5,6,7-tétrahydro-4,8-méthano-thiéno[2,3-c]azocine-3-carboxylique ;
acide 9-heptyl-2-(oxalylamino)-4,5,6,7-tétrahydro-4,8-méthano-thiéno[2,3-c]azocine-3-carboxylique ;
acide 2-(oxalylamino)-7-phényl-9H-4,5,6,7-tétrahydro-4,8-méthano-thiéno[2,3-c]azocine-3-carboxylique ;
acide 2-(oxalylamino)-9-pentyl-4,5,6,7-tétrahydro-4,8-méthano-thiéno[2,3-c]azocine-3-carboxylique acid;
acide 9-(2-cyclohexyl-éthyl)-2-(oxalylamino)-4,5,6,7-tétrahyero-4, 8-méthano-thiéno[2, 3-c]azocine-3-carboxylique ;
acide 9-(2-méthylsultanyl-éthyl)-2-(oxalylamino)-4,5,6,7-tétrahydro-4,8-méthano-thiéno[2,3-c]azocine-3-carboxylique ;
acide 9-(éthyl)-2-(oxalylamino)-4,5,6,7-tétrahydro-4,8-méthano-thiéno[2,3-c]azocine-3-carboxylique ;
acide 2-(oxalylamino)-9-(propyl)-4,5,6,7-tétrahydro-4,8-méthano-thiéno[2,3-c]azocine-3-carboxylique ;
ester éthylique d'acide 2-(tert-butoxyoxalylamino)-4,5,6,7-tétrahydro-4,8-méthano-thiéno[2,3-c]azocine-3-carboxylique ;
ester éthylique d'acide 2-(isopropoxyoxalylamino)-4,5,6,7-tétrahydro-4,8-méthano-thiéno[2,3-c]azocine-3-carboxylique ;
ester éthylique d'acide 2-(benzoxyoxalylamino)-4,5,6,7-tétrahydro-4,8-méthano-thiéno[2,3-c]azocine-3-carboxylique ;
ester benzylique d'acide 2-(benzoxyoxalylamino)-4,5,6,7-tétrahydro-4,8-méthano-thiéno[2,3-c]azocine-3-carboxylique ;
ester benzylique d'acide 2-(tert-butoxyoxalylamino)-4,5,6,7-tétrahydro-4,8-méthano-thiéno[2,3-c]azocine-3-carboxylique ;
ester benzylique d'acide 2-(tert-butoxyoxalylamino)-9-pentyl-4,5,6,7-tétrahydro-4,8-méthano-thiéno[2,3-c]azocine-3-carboxylique ;
ester éthylique d'acide 2-(tert-butoxyoxalylamino)-9-pentyl-4,5,6,7-tétrahydro-4,8-méthano-thiéno[2,3-c]azocine-3-carboxylique ;
acide 2-(oxalylamino)-9-(octyl)-4,5,6,7-tétrahydro-4,8-méthano-thiéno[2,3-c]azocine-3-carboxylique;
acide 2-(oxalylamino)-9-(4-phényl-butyl))-4,5,6,7-tétrahydro-4,8-méthano- thiéno[2,3-c]azocine-3-carboxylique ;
acide 2-(oxalylamino)-9-(2-cyclopentyl-éthyl)-4,5,6,7-tétrahydro-4,8-méthano-thiéno[2,3-c]azocine-3-carboxylique ;
acide 9-(3-méthyl-butyl)-2-(oxalylamino)-4,5,6,7-tétrahydro-4,8-méthano-thiéno[2,3-c]azocine-3-carboxylique ;
acide 2-(oxalyl-amino)-9-(4-phényl-2-méthyl-butyl)-4,5,6,7-tétrahydro-4,8-méthano-thiéno[2,3-c]azocine-3-carboxylique ;
ester éthylique d'acide 2-(tert-butoxyoxalylamino)-4,5,6,7-tétrahydro-4,8-méthano-thiéno[2,3-c]azocine-3-carboxylique ;
ester éthylique d'acide 2-(isopropoxyoxalylamino)-4,5,6,7-tétrahydro-4,8-méthano-thiéno[2,3-c]azocine-3-carboxylique ;
ester benzylique d'acide 2-(tert-butoxyoxalylemino)-4,5,6,7-tétrahydro-4,8-méthano-thiéno[2,3-c]azocine-3-carboxylique ;
ester benzylique d'acide 2-(tert-butoxyoxalylamino)-9-pentyl-4,5,6,7-tétrahydro-4,8-méthano-thiéno[2,3-c]azocine-3-carboxylique ;
ester éthylique d'acide 2-(tert-butoxyoxalylamino)-9-pentyl-4,5,6,7-tétrahydro-4,8-méthano-thiéno[2,3-c]azocine-3-carboxylique ;
acide 9-(3-cyclohexyl-propyl)-2-(oxalylamino)-4,5,6,7-tétrahydro-4,8-méthano-thiéno[2,3-c]azocine-3-carboxylique ;
ester tert-butylique d'acide 9-pentyl-2-(iso-propoxyoxalyl-amino)-4,5,6,7-tétrahydro-4,8-méthano-thiéno[2,3-c]azocine-3-carboxylique ;
acide 9-pentyl-2-(iso-propoxyoxalyl-amino)-4,5,6,7-tétrahydro-4,8-méthano-thiéno[2, 3-c]azocine-3-carboxylique ;
acide 9-(3-cyclohexyl-propyl)-2-(oxalylamino)-4,5,6,7-tétrahydro-4,8-méthano-thiéno[2,3-c]azocine-3-carboxylique ;
ester tert-butylique d'acide 9-pentyl-2-(iso-propoxyoxalyl-amino)-4,5,6,7-tétrahydro-4,8-méthano-thiéno[2,3-c]azocine-3-carboxylique ;
acide 9-pentyl-2-(iso-propoxyoxalyl-amino)-4,5,6,7-tétrehydro-4,8 méthano-thiéno[2,3-c]azacine-3-carboxylique.

30. Composition pharmaceutique comprenant un composé selon l'une quelconque des revendications 1 à 29 ou un sel acceptable en pharmacie de celui-ci avec un acide ou une base acceptable en pharmacie, ou n'importe quel isomère optique ou mélange d'isomères optiques, y compris un mélange racémique, ou n'importe quelles formes tautomères, conjointement avec un ou plusieurs véhicules ou diluants acceptables en pharmacie.

31. , Composition pharmaceutique selon la revendication 30, convenant pour traiter, gérer ou prévenir le diabète de type 1, le diabète de type 2, une altération de la tolérance au glucose, la résistance à l'insuline, la résistance à la leptine et/ou l'obésité.

32. Utilisation d'un composé selon l'une quelconque des revendications 1 à 29 ou d'un sel acceptable en pharmacie de celui-ci avec un acide ou une base acceptable en pharmacie, ou de n'importe quel isomère optique ou mélange d'isomères optiques, y compris un mélange racémique, ou de n'importe quelles formes tautomères, conjointement avec un ou plusieurs véhicules ou diluants acceptables en pharmacie, en tant que médicament.

33. Utilisation d'un composé selon l'une quelconque des revendications 1 à 29 ou d'un sel acceptable en pharmacie de celui-ci avec un acide ou une base acceptable en pharmacie, ou de n'importe quel isomère optique ou mélange d'isomères optiques, y compris un mélange racémique, ou de n'importe quelles formes tautomères, conjointement avec un ou plusieurs véhicules ou diluants acceptables en pharmacie, pour la préparation d'un médicament.

34. Utilisation selon la revendication 33, pour la préparation d'un médicament convenable pour le traitement des dysfonctionnements immunitaires, y compris une auto-immunité, des maladies avec des dysfonctionnements du système de la coagulation, des maladies allergiques, de l'ostéoporose, des troubles prolifératifs, y compris le cancer et la psoriasis, des maladies avec une diminution ou une augmentation de la synthèse ou des effets de l'hormone de croissance, des maladies avec une diminution ou une augmentation de la synthèse d'hormones ou de cytokines qui régulent la libération de et/ou la réponse à l'hormone de croissance, des maladies du cerveau, y compris la maladie d'Alzheimer et la schizophrénie, et des maladies infectieuses.
